# EUROPEAN PATENT SPECIFICATION

(11) **EP 4 251 615 B1**
(45) Date of publication and mention of the grant of the patent: **15.10.2025**
(21) Application number: 21819402.5
(22) Date of filing: 22.11.2021
(51) Int. Cl.: C07D 277/64, C07D 417/12, C07D 513/04, C07F 9/6541, A61P 31/20, A61K 31/428

(54) **SPIRO[3.3]HEPTANE DERIVATIVES FOR THE TREATMENT AND PROPHYLAXIS OF HEPATITIS B VIRUS INFECTION**
SPIRO[3.3]HEPTAN-DERIVATE ZUR BEHANDLUNG UND PROPHYLAXE VON HEPATITIS-B-VIRUS-INFEKTIONEN
DÉRIVÉS DE SPIRO[3.3]HEPTANE POUR LE TRAITEMENT ET LA PROPHYLAXIE D'UNE INFECTION PAR LE VIRUS DE L'HÉPATITE B

(30) Priority: 24.11.2020 WO PCT/CN2020/131154
(43) Date of publication of application: 04.10.2023
(73) Proprietor: F. Hoffmann-La Roche AG, 4070 Basel (CH)
(72) Inventor: LIN, Xianfeng, Shanghai 201203 (CN); WANG, Jianping, Shanghai 201203 (CN); YUN, Hongying, Shanghai 201203 (CN); ZHENG, Xiufang, Shanghai 201203 (CN)
(74) Representative: Schirlin, Julien
(86) International application number: PCT/EP2021/082540
(87) International publication number: WO 2022/112188

(56) References cited:
- FENG SONG ET AL: "Discovery of Small Molecule Therapeutics for Treatment of Chronic HBV Infection", ACS INFECTIOUS DISEASES, vol. 4, no. 3, 2018, pages 257 - 277, XP055882735, ISSN: 2373-8227, DOI: 10.1021/acsinfecdis.7b00144
- PEI YAMENG ET AL: "Past, Current, and Future Developments of Therapeutic Agents for Treatment of Chronic Hepatitis B Virus Infection", JOURNAL OF MEDICINAL CHEMISTRY, vol. 60, no. 15, 2017, pages 6461 - 6479, XP055887884, ISSN: 0022-2623, DOI: 10.1021/acs.jmedchem.6b01442

## Description

The present invention relates to organic compounds useful for therapy and/or prophylaxis of HBV infection in a mammal, and in particular to HBsAg (HBV Surface antigen) and HBeAg (HBV e antigen) inhibitors useful for treating HBV infection.

### FIELD OF THE INVENTION

The present invention relates to spiral ring compounds and their corresponding derivatives that have anti-virus activity, as well as their manufacture, pharmaceutical compositions containing them and their potential use as medicaments.

Hepatitis B virus (HBV) is one of the most dangerous human pathogens. A safe and effective vaccine has been available for longer than two decades; however, WHO estimated that approximately 257 million people are chronically infected with HBV. Chronic Hepatitis B (CHB) infection predisposes its host to severe liver disease, including liver cirrhosis and hepatocellular carcinoma, if left untreated. HBV infection is ranked among the top unmet medical need worldwide. The currently approved drugs have contributed to substantial progress in CHB treatment; however, the cure rate remains less than 10%.

The control of viral infection needs an effective immune surveillance. Upon recognition of viral infection, the host innate immune system could respond within minutes to impede viral replication and limits the development of a chronic and persistent infection. The secretion of antiviral cytokines from infected hepatocytes and intra-hepatic immune cells is critically important for the clearance of viral infection. However, chronically infected patients only display a weak immune response due to various escape strategies adopted by the virus to counteract the host cell recognition systems and the subsequent antiviral responses.

Many observations showed that several HBV viral proteins could counteract the initial host cellular response by interfering with the viral recognition signaling system and subsequently the interferon (IFN) antiviral activity. Among these, the excessive secretion of HBV empty subviral particles (SVPs, HBsAg) may contribute to immune tolerant state observed in CHB patients. The persistent exposure to HBsAg and other viral antigens can lead to HBV-specific T-cell functional impairment and depletion (Kondo et al. Journal of immunology (1993), 150, 4659-4671; Kondo et al. Journal of Medical Virology (2004), 74, 425-433; Fisicaro et al. Gastroenterology, (2010), 138, 682-693;). Moreover, HBsAg has been reported to suppress immune cell functions, including monocytes, dendritic cells (DCs) and natural killer (NK) cells (Op den Brouw et al. Immunology, (2009b), 126, 280-289; Woltman et al. PLoS One, (2011), 6, e15324; Shi et al. J Viral Hepat. (2012), 19, e26-33; Kondo et al. ISRN Gasteroenterology, (2013), Article ID 935295).

HBsAg is an important biomarker for prognosis and treatment response in CHB. However, the achievement of HBsAg loss and seroconversion is rarely achieved in CHB patients. HBsAg loss with or without anti-HBsAg seroconversion remains the ideal clinical treatment endpoints. Current therapies, such as nucleos(t)ide analogues, are effective in supressing HBV DNA, but are not effective in reducing HBsAg level. Nucleos(t)ide analogues, even with prolonged therapy, have demonstrated HBsAg clearance rates comparable to those observed naturally (Janssen et al. Lancet, (2005), 365, 123-129; Marcellin et al. N. Engl. J. Med., (2004), 351, 1206-1217; Buster et al. Hepatology, (2007), 46, 388-394). Therefore, there is an urgent need for the development of novel therapeutic agents that could efficiently reduce HBsAg. (Wieland, S. F. & F. V. Chisari. J Virol, (2005), 79, 9369-9380; Kumar et al. J Virol, (2011), 85, 987-995; Woltman et al. PLoS One, (2011), 6, e15324; Op den Brouw et al. Immunology, (2009b), 126, 280-289).

Reviews about antiviral agents useful for the treatment of HBV infection are disclosed in Feng, S. et al., ACS Infectious Diseases, vol. 4, no. 3 (2018), p. 257-277 and Pei, Y. et al, J. Med. Chem., vol. 60, no. 15 (2017), p. 6461-6479.

### SUMMARY OF THE INVENTION

Objects of the present invention are novel compounds of formula (I), their manufacture, medicaments based on a compound in accordance with the invention and their production as well as the use of compounds of formula (I) as HBV inhibitors and for the treatment or prophylaxis of HBV infection. The compounds of formula (I) show superior anti-HBV activity. In addition, the compounds of formula (I) also show good safety and good PK profiles.

The references to methods of treatment in the summary and detailed description of the invention in this description are to be interpreted as references to the compounds, pharmaceutical compositions and medicaments of the present invention for use in a method for treatment of the human (or animal) body by therapy.

The present invention relates to a compound of formula (I) 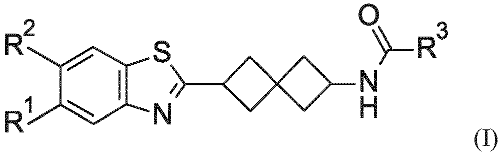 wherein
R¹ is H or halogen;
R² is H or halogen;
or R¹ and R² together with the phenyl they are attached to form a 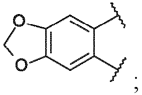
R³ is (1,1-dioxothian-3-yl)C₁₋₆alkyl;
   (1,1-dioxothiazinan-2-yl)C₁₋₆alkyl;
   (1,1-dioxothietan-3-yl)C₁₋₆alkyl;
   (1,1-dioxothiolan-2-yl)C₁₋₆alkyl;
   (5-oxopyrrolidin-2-yl)C₁₋₆alkyl;
   1,1-dioxo-1,2-thiazolidinyl;
   1,1-dioxo-thietanyl;
   1,1-dioxo-thiolanyl;
   aminobenzothiazolyl;
   C₁₋₆alkyl-2-oxo-piperidinyl;
   C₁₋₆alkyl-5-oxo-pyrrolidinyl;
   furanyl which is substituted by ((haloC₁₋₆alkyl)C₃₋₇cycloalkyl)sulfamoyl, (haloazetidinyl)sulfonyl, (haloC₃₋₇cycloalkyl)sulfamoyl, azetidin-1-ylsulfonyl, C₁₋₆alkylsulfonylC₁₋₆alkyl, C₃₋₇cycloalkylsulfamoyl, C₃₋₇cycloalkylsulfonyl, haloC₁₋₆alkyl or hydroxyhaloC₁₋₆alkyl;
   phenyl which is substituted by (C₁₋₆alkyl)₂phosphoryl, haloC₁₋₆alkyl or sulfamoyl;
   pyridinyl which is substituted by (C₁₋₆alkyl)₂amino, C₁₋₆alkoxy, C₁₋₆alkyl, C₁₋₆alkylamino, C₁₋₆alkylsulfonimidoyl, C₁₋₆alkylsulfonyl, C₁₋₆alkylsulfonylC₁₋₆alkyl, C₃₋₇cycloalkylamino, C₃₋₇cycloalkylC₁₋₆alkylsulfonyl, C₃₋₇cycloalkylsulfonimidoyl, C₃₋₇cycloalkylsulfonyl, carbamoyl, cyano, haloC₁₋₆alkyl, morpholinyl, piperidinyl, pyrrolidinyl or ureido; or
   pyrimidinyl which is substituted by carbamoyl;
with the proviso that R¹ and R² are not H or halogen simultaneously;
or a pharmaceutically acceptable salt thereof.

### DETAILED DESCRIPTION OF THE INVENTION

### DEFINITIONS

As used herein, the term "C₁₋₆alkyl" alone or in combination signifies a saturated, linear- or branched chain alkyl group containing 1 to 6, particularly 2 to 6 or 1 to 4 carbon atoms, for example methyl, ethyl, propyl, isopropyl, butyl, isobutyl, tert-butyl and the like. Particular "C₁₋₆alkyl" groups are methyl and ethyl.

The term "C₁₋₆alkoxy" alone or in combination signifies a group C₁₋₆alkyl-O-, wherein the "C₁₋₆alkyl" is as defined above; for example methoxy, ethoxy, propoxy, iso-propoxy, n-butoxy, iso-butoxy, 2-butoxy, tert-butoxy, pentoxy, hexyloxy and the like. Particular "C₁₋₆alkoxy" groups are methoxy and ethoxy and propoxy.

The term "halogen" denotes fluoro, chloro, bromo, or iodo.

The term "haloC₁₋₆alkyl" denotes an alkyl group wherein at least one of the hydrogen atoms of the alkyl group is replaced by same or different halogen atoms, particularly fluoro atoms. Examples of haloC₁₋₆alkyl include monochloro-, difluoro-or trifluoro-methyl, -ethyl or - propyl, for example difluoromethyl.

The term "C₃₋₇cycloalkyl" denotes to a saturated carbon ring containing from 3 to 7 carbon atoms, particularly from 3 to 6 carbon atoms, for example, cyclopropyl, cyclobutyl, cyclopentyl, cyclohexyl, cycloheptyl and the like. Particular "C₃₋₇cycloalkyl" group is cyclopropyl.

The term "carbonyl" alone or in combination refers to the group -C(O)-.

The term "sulfonyl" alone or in combination refers to the group -S(O)₂-.

The term "sulfonimidoyl" alone or in combination refers to the group -S(O)(NH)-, whose formula is

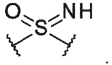

The term "bond" refers to a chemical bond between two atoms, or two moieties when the atoms joined by the bond are considered to be part of larger substructure. In one aspect, when a group described herein is a bond, the referenced group is absent thereby allowing a bond to be formed between the remaining identified groups.

The compounds according to the present invention may exist in the form of their pharmaceutically acceptable salts. The term "pharmaceutically acceptable salt" refers to conventional acid-addition salts or base-addition salts that retain the biological effectiveness and properties of the compounds of formula (I) and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flowability and solubility of compounds. It is for example described in Bastin R.J., et al., Organic Process Research & Development 2000, 4, 427-435. Particular are the sodium salts of the compounds of formula (I).

### HBV INHIBITORS

The present invention provides (i) a compound having the general formula (I): 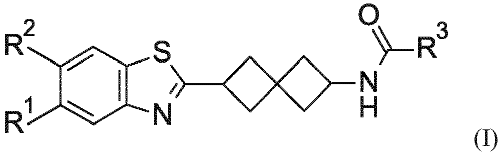 wherein
R¹ is H or halogen;
R² is H or halogen;
or R¹ and R² together with the phenyl they are attached to form a 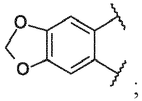
R³ is (1,1-dioxothian-3-yl)C₁₋₆alkyl;
   (1,1-dioxothiazinan-2-yl)C₁₋₆alkyl;
   (1,1-dioxothietan-3-yl)C₁₋₆alkyl;
   (1,1-dioxothiolan-2-yl)C₁-₆alkyl;
   (5-oxopyrrolidin-2-yl)C₁₋₆alkyl;
   1,1-dioxo-1,2-thiazolidinyl;
   1,1-dioxo-thietanyl;
   1,1-dioxo-thiolanyl;
   aminobenzothiazolyl;
   C₁₋₆alkyl-2-oxo-piperidinyl;
   C₁₋₆alkyl-5-oxo-pyrrolidinyl;
   furanyl which is substituted by ((haloC₁₋₆alkyl)C₃₋₇cycloalkyl)sulfamoyl, (haloazetidinyl)sulfonyl, (haloC₃₋₇cycloalkyl)sulfamoyl, azetidin-1-ylsulfonyl, C₁₋₆alkylsulfonylC₁₋₆alkyl, C₃₋₇cycloalkylsulfamoyl, C₃₋₇cycloalkylsulfonyl, haloC₁₋₆alkyl or hydroxyhaloC₁₋₆alkyl;
   phenyl which is substituted by (C₁₋₆alkyl)₂phosphoryl, haloC₁₋₆alkyl or sulfamoyl;
   pyridinyl which is substituted by (C₁₋₆alkyl)₂amino, C₁₋₆alkoxy, C₁₋₆alkyl, C₁₋₆alkylamino, C₁₋₆alkylsulfonimidoyl, C₁₋₆alkylsulfonyl, C₁₋₆alkylsulfonylC₁₋₆alkyl, C₃₋₇cycloalkylamino, C₃₋₇cycloalkylC₁₋₆alkylsulfonyl, C₃₋₇cycloalkylsulfonimidoyl, C₃₋₇cycloalkylsulfonyl, carbamoyl, cyano, haloC₁₋₆alkyl, morpholinyl, piperidinyl, pyrrolidinyl or ureido; or
   pyrimidinyl which is substituted by carbamoyl;
with the proviso that R¹ and R² are not H or halogen simultaneously;
or a pharmaceutically acceptable salt thereof.

A further embodiment of the present invention is (ii) a compound of formula (I) according to (i), wherein
R¹ is H or chloro;
R² is H or chloro;
or R¹ and R² together with the phenyl they are attached to form a 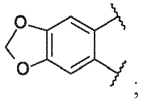
R³ is (1,1-dioxothian-3-yl)methyl;
   (1,1-dioxothiazinan-2-yl)methyl;
   (1,1-dioxothietan-3-yl)methyl;
   (1,1-dioxothiolan-2-yl)methyl;
   (5-oxopyrrolidin-2-yl)methyl;
   1,1-dioxo-1,2-thiazolidinyl;
   1,1-dioxo-thietanyl;
   1,1-dioxo-thiolanyl;
   aminobenzothiazolyl;
   1-ethyl-5-oxo-pyrrolidinyl;
   1-methyl-2-oxo-piperidinyl;
   ((3-(trifluoromethyl)cyclobutyl)sulfamoyl)furanyl;
   (1-methyl-1-methylsulfonyl-ethyl)furanyl;
   (1-methylsulfonylethyl)furanyl;
   (2,2,2-trifluoro-1-hydroxy-ethyl)furanyl,
   (3,3-difluoroazetidin-1-yl)sulfonylfuranyl;
   (azetidin-1-ylsulfonyl)furanyl;
   (cyclobutylsulfamoyl)furanyl;
   (trifluoromethyl)furanyl;
   [(3,3-difluorocyclobutyl)sulfamoyl]furanyl;
   cyclopropylsulfonylfuranyl;
   dimethylphosphorylphenyl;
   sulfamoylphenyl;
   trifluoromethylphenyl;
   (1-methyl-1-methylsulfonyl-ethyl)pyridinyl;
   (1-piperidinyl)pyridinyl;
   (cyclopropylamino)pyridinyl;
   (cyclopropylmethylsulfonyl)pyridinyl;
   (cyclopropylsulfonimidoyl)pyridinyl;
   (diethylamino)pyridinyl;
   (dimethylamino)pyridinyl;
   (methylamino)pyridinyl;
   (methylsulfonimidoyl)pyridinyl;
   (methylsulfonylmethyl)pyridinyl;
   [ethyl(methyl)amino]pyridinyl;
   [isopropyl(methyl)amino]pyridinyl;
   carbamoylpyridinyl;
   cyanopyridinyl;
   cyclopropylsulfonylpyridinyl;
   ethylsulfonylpyridinyl;
   isopropoxypyridinyl;
   mehtylpyridinyl;
   methoxypyridinyl;
   methylsulfonylpyridinyl;
   morpholinylpyridinyl;
   pyrrolidin-1-ylpyridinyl;
   tert-butylsulfonylpyridinyl;
   trifluoromethylpyridinyl;
   ureidopyridinyl; or
   carbamoylpyrimidinyl;
with the proviso that R¹ and R² are not H or halogen simultaneously;
   or a pharmaceutically acceptable salt thereof.

A further embodiment of the present invention is (iii) a compound of formula (I), or a pharmaceutically acceptable salt thereof, according to any one of (i) - (ii), wherein R¹ is halogen.

A further embodiment of the present invention is (iv) a compound of formula (I) according to any one of (i) - (iii), or a pharmaceutically acceptable salt thereof, wherein R¹ is chloro.

A further embodiment of the present invention is (v) a compound of formula (I) according to any one of (i) - (iv), or a pharmaceutically acceptable salt thereof, wherein R¹ is H.

A further embodiment of the present invention is (vi) a compound of formula (I) according to any one of (i) - (v), or a pharmaceutically acceptable salt thereof, wherein R³ is furanyl which is substituted by C₃₋₇cycloalkylsulfonyl; or pyridinyl which is substituted by C₁₋₆alkylsulfonyl, C₃₋₇cycloalkylC₁₋₆alkylsulfonyl, C₃₋₇cycloalkylsulfonimidoyl, C₃₋₇cycloalkylsulfonyl or carbamoyl.

A further embodiment of the present invention is (vii) a compound of formula (I) according to any one of (i) - (vi), or a pharmaceutically acceptable salt thereof, wherein R³ is cyclopropylsulfonylfuranyl, cyclopropylsulfonylpyridinyl, (cyclopropylmethylsulfonyl)pyridinyl, (cyclopropylsulfonimidoyl)pyridinyl, carbamoylpyridinyl or methylsulfonylpyridinyl.

A further embodiment of the present invention is (viii) a compound of formula (I) according to any one of (i) - (vii), or a pharmaceutically acceptable salt thereof, wherein
R¹ is halogen;
R¹ is H;
R³ is furanyl which is substituted by C₃₋₇cycloalkylsulfonyl; or
   pyridinyl which is substituted by C₁₋₆alkylsulfonyl, C₃₋₇cycloalkylC₁₋₆alkylsulfonyl, C₃₋₇cycloalkylsulfonimidoyl, C₃₋₇cycloalkylsulfonyl or carbamoyl;
or a pharmaceutically acceptable salt thereof.

A further embodiment of the present invention is (ix) a compound of formula (I) according to any one of (i) - (viii), or a pharmaceutically acceptable salt thereof, wherein
R¹ is chloro;
R² is H;
R³ is cyclopropylsulfonylfuranyl, cyclopropylsulfonylpyridinyl, (cyclopropylmethylsulfonyl)pyridinyl, (cyclopropylsulfonimidoyl)pyridinyl, carbamoylpyridinyl or methylsulfonylpyridinyl;
or a pharmaceutically acceptable salt thereof.

In another embodiment (x) of the present invention, particular compounds of the present invention are selected from:
*N*-[6-(5-chloro-1,3-benzothiazol-2-yl)spiro[3.3]heptan-2-yl]-3-(trifluoromethyl)benzamide;
*N*-[6-(5-chloro-1,3-benzothiazol-2-yl)spiro[3.3]heptan-2-yl]-3-sulfamoyl-benzamide;
*N*-[6-(5-chloro-1,3-benzothiazol-2-yl)spiro[3.3]heptan-2-yl]-3-dimethylphosphoryl-benzamide;
*N*-[6-(5-chloro-1,3-benzothiazol-2-yl)spiro[3.3]heptan-2-yl]-2-(trifluoromethyl)pyridine-4-carboxamide;
*N*-[6-(5-chloro-1,3-benzothiazol-2-yl)spiro[3.3]heptan-2-yl]-2-cyano-pyridine-4-carboxamide;
*N*-[6-(5-chloro-1,3-benzothiazol-2-yl)spiro[3.3]heptan-2-yl]-2-methyl-pyridine-4-carboxamide;
*N*-[6-(5-chloro-1,3-benzothiazol-2-yl)spiro[3.3]heptan-2-yl]-2-(methylsulfonylmethyl)pyridine-4-carboxamide;
*N*-[6-(5-chloro-1,3-benzothiazol-2-yl)spiro[3.3]heptan-2-yl]-2-(1-methyl-1-methylsulfonylethyl)pyridine-4-carboxamide;
*N*-[6-(5-chloro-1,3-benzothiazol-2-yl)spiro[3.3]heptan-2-yl]-2-ethylsulfonyl-pyridine-4-carboxamide;
*N*-[6-(5-chloro-1,3-benzothiazol-2-yl)spiro[3.3]heptan-2-yl]-2-ureido-pyridine-4-carboxamide;
*N4*-[6-(5-chloro-1,3-benzothiazol-2-yl)spiro[3.3]heptan-2-yl]pyridine-2,4-dicarboxamide;
*N4*-[6-([1,3]dioxolo[4,5-f][1,3]benzothiazol-6-yl)spiro[3.3]heptan-2-yl]pyridine-2,4-dicarboxamide;
*N*-[6-(5-chloro-l,3-benzothiazol-2-yl)spiro[3.3]heptan-2-yl]-5-cyclopropylsulfonyl-furan-2-carboxamide;
*N*-[6-(5-chloro-1,3-benzothiazol-2-yl)spiro[3.3]heptan-2-yl]-5-(trifluoromethyl)furan-2-carboxamide;
*N*-[6-(6-chloro-1,3-benzothiazol-2-yl)spiro[3.3]heptan-2-yl]-5-(trifluoromethyl)furan-2-carboxamide;
*N*-[6-(5-chloro-1,3-benzothiazol-2-yl)spiro[3.3]heptan-2-yl]-5-(1-methylsulfonylethyl)furan-2-carboxamide;
*N*-[6-(5-chloro-1,3-benzothiazol-2-yl)spiro[3.3]heptan-2-yl]-5-(1-methyl-1-methylsulfonylethyl)furan-2-carboxamide;
*N*-[6-(5-chloro-1,3-benzothiazol-2-yl)spiro[3.3]heptan-2-yl]-5-(2,2,2-trifluoro-1-hydroxyethyl)furan-2-carboxamide;
2-amino-*N*-[6-(5-chloro-1,3-benzothiazol-2-yl)spiro[3.3]heptan-2-yl]thiazole-4-carboxamide;
5-(azetidin-1-ylsulfonyl)-*N*-[6-(5-chloro-1,3-benzothiazol-2-yl)spiro[3.3]heptan-2-yl]furan-2-carboxamide;
*N*-[6-(5-chloro-1,3-benzothiazol-2-yl)spiro[3.3]heptan-2-yl]-5-(3,3-difluoroazetidin-1-yl)sulfonyl-furan-2-carboxamide;
*N*-[6-(5-chloro-1,3-benzothiazol-2-yl)spiro[3.3]heptan-2-yl]-5-(cyclobutylsulfamoyl)furan-2-carboxamide;
*N*-[6-(5-chloro-1,3-benzothiazol-2-yl)spiro[3.3]heptan-2-yl]-5-[(3,3-difluorocyclobutyl)sulfamoyl]furan-2-carboxamide;
*N*-[6-(5-chloro-1,3-benzothiazol-2-yl)spiro[3.3]heptan-2-yl]-5-[[3-(trifluoromethyl)cyclobutyl] sulfamoyl]furan-2-carboxamide;
*N*-[6-(5-chloro-1,3-benzothiazol-2-yl)spiro[3.3]heptan-2-yl]-1,1-dioxo-thietane-3-carboxamide;
*N*-[6-(5-chloro-1,3-benzothiazol-2-yl)spiro[3.3]heptan-2-yl]-1,1-dioxo-thiolane-3-carboxamide;
*N*-[6-(6-chloro-1,3-benzothiazol-2-yl)spiro[3.3]heptan-2-yl]-1,1-dioxo-thiolane-3-carboxamide;
*N*-[6-(5-chloro-1,3-benzothiazol-2-yl)spiro[3.3]heptan-2-yl]-2-(1,1-dioxothietan-3-yl)acetamide;
*N*-[6-(5-chloro-1,3-benzothiazol-2-yl)spiro[3.3]heptan-2-yl]-2-(1,1-dioxothiolan-2-yl)acetamide;
*N*-[6-(5-chloro-1,3-benzothiazol-2-yl)spiro[3.3]heptan-2-yl]-2-(1,1-dioxothian-3-yl)acetamide;
*N*-[6-(5-chloro-1,3-benzothiazol-2-yl)spiro[3.3]heptan-2-yl]-1-ethyl-5-oxo-pyrrolidine-3-carboxamide;
*N-*[6-(5-chloro-1,3-benzothiazol-2-yl)spiro[3.3]heptan-2-yl]-1-methyl-2-oxo-piperidine-4-carboxamide;
*N*-[6-(5-chloro-1,3-benzothiazol-2-yl)spiro[3.3]heptan-2-yl]-2-(5-oxopyrrolidin-2-yl)acetamide;
*N*-[6-(5-chloro-1,3-benzothiazol-2-yl)spiro[3.3]heptan-2-yl]-1,1-dioxo-1,2-thiazolidine-3-carboxamide;
*N*-[6-(5-chloro-1,3-benzothiazol-2-yl)spiro[3.3]heptan-2-yl]-2-(1,1-dioxothiazinan-2-yl)acetamide;
*N-*[6-(5-chloro-1,3-benzothiazol-2-yl)spiro[3.3]heptan-2-yl]-2-methylsulfonyl-pyridine-4-carboxamide;
*N*-[6-(6-chloro-1,3-benzothiazol-2-yl)spiro[3.3]heptan-2-yl]-2-cyclopropylsulfonyl-pyridine-4-carboxamide;
2-*tert*-butylsulfonyl-*N*-[6-(5-chloro-1,3-benzothiazol-2-yl)spiro[3.3]heptan-2-yl]pyridine-4-carboxamide;
*N*-[6-(5-chloro-1,3-benzothiazol-2-yl)spiro[3.3]heptan-2-yl]-2-(cyclopropylmethylsulfonyl)pyridine-4-carboxamide;
*N-*[6-(5-chloro-1,3-benzothiazol-2-yl)spiro[3.3]heptan-2-yl]-2-(methylsulfonimidoyl)pyridine-4-carboxamide;
*N*-[6-(5-chloro-1,3-benzothiazol-2-yl)spiro[3.3]heptan-2-yl]-2-(cyclopropylsulfonimidoyl)pyridine-4-carboxamide;
*N*-[6-(5-chloro-1,3-benzothiazol-2-yl)spiro[3.3]heptan-2-yl]-2-methoxy-pyridine-4-carboxamide;
*N*-[6-(5-chloro-1,3-benzothiazol-2-yl)spiro[3.3]heptan-2-yl]-2-isopropoxy-pyridine-4-carboxamide;
*N*-[6-(5-chloro-1,3-benzothiazol-2-yl)spiro[3.3]heptan-2-yl]-2-(methylamino)pyridine-4-carboxamide;
*N*-[6-(5-chloro-1,3-benzothiazol-2-yl)spiro[3.3]heptan-2-yl]-2-(cyclopropylamino)pyridine-4-carboxamid;
*N*-[6-(5-chloro-1,3-benzothiazol-2-yl)spiro[3.3]heptan-2-yl]-2-(dimethylamino)pyridine-4-carboxamide;
*N*-[6-(5-chloro-1,3-benzothiazol-2-yl)spiro[3.3]heptan-2-yl]-2-[ethyl(methyl)amino]pyridine-4-carboxamide;
*N*-[6-(5-chloro-1,3-benzothiazol-2-yl)spiro[3.3]heptan-2-yl]-2-(diethylamino)pyridine-4-carboxamide;
*N*-[6-(5-chloro-1,3-benzothiazol-2-yl)spiro[3.3]heptan-2-yl]-2-[isopropyl(methyl)amino]pyridine-4-carboxamide;
*N*-[6-(5-chloro-1,3-benzothiazol-2-yl)spiro[3.3]heptan-2-yl]-2-pyrrolidin-1-yl-pyridine-4-carboxamide;
*N*-[6-(5-chloro-1,3-benzothiazol-2-yl)spiro[3.3]heptan-2-yl]-2-(1-piperidyl)pyridine-4-carboxamide;
*N*-[6-(5-chloro-1,3-benzothiazol-2-yl)spiro[3.3]heptan-2-yl]-2-morpholino-pyridine-4-carboxamide;
(*S*ₐ)-*N4*-[6-(5-chloro-1,3-benzothiazol-2-yl)spiro[3.3]heptan-2-yl]pyridine-2,4-dicarboxamide;
(*Rₐ*)-*N4*-[6-(5-chloro-1,3-benzothiazol-2-yl)spiro[3.3]heptan-2-yl]pyridine-2,4-dicarboxamide;
(*S*ₐ)-*N*-[6-(5-chloro-1,3-benzothiazol-2-yl)spiro[3.3]heptan-2-yl]-2-methylsulfonyl-pyridine-4-carboxamide;
(*Rₐ*)-*N*-[6-(5-chloro-1,3-benzothiazol-2-yl)spiro[3.3]heptan-2-yl]-2-methylsulfonyl-pyridine-4-carboxamide;
*N*-[6-(5-chloro-1,3-benzothiazol-2-yl)spiro[3.3]heptan-2-yl]-2-cyclopropylsulfonyl-pyridine-4-carboxamide;
(*S*ₐ)-*N*-[6-(5-chloro-1,3-benzothiazol-2-yl)spiro[3.3]heptan-2-yl]-2-cyclopropylsulfonyl-pyridine-4-carboxamide;
(*Rₐ*)-*N*-[6-(5-chloro-1,3-benzothiazol-2-yl)spiro[3.3]heptan-2-yl]-2-cyclopropylsulfonyl-pyridine-4-carboxamide;
*N6*-[6-(5-chloro-1,3-benzothiazol-2-yl)spiro[3.3]heptan-2-yl]pyrimidine-4,6-dicarboxamide; (*S*ₐ)-*N6*-[6-(5-chloro-1,3-benzothiazol-2-yl)spiro[3.3]heptan-2-yl]pyrimidine-4,6-dicarboxamide; and
(*Rₐ*)-*N6*-[6-(5-chloro-1,3-benzothiazol-2-yl)spiro[3.3]heptan-2-yl]pyrimidine-4,6-dicarboxamide; or a pharmaceutically acceptable salt thereof.

### SYNTHESIS

The compounds of the present invention can be prepared by any conventional means. Suitable processes for synthesizing these compounds as well as their starting materials are provided in the schemes below and in the examples. All substituents, in particular, R¹ to R³ are as defined above unless otherwise indicated. Furthermore, and unless explicitly otherwise stated, all reactions, reaction conditions, abbreviations and symbols have the meanings well known to a person of ordinary skill in organic chemistry.

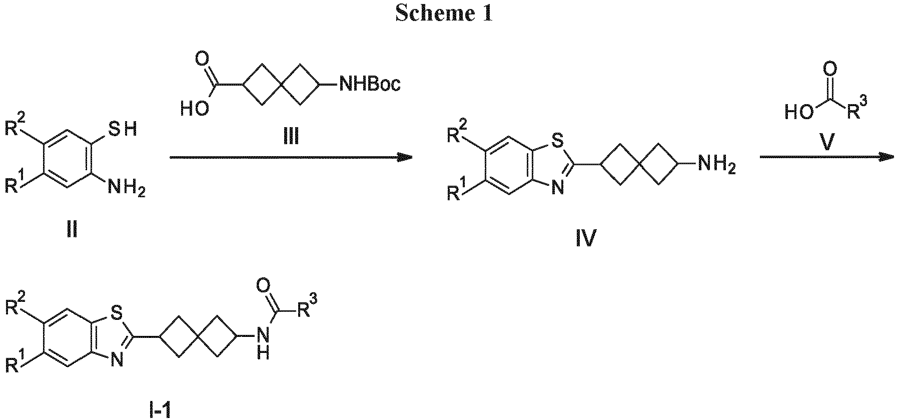

Compound of formula **(II)** is heated with a carboxylic acid **(III)** in the presence of an acid, such as polyphosphoric acid, to give compound of formula **(IV),** which then reacts with compound of formula **(V)** in the presence of a coupling reagent, such as HATU or T₃P, and a base such as TEA or DIPEA, in a solvent such as DMF or DCM, to afford compound of formula **(I-1).** 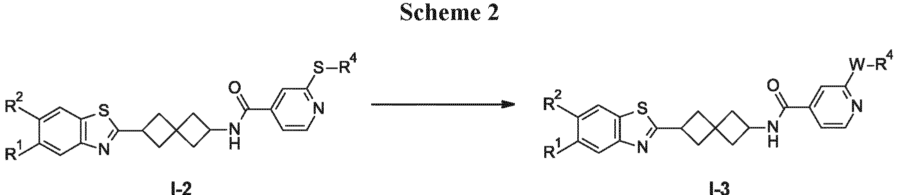 wherein R⁴ is C₁₋₆alkyl, C₃₋₇cycloalkylC₁₋₆alkyl, or C₃₋₇cycloalkyl; W is S(O)₂ or S(O)(NH).

Compound of formula **(I-2)** is prepared according to Scheme 1. Oxidation of compound of formula **(I-2)** in the presence of an oxidant, such as Oxone, or PhI(OAc)₂ and (NH₄)₂CO₃, in a suitable solvent, such as MeOH or DCM, affords compound of formula **(I-3).** 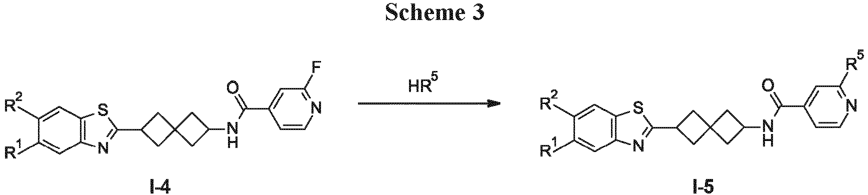 wherein R⁵ is C₁₋₆alkoxy, C₁₋₆alkylamino, (C₁₋₆alkyl)₂amino, C₃₋₇cycloalkylamino, morpholinyl, piperidinyl or pyrrolidinyl.

Compound of formula **(I-4)** is prepared according to Scheme 1. Substitution of compound of formula **(I-4)** with an alcohol or amine (compound HR⁵) in the presence of a base, such as NaH or Cs₂CO₃, in a suitable solvent, such as THF or DMF, affords compound of formula **(I-5).**

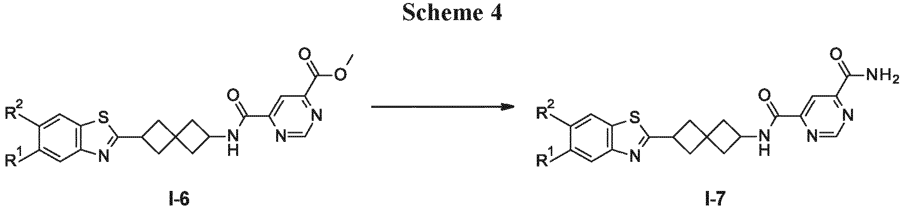

Compound of formula **(I-6)** is prepared according to Scheme 1. Amination of compound of formula **(I-6)** in a solution of ammonia in methanol affords compound of formula **(I-7).**

This invention also relates to a process for the preparation of a compound of formula (I) comprising any one or more of the following steps:
(a) Reaction of a compound of formula (IV), 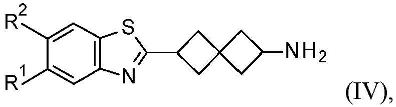 with a compound of formula (V), 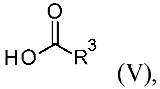 in the presence of a coupling reagent, such as HATU or T₃P;
(b) Reaction of a compound of formula (I-2), 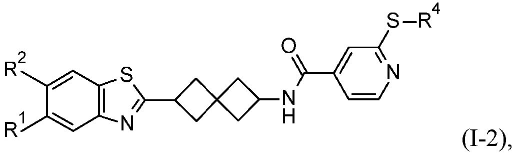 with an oxidant, such as Oxone, or PhI(OAc)₂ and (NH₄)₂CO₃; wherein R⁴ is C₁₋₆alkyl, C₃₋₇cycloalkylC₁₋₆alkyl, or C₃₋₇cycloalkyl; W is S(O)₂ or S(O)(NH);
(c) Reaction of a compound of formula (I-4), 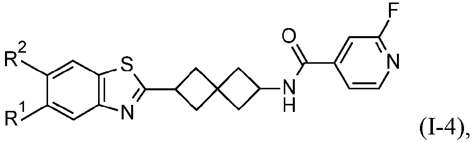 with an alcohol or amine (compound HR⁵) in the presence of a base, such as NaH or Cs₂CO₃; wherein R⁵ is C₁₋₆alkoxy, C₁₋₆alkylamino, (C₁₋₆alkyl)₂amino, C₃₋₇cycloalkylamino, morpholinyl, piperidinyl or pyrrolidinyl;
(d) Amination of a compound of formula (I-6), 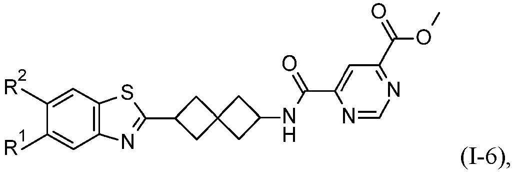 in a solution of ammonia in methanol.

A compound of formula (I) when manufactured according to the above process is also an object of the invention.

### PHARMACEUTICAL COMPOSITIONS AND ADMINISTRATION

The invention also relates to a compound of formula (I) for use as therapeutically active substance. Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compounds of formula (I) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to reduction of HBsAg and HBeAg in HBV patients. For example, such amount may be below the amount that is toxic to normal cells, or the mammal as a whole.

In one example, the pharmaceutically effective amount of the compound of the invention administered parenterally per dose will be in the range of about 0.1 to 100 mg/kg, alternatively about 0.1 to 50 mg/kg of patient body weight per day, with the typical initial range of compound used being 0.3 to 15 mg/kg/day. In another embodiment, oral unit dosage forms, such as tablets and capsules, preferably contain from about 25 to about 1000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, *e.g.,* Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 25 to 500 mg of the compound of the invention compounded with about 90 to 30 mg anhydrous lactose, about 5 to 40 mg sodium croscarmellose, about 5 to 30 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 400 mg, of the invention in a suitable buffer solution, *e.g.* a phosphate buffer, adding a tonicifier, *e.g.* a salt such sodium chloride, if desired. The solution may be filtered, *e.g.,* using a 0.2 micron filter, to remove impurities and contaminants.

An embodiment, therefore, includes a pharmaceutical composition comprising a compound of formula (I), or a pharmaceutically acceptable salt thereof.

In a further embodiment includes a pharmaceutical composition comprising a compound of formula (I), or a pharmaceutically acceptable salt thereof, together with a pharmaceutically acceptable carrier or excipient.

Another embodiment includes a pharmaceutical composition comprising a compound of formula (I), or a pharmaceutically acceptable salt thereof for use in the treatment of HBV infection.

### INDICATIONS AND METHODS OF TREATMENT

The references to methods of treatment are to be interpreted as references to the compounds, pharmaceutical compositions and medicaments of the present invention for use in a method for treatment of the human (or animal) body by therapy.

The compounds of the invention have anti-HBV activity. Accordingly, the compounds of the invention are useful for the treatment or prophylaxis of HBV infection.

The invention also relates to the use of a compound of formula (I) for the inhibition of HBeAg.

The invention further relates to the use of a compound of formula (I) for the inhibition of HBsAg.

The invention relates to the use of a compound of formula (I) for the inhibition of HBV DNA.

The invention relates to the use of a compound of formula (I) for use in the treatment or prophylaxis of HBV infection.

The use of a compound of formula (I) for the preparation of medicaments useful in the treatment or prophylaxis diseases that are related to HBV infection is an object of the invention.

The invention relates in particular to the use of a compound of formula (I) for the preparation of a medicament for the treatment or prophylaxis of HBV infection.

Another embodiment includes a method for the treatment or prophylaxis of HBV infection, which method comprises administering an effective amount of a compound of formula (I), or a pharmaceutically acceptable salt thereof.

The invention relates in particular to a compound of formula (I) for use in the treatment or prophylaxis of HBV infection.

### EXAMPLES

The invention will be more fully understood by reference to the following examples.

### ABBREVIATIONS

- ACN: acetonitrile
- CDCl₃: deuterated chloroform
- CD₃OD: deuterated methanol
- DIEA: N,N-diisopropylethylamine
- DMF: dimethylformamide
- DMSO-d6: deuterated dimethylsulfoxide
- HATU: O-(7-aza-1H-benzotriazole-1-yl)-N,N,N',N'-tetramethyluronium hexafluorophosphate
- HPLC: high performance liquid chromatography
- h: hour
- IC50: the half maximal inhibitory concentration
- LC-MS: liquid chromatography-mass spectrometry
- M: molarity
- MHz: megahertz
- MS (ESI): mass spectroscopy (electron spray ionization)
- obsd.: observed
- SFC: supercritical fluid chromatography
- TEA: triethylamine
- TFA: trifluoroacetic acid
- THF: tetrahydrofuran
- T₃P: 2,4,6-tripropyl-1,3,5,2,4,6-trioxatriphosphinane 2,4,6-trioxide
- δ: chemical shift

### GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel Brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using X Bridge^{™} Perp C₁₈ (5 µm, OBD^{™} 30 × 100 mm) column or SunFire^{™} Perp C₁₈ (5 µm, OBD^{™} 30 × 100 mm) column.

Chiral Separation was conducted on Thar 350 preparative SFC using ChiralPak AD-10µ (200 × 50 mm I.D.) with mobile phase A for CO₂ and B for ethanol.LC/MS spectra were obtained using a Waters UPLC-SQD Mass. Standard LC/MS conditions were as follows (running time: 3 minutes):
Acidic condition: A: 0.1% formic acid and 1% acetonitrile in H₂O; B: 0.1% formic acid in acetonitrile;
Basic condition: A: 0.05% NH₃·H₂O in H₂O; B: acetonitrile.
Mass spectra (MS): generally only ions which indicate the parent mass are reported, and unless otherwise stated the mass ion quoted is the positive mass ion (M+H)⁺.

NMR Spectra were obtained using Bruker Avance 400MHz.

All reactions involving air-sensitive reagents were performed under an argon atmosphere. Reagents were used as received from commercial suppliers without further purification unless otherwise noted.

### PREPARATIVE EXAMPLES

### Intermediate Int-1

### 3-dimethylphosphorylbenzoic acid

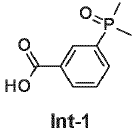

The title compound was prepared according to the following scheme:

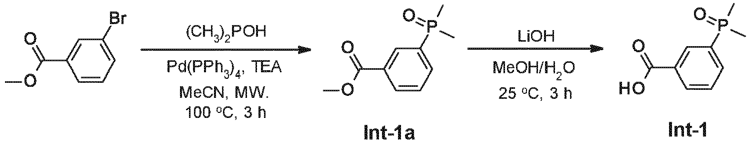

### Step 1: Preparation of methyl 3-dimethylphosphorylbenzoate (Int-1a)

To a solution of methyl 3-bromobenzoate (1.08 g) and hydroxydimethylphosphane (390 mg) in MeCN (10 ml) was added Pd(Ph₃P)₄ (0.25 g) and TEA (1.82 g, 2.5 ml). The mixture was heated with stirring under microwave irradiation at 100 °C for 3 h. The reaction mixture was concentrated and the residue was purified by flash column (eluting with 0-20% MeOH/DCM) to afford the product **(Int-1a,** 0.64 g).

### Step 2: Preparation of 3-dimethylphosphorylbenzoic acid (Int-1)

To a solution of methyl 3-dimethylphosphorylbenzoate (0.64 g) in MeOH (10 mL) and water (5 mL) was added LiOH (722 mg). After being stirred at 25 °C for 3 h, the reaction mixture was concentrated under reduced pressure to remove the organic solvent. The residue was acidified by HCl (1 M) to pH = 2 and extracted with DCM (50 mL × 3). The combined organic layer was dried over Na₂SO₄, filtered and concentrated *in vacuo* to give 3-dimethylphosphorylbenzoic acid (**Int-1,** 598 mg).

### Intermediate Int-2

### [2-(methylsulfonylmethyl)pyridine-4-carbonyl] oxylithium

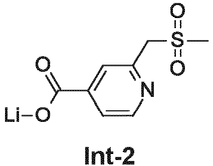

The title compound was prepared according to the following scheme:

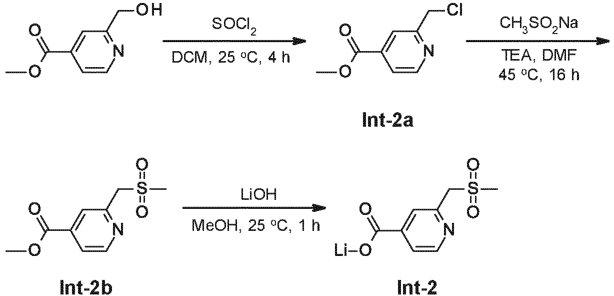

### Step 1: Preparation of methyl 2-(chloromethyl)pyridine-4-carboxylate (Int-2a)

To a solution of methyl 2-(hydroxymethyl)pyridine-4-carboxylate (1.0 g) in DCM (25 mL) was added thionyl chloride (0.87 mL). The reaction mixture was stirred at ambient temperature for 4 h and then concentrated under reduced pressure to afford methyl 2-(chloromethyl)pyridine-4-carboxylate **(Int-2a,** 1110 mg) as a light yellow solid. MS obsd. (ESI⁺) [(M+H)⁺]: 186.0.

### Step 2: Preparation of methyl 2-(methylsulfonylmethyl)pyridine-4-carboxylate (Int-2b)

To a solution of methylsulfinyloxysodium (1.28 g) in DMF (25 mL) was added methyl 2-(chloromethyl)pyridine-4-carboxylate **(Int-2a,** 1.56 g). The reaction mixture was stirred at 45 °C for 16 h. The resulted solution was diluted with ethyl acetate (80 mL), and then adjusted to pH = 2~3 with HCl aqueous solution (1 M). The separated organic phase was washed with brine (80 mL) and dried over anhydrous sodium sulfate and then concentrated under reduced pressure. The residue was purified by flash column (eluting with EtOAc/PE = 50/50) to give methyl 2-(methylsulfonylmethyl)pyridine-4-carboxylate **(Int-2b,** 1.55 g) as a brown solid. MS obsd. (ESI⁺) [(M+H)⁺]: 230.1.

### Step 3: Preparation of [2-(methylsulfonylmethyl)pyridine-4-carbonyl]oxylithium (Int-2)

To a solution of methyl 2-(methylsulfonylmethyl)pyridine-4-carboxylate **(Int-2b,** 100.0 mg) in MeOH (5 mL) was added lithium hydroxide (42 mg). The reaction mixture was stirred at ambient temperature for 1 h and then concentrated under reduced pressure to give the product [2-(methylsulfonylmethyl)pyridine-4-carbonyl]oxylithium (**Int-2,** 94 mg) as an off white solid. MS obsd. (ESI⁺) [(M+H)⁺]: 216.1.

### Intermediate Int-3

### 2-(1-methyl-1-methylsulfonyl-ethyl)pyridine-4-carboxylic acid

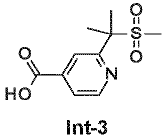

The title compound was prepared according to the following scheme:

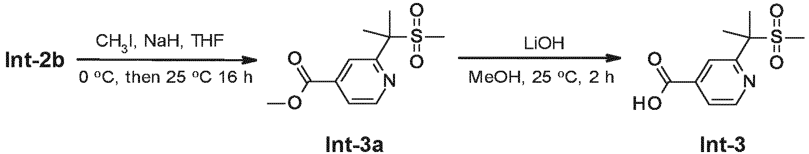

### Step 1: Preparation of methyl 2-(1-methyl-1-methylsulfonyl-ethyl)pyridine-4-carboxylate (Int-3a)

To a solution of methyl 2-(methylsulfonylmethyl)pyridine-4-carboxylate (**Int-2b,** 4.7 g) in THF (150 mL) was added sodium hydride (820.07 mg) at 0 °C. After being stirred at 0 °C for 30 min, iodomethane (2.55 mL) was added slowly. The resulting mixture was stirred for another 2 h at 25 °C. The reaction was quenched with MeOH (15 mL). The resulting solution was concentrated under reduced pressure. The residue was purified by flash column (eluting with EtOAc/PE = 70/30) to give the product methyl 2-(1-methylsulfonylethyl)pyridine-4-carboxylate (2.18 g) as a light yellow solid. MS obsd. (ESI⁺) [(M+H)⁺]: 244.1.

To a solution of methyl 2-(1-methylsulfonylethyl)pyridine-4-carboxylate (2.0 g) in THF (40 mL) was added sodium hydride (394.61 mg) at 0 °C. After being stirred at 0 °C for 30 min, iodomethane (1.02 mL, 16.44 mmol) was added at 0 °C dropwise, followed by stirring at 25 °C for 16 h. The reaction was quenched with MeOH (15 mL). The resulting solution was concentrated under reduced pressure to give the product methyl 2-(1-methyl-1-methylsulfonyl-ethyl)pyridine-4-carboxylate (**Int-3a,** 1.65 g) as a light yellow solid, which was used in next step directly without any purification. MS obsd. (ESI⁺) [(M+H)⁺]: 258.1.

### Step 2: Preparation of 2-(1-methyl-1-methylsulfonyl-ethyl)pyridine-4-carboxylic acid (Int-3)

To a solution of methyl 2-(1-methyl-1-methylsulfonyl-ethyl)pyridine-4-carboxylate (**Int-3a,** 1.65 g) in methanol (30 mL) was added lithium hydroxide (384 mg). The reaction mixture was stirred at ambient temperature for 2 h. The resulting solution was concentrated under reduced pressure. The residue was purified by prep-HPLC [columns: Kromasil-C18 100 × 21.2 mm, 5 µm; Mobile Phase: ACN-H₂O (0.1% FA), Gradient: 15%-25%] to give 2-(1-methyl-1-methylsulfonyl-ethyl)pyridine-4-carboxylic acid **(Int-3,** 605.7 mg) as a light yellow solid. MS obsd. (ESI⁺) [(M+H)⁺]: 244.1. ¹H NMR (400 MHz, DMSO-*d₆*) δ ppm: 13.85 (s, 1H), 8.83 (d, *J*= 4.8 Hz, 1H), 8.06 (s, 1H), 7.84 (d, *J* = 4.8 Hz, 1H), 2.83 (s, 3H), 1.82 (s, 6H).

### Intermediate Int-4

### 2-ethylsulfonylpyridine-4-carboxylic acid

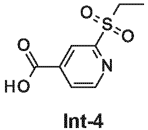

The title compound was prepared according to the following scheme:

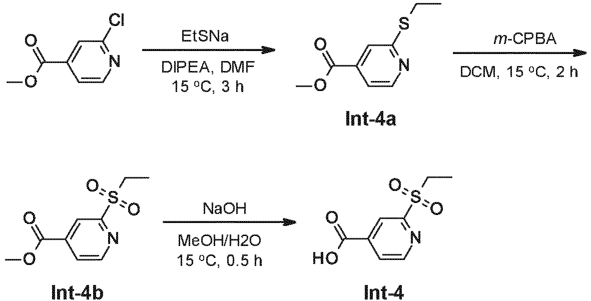

### Step 1: Preparation of methyl 2-ethylsulfanylpyridine-4-carboxylate (Int-4a)

To a solution of methyl 2-chloroisonicotinate (8.0 g) in DMF (80 mL) was added ethylsulfanylsodium (4.8 g) and *N,N-*diisopropylethylamine (12.8 g), then the solution was stirred at 15 °C for 3 h. The reaction mixture was poured into water (300 mL) and extracted with EtOAc (80 mL × 2). The organic layers were combined and dried over anhydrous Na₂SO₄. Then the crude material was purified by silica gel chromatography with EtOAc in Petroleum ether from 2% to 10% to give methyl 2-ethylsulfanylpyridine-4-carboxylate **(Int-4a,** 2.45 g) as a colorless liquid. MS obsd. (ESI⁺) [(M+H)⁺]: 197.8. ¹H NMR (400 MHz, DMSO-*d₆*) *δ* ppm: 8.62 (d, *J=* 5.2 Hz, 1H), 7.62 (s, 1H), 7.49 (d, *J* = 5.2 Hz, 1H), 3.87 (s, 3H), 3.15 (q, *J* = 7.6 Hz, 2H), 1.29 (t, *J=* 7.6 Hz, 3H).

### Step 2: Preparation of methyl 2-ethylsulfonylpyridine-4-carboxylate (Int-4b)

To a solution of methyl 2-ethylsulfanylpyridine-4-carboxylate (2.4 g) in DCM (50 mL) was added 3-chloroperoxybenzoic acid (5.28 g), then the reaction mixture was stirred at 15 °C for 2 h. The reaction mixture was diluted with DCM (50 mL), washed with sat. Na₂CO₃ (80 mL × 2), brine (80 mL × 2). The organic layer was dried over anhydrous Na₂SO₄, filtered and concentrated to give a crude material, which was purified by silica gel chromatography with EtOAc in Petroleum ether from 10% to 70% to give methyl 2-ethylsulfonylpyridine-4-carboxylate (**Int-4b,** 2.65 g) as a colorless gum. MS obsd. (ESI⁺) [(M+H)⁺]: 229.8. ¹H NMR (400 MHz, DMSO-*d₆*) *δ* ppm: 9.03 (d, *J* = 4.8 Hz, 1H), 8.33 (s, 1H), 8.19 (d, *J* = 4.8 Hz, 1H), 3.95 (s, 3H), 3.49 (q, *J* = 7.6 Hz, 2H), 1.42 (t, *J* = 7.6 Hz, 3H).

### Step 3: Preparation of 2-ethylsulfonylpyridine-4-carboxylic acid (Int-4)

To a solution of methyl 2-ethylsulfonylpyridine-4-carboxylate (2.65 g) in MeOH (25 mL) was added sodium hydroxide aqueous solution (6.0 mL, 2 N), then the resulting solution was stirred at 15 °C for 0.5 h. The reaction mixture was concentrated to remove the organic solvent and the residual was acidified with HCl (2 N) to pH = 3~4. The solid was collected by filtration and the filtrate was extracted with EtOAc (80 mL × 3). The organic layer was washed with brine (80 mL × 3), dried over anhydrous Na₂SO₄ and filtered. The combined filtrate and solid was concentrated to give 2-ethylsulfonylpyridine-4-carboxylic acid (**Int-4,** 2.35 g) as a white solid. MS obsd. (ESI⁺) [(M+H)⁺]: 215.8. ¹H NMR (400 MHz, DMSO-*d*₆) & ppm: 14.20 (s, 1H), 9.01 (d, *J* = 4.0 Hz, 1H), 8.32 (s, 1H), 8.16 (d, *J* = 4.0 Hz, 1H), 3.49 (q, *J* = 7.6 Hz, 2H), 1.43 (t, *J* = 7.6 Hz, 3H).

### Intermediate Int-5

### 5-cyclopropylsulfonylfuran-2-carboxylic acid

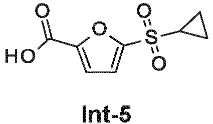

The title compound was prepared according to the following scheme:

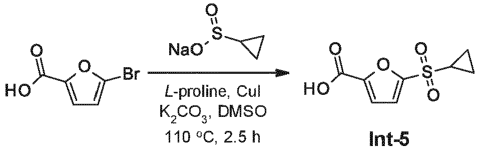

The mixture of 5-bromo-2-furoic acid (400.0 mg), cyclopropylsulfinyloxysodium (644.07 mg), 2-pyrrolidinecarboxylic acid (96.45 mg), potassium carbonate (115.79 mg) and copper(I) iodide (159.56 mg) was stirred in DMSO (20 mL) at 110 °C for 2.5 h. The resulting solution was adjusted pH = 3~4 with HCl (5 M) aqueous solution and extracted with ethyl acetate (50 mL × 3). The combined organic layer was washed with brine (50 mL × 3), dried over sodium sulfate and concentrated under reduced pressure. The residue was purified by flash column (eluting with EtOAc/PE = 50/50) to give 5-cyclopropylsulfonylfuran-2-carboxylic acid (**Int-5,** 210 mg) as a light yellow solid. MS obsd. (ESI⁺) [(M+H)⁺]: 217.0.

### Intermediate Int-6 and Int-7

### 5-(1-methylsulfonylethyl)furan-2-carboxylic acid & 5-(1-methyl-1-methylsulfonylethyl)furan-2-carboxylic acid

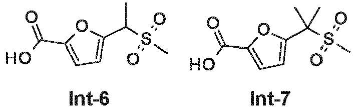

The title compounds were prepared according to the following scheme:

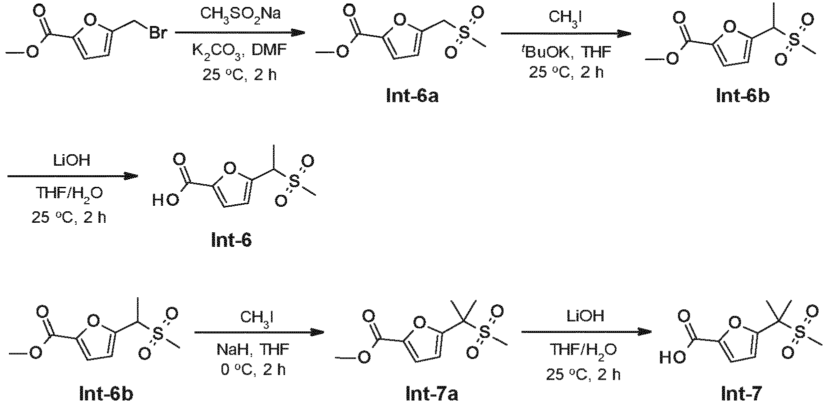

### Step 1: Preparation of methyl 5-(methylsulfonylmethyl)furan-2-carboxylate (Int-6a)

A suspension of methyl 5-(bromomethyl)furan-2-carboxylate (225.0 mg), methylsulfinyloxysodium (252.13 mg) and potassium carbonate (283.95 mg) in DMF (5 mL) was stirred at 25 °C for 2 h. The reaction was quenched with water (15 mL) and extracted with EtOAc (50 mL × 3). The combined organic layer was washed with brine (50 mL × 2), dried over anhydrous sodium sulfate, filtered and concentrated under reduced pressure to afford methyl 5-(methylsulfonylmethyl)furan-2-carboxylate (**Int-6a,** 200 mg) as a light yellow solid. MS obsd. (ESI⁺) [(M+H)⁺]: 219.1.

### Step 2: Preparation of methyl 5-(1-methylsulfonylethyl)furan-2-carboxylate (Int-6b)

To a solution of methyl 5-(methylsulfonylmethyl)furan-2-carboxylate (**Int-6a,** 200.0 mg) in THF (10 mL) was added potassium *tert*-butoxide (123.4 mg) at 0 °C. After being stirred for 30 min, iodomethane (260.17 mg) was added slowly at 0 °C. The reaction mixture was stirred for another 2 h at ambient temperature. The resulting solution was concentrated under reduced pressure. The residue was purified by flash column (eluting with EtOAc/PE = 30/70) to give methyl 5-(1-methylsulfonylethyl)furan-2-carboxylate (**Int-6b,** 120 mg) as a yellow solid. MS obsd. (ESI⁺) [(M+Na)⁺]: 255.0.

### Step 3: Preparation of 5-(1-methylsulfonylethyl)furan-2-carboxylic acid (Int-6)

To a solution of methyl 5-(1-methylsulfonylethyl)furan-2-carboxylate (**Int-6b,** 155.43 mg) in THF (5 mL) and water (1 mL) was added LiOH (19.23 mg). The reaction was stirred at 25 °C for 2 h. The resulting solution was diluted with water (20 mL) and washed with EtOAc (20 mL × 2). The aqueous was adjusted to pH = 6 with HCl (1 M) and extracted with EtOAc (20 mL × 2). The combined organic phase was dried over anhydrous sodium sulfate and concentrated under reduced pressure to give the crude product 5-(1-methylsulfonylethyl)furan-2-carboxylic acid (100 mg) as a white solid. MS obsd. (ESI⁺) [(M+H)⁺]: 236.1.

### Step 4: Preparation of methyl 5-(1-methyl-1-methylsulfonyl-ethyl)furan-2-carboxylate (Int-7a)

To a solution of methyl 5-(1-methylsulfonylethyl)furan-2-carboxylate (**Int-6b**, 120.0 mg) in anhydrous THF (5 mL) was added sodium hydride (24.8 mg) at 0 °C. The mixture was stirred at 0 °C for 30 min and then iodomethane (73.34 mg) was added slowly. The reaction was stirred for another 2 h at 0 °C and then quenched with saturated aqueous ammonium chloride (2 mL). THF was removed *in vacuo,* the residue was diluted with EtOAc (50 mL) and washed with water (50 mL) and brine (50 mL). The organic phase was dried over anhydrous sodium sulfate and concentrated under reduced pressure. The residue was purified by flash column (eluting with EtOAc/PE = 30/70) to give methyl 5-(1-methyl-1-methylsulfonyl-ethyl)furan-2-carboxylate (**Int-7a**, 70 mg) as a yellow solid. MS obsd. (ESI⁺) [(M+Na)⁺]: 269.1.

### Step 5: Preparation of 5-(1-methyl-1-methylsulfonyl-ethyl)furan-2-carboxylic acid (Int-7)

To a solution of methyl 5-(1-methyl-1-methylsulfonyl-ethyl)furan-2-carboxylate (**Int-7a**, 164.82 mg) in THF (5 mL) and water (1 mL) was added LiOH (19.23 mg) at ambient temperature. After being stirred for 2 h, the reaction was diluted with water (40 mL) and washed with EtOAc (20 mL × 2). The aqueous was adjusted to pH = 6 with aqueous HCl solution (1 M) and extracted with EtOAc (20 mL × 2). The combined organic phase was dried over anhydrous sodium sulfate and concentrated under reduced pressure to give 5-(1-methyl-1-methylsulfonylethyl)furan-2-carboxylic acid (**Int-7**, 100 mg) as a white solid. MS obsd. (ESI⁺) [(M+H)⁺]: 250.1.

### Intermediate Int-8

### 5-(2,2,2-trifluoro-1-hydroxy-ethyl)furan-2-carboxylic acid

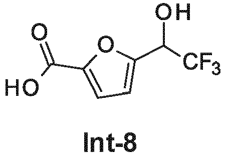

The title compound was prepared according to the following scheme:

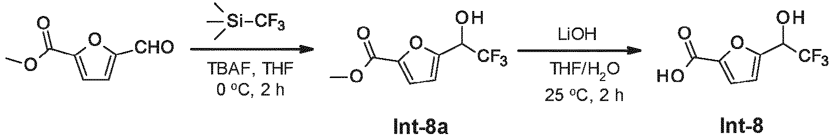

### Step 1: Preparation of methyl 5-(2,2,2-trifluoro-1-hydroxy-ethyl)furan-2-carboxylate (Int-8a)

To a solution of tetrabutylammonium fluoride (4.87 mL, 4.87 mmol) in tetrahydrofuran (50 mL) were added trifluoromethyltrimethylsilane (830.33 mg) and methyl 5-formylfuran-2-carboxylate (750.0 mg). After being stirred at 0 °C for 2 h, the resulting solution was diluted with ethyl acetate (100 mL) and washed with brine (50 mL × 3). The organic layer was dried over anhydrous sodium sulfate and concentrated under reduced pressure. The residue was purified by flash column (eluting with ethyl acetate/petrol ether = 40/60) to give methyl 5-(2,2,2-trifluoro-1-hydroxy-ethyl)furan-2-carboxylate (**Int-8a,** 600 mg) as a light yellow solid. MS obsd. (ESI⁺) [(M+H)⁺]: 225.2.

### Step 2: Preparation of 5-(2,2,2-trifluoro-1-hydroxy-ethyl)furan-2-carboxylic acid (Int-8)

To a solution of methyl 5-(2,2,2-trifluoro-1-hydroxy-ethyl)furan-2-carboxylate (150.0 mg) in THF (1 mL) and water (1 mL) was added LiOH (19.23 mg). The reaction was stirred at 25 °C for 2 h. After that, the resulted solution was diluted with water (20 mL), adjusted to pH = 6 with aqueous HCl solution (1 M), and extracted with ethyl acetate (20 mL × 2). The combined organic phase was dried over anhydrous sodium sulfate and concentrated under reduced pressure to give (5-(2,2,2-trifluoro-1-hydroxy-ethyl)furan-2-carboxylic acid (**Int-8,** 100 mg), which was used in next step directly. MS obsd. (ESI⁺) [(M+H)⁺]: 211.0.

### Intermediate Int-9

### [5-(azetidin-1-ylsulfonyl)furan-2-carbonyl]oxylithium

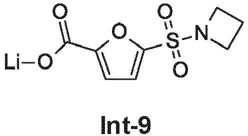

The title compound was prepared according to the following scheme:

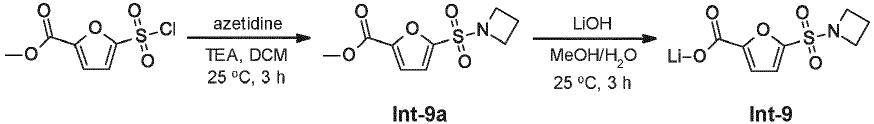

### Step 1: Preparation of methyl 5-(azetidin-1-ylsulfonyl)furan-2-carboxylate (Int-9a)

Methyl 5-(chlorosulfonyl)furan-2-carboxylate (112 mg) was added to a solution of azetidine (28.5 mg) and TEA (151 mg, 209 µL) in DCM (10 mL) and stirred at 25 °C for 3 h. LC-MS indicated that the reaction was complete. Then the reaction mixture was concentrated to afford the crude product of methyl 5-(azetidin-1-ylsulfonyl)furan-2-carboxylate (**Int-9a,** 122 mg).

### Step 2: Preparation of [5-(azetidin-1-ylsulfonyl)furan-2-carbonyl]oxylithium (Int-9)

Lithium hydroxide (119 mg) was added to a stirred solution of methyl 5-(azetidin-1-ylsulfonyl)furan-2-carboxylate (**Int-9a,** 122 mg) in MeOH (10 ml) and water (3 mL) at 25 °C for 3 h. LC-MS indicated that the reaction was complete. Then the reaction mixture was concentrated to afford the crude product of [5-(azetidin-1-ylsulfonyl)furan-2-carbonyl]oxylithium (**Int-9,** 115 mg).

### Intermediate Int-10

### [5-(3,3-difluoroazetidin-1-yl)sulfonylfuran-2-carbonyl]oxylithium

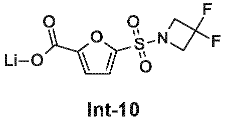

The title compound was prepared in analogy to the procedure described for the preparation of [5-(azetidin-1-ylsulfonyl)furan-2-carbonyl]oxylithium (**Int-9**), by using 3,3-difluoroazetidine instead of azetidine.

### Intermediate Int-11

### [5-(cyclobutylsulfamoyl)furan-2-carbonyl]oxylithium

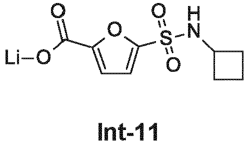

The title compound was prepared in analogy to the procedure described for the preparation of [5-(azetidin-1-ylsulfonyl)furan-2-carbonyl]oxylithium (**Int-9**), by using cyclobutanamine instead of azetidine.

### Intermediate Int-12

### [5-[(3,3-difluorocyclobutyl)sulfamoyl]furan-2-carbonyl]oxylithium

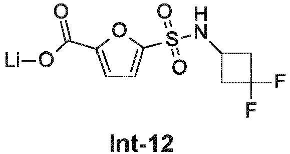

The title compound was prepared in analogy to the procedure described for the preparation of [5-(azetidin-1-ylsulfonyl)furan-2-carbonyl]oxylithium (**Int-9**), by using 3,3-difluorocyclobutanamine instead of azetidine.

### Intermediate Int-13

### [5-[[3-(trifluoromethyl)cyclobutyl]sulfamoyl]furan-2-carbonyl]oxylithium

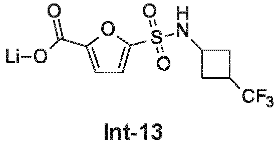

The title compound was prepared in analogy to the procedure described for the preparation of [5-(azetidin-1-ylsulfonyl)furan-2-carbonyl]oxylithium (**Int-9**), by using 3-(trifluoromethyl)cyclobutanamine instead of azetidine.

### Intermediate Int-14

### 5-(azetidin-1-ylsulfonyl)furan-2-carboxylic acid

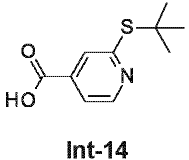

The title compound was prepared according to the following scheme:

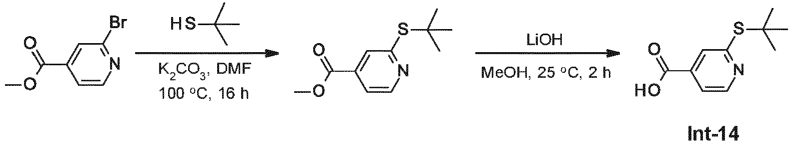

### Step 1: Preparation of methyl 2-tert-butylsulfanylpyridine-4-carboxylate

To a solution of methyl 2-bromoisonicotinate (2 g) in DMF (20 mL) was added dipotassium carbonate (1.5 g) and 2-methyl-2-propanethiol (2.09 mL). The mixture was stirred at 100 °C for 16 h and then filtered. The filtrate was concentrated under reduced pressure. The residue was purified by flash column (eluting with EtOAc/PE = 30/70) to afford methyl 2-*tert-*butylsulfanylpyridine-4-carboxylate (1700 mg) as yellow oil. MS obsd. (ESI⁺) [(M+H)⁺]: 226.2.

### Step 2: Preparation of 2-tert-butylsulfanylpyridine-4-carboxylic acid (Int-14)

To a solution of methyl 2-*tert*-butylsulfanylpyridine-4-carboxylate (1.2 g) in methanol (20 mL) was added LiOH (458.19 mg). The resulting mixture was stirred at ambient temperature for 16 h and then concentrated under reduced pressure. The residue was purified by prep-HPLC (column: Gemini - C18, 150 x 21.2 mm, 5µm; mobile phase: ACN - H₂O (0.05% FA); gradient: 20 - 25% ACN, flow rate: 20 mL/min) to give 2-*tert*-butylsulfanylpyridine-4-carboxylic acid **(Int-14,** 1 g) as a white solid. MS obsd. (ESI⁺) [(M+H)⁺]: 212.1. ¹H NMR (400 MHz, CD₃OD) δ ppm: 8.61 (d, *J* = 5.2 Hz, 1H), 7.83 (s, 1H), 7.66 (d, *J* = 5.2 Hz, 1H), 1.50 (s, 9H).

### Example 1

### N-[6-(5-chloro-1,3-benzothiazol-2-yl)spiro[3.3]heptan-2-yl]-3-(trifluoromethyl)benzamide

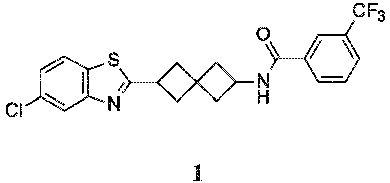

The title compound was prepared according to the following scheme:

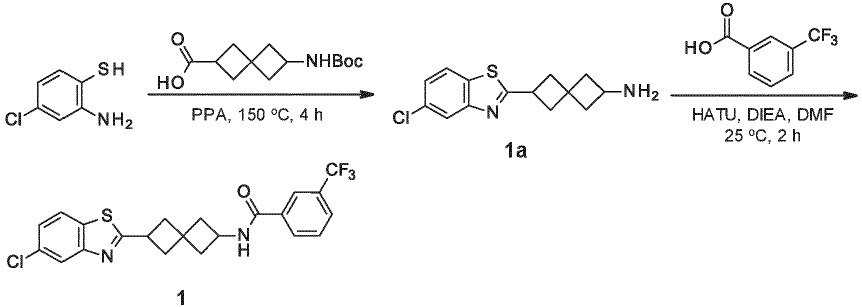

### Step 1: Preparation of 6-(5-chloro-1,3-benzothiazol-2-yl)spiro[3.3]heptan-2-amine (1a)

To polyphosphoric acid (3.30 g) was added 2-amino-4-chlorothiophenol (223.0 mg as the **"Thiophenol"** in Table 1) and 2-(*tert*-butoxycarbonylamino)spiro[3.3]heptane-6-carboxylic acid (427.97 mg) at 110 °C. The mixture was heated to 150 °C and stirred at this temperature for 4 h. After being cooled to ambient temperature, the reaction was quenched with water (30 mL), adjusted to pH = 8~9 with aqueous ammonium hydroxide solution (14 mL), and then extracted with ethyl acetate (50 mL × 3). The combined organic layer was washed with brine (20 mL), dried over anhydrous sodium sulfate and concentrated under reduced pressure to give 6-(5-chloro-1,3-benzothiazol-2-yl)spiro[3.3]heptan-2-amine (compound **1a**,302 mg) as dark brown oil, which was used in next step directly without purification. MS obsd. (ESI⁺) [(M+H)⁺]: 279.1.

### Step 2: Preparation of N-[6-(5-chloro-1,3-benzothiazol-2-yl)spiro[3.3]heptan-2-yl]-3-(trifluoromethyl)benzamide (Example 1)

To a solution of 6-(5-chloro-1,3-benzothiazol-2-yl)spiro[3.3]heptan-2-amine (compound **1a**, 146.64 mg) in DMF (4 mL) were added HATU (400.0 mg), DIEA (67.98 mg) and 3-(trifluoromethyl)benzoic acid (100.0 mg, as the **"Acid"** in Table 1). After being stirred at 25 °C for 2 h, the solution was diluted with ethyl acetate (30 mL) and washed with water (20 mL × 2). The organic layer was dried over anhydrous sodium sulfate and concentrated under reduced pressure. The residue was purified by prep-HPLC to give *N*-[6-(5-chloro-1,3-benzothiazol-2-yl)spiro[3.3]heptan-2-yl]-3-(trifluoromethyl)benzamide (55.8 mg) as a white solid. MS obsd. (ESI⁺) [(M+H)⁺]: 451.1. ¹H NMR (400 MHz, CD₃OD) δ ppm: 8.14 (s, 1H), 8.08 (d, *J=* 7.8 Hz, 1H), 7.88-7.93 (m, 2H), 7.83 (d, *J=* 7.8 Hz, 1H), 7.66 (t, *J* = 7.8 Hz, 1H), 7.39 (dd, *J=* 8.6, 2.0 Hz, 1H), 4.42-4.46 (m, 1H), 3.84-3.96 (m, 1H), 2.65-2.79 (m, 2H), 2.40-2.59 (m, 4H), 2.27 (dd, *J=* 10.8, 8.8 Hz, 1H), 2.18 (dd, *J* = 11.4, 8.8 Hz, 1H).

The following **Example 2** to **Example 37** were prepared in analogy to the procedure described for the preparation of **Example 1,** replacing 2-amino-4-chlorothiophenol with **"Thiophenol",** and replacing 3-(trifluoromethyl)benzoic acid with **"Acid".** The **"Thiophenol", "Acid"** are the reagents indicated in Table 1.

**Table 1: Compound synthesis and characterization**

| Examples | Compound Name and Structure | **Thiophenol & Acid** | MS (ESI⁺) and ¹H NMR |
|---|---|---|---|
| 2 | *N*-[6-(5-chloro-1,3-benzothiazol-2-yl)spiro[3.3]heptan-2-yl]-3-sulfamoylbenzamide | **Thiophenol:** 2-amino-4-chlorothiophenol | MS obsd. (ESI⁺) [(M+H)⁺]: 462.01. |
| | | | ¹H NMR (400 MHz, CD₃OD) *δ* ppm: 8.34 (s, 1H), 8.01-8.05 (m, 2H), 7.90-7.94 (m, 2H), 7.64 (t, *J* = 7.8 Hz, 1H), 7.40 (d,*J* = 8.4 Hz, 1H), 4.38-4.49 (m, 1H), 3.87-3.96 (m, 1H), 2.71-2.74 (m, 2H), 2.40-2.62 (m, 4H), 2.23-2.31 (m, 1H), 2.14-2.21 (m, 1H). |
| | | **Acid:** 3-sulfamoylbenzoi c acid | |
| | 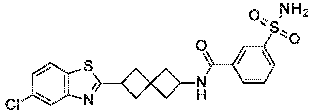 | | |
| 3 | *N*-[6-(5-chloro-1,3-benzothiazol-2-yl)spiro[3.3]heptan-2-yl]-3-dimethylphosphoryl-benzamide | **Thiophenol:** 2-amino-4-chlorothiophenol | MS obsd. (ESI⁺) [(M+H)⁺]: 459.1. |
| | | | ¹H NMR (400 MHz, CD₃OD) δ ppm: 8.23 (dt, *J* = 12.3, 1.4 Hz, 1H), 7.92-8.07 (m, 4H), 7.68 (td, *J=* 7.7, 2.6 Hz, 1H), 7.42 (dd, *J* = 8.6, 2.1 Hz, 1H), 4.47 (quin, *J* = 8.2 Hz, 1H), 3.94 (quin, *J* = 8.6 Hz, 1H), 2.68-2.82 (m, 2H), 2.45-2.63 (m, 4H), 2.16-2.33 (m, 2H), 1.86 (s, 3H), 1.83 (s, 3H). |
| | | **Acid: Int-1** | |
| | 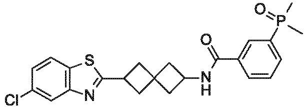 | | |
| 4 | *N*-[6-(5-chloro-1,3-benzothiazol-2-yl)spiro[3.3]heptan-2-yl]-2-(trifluoromethyl)pyridine-4-carboxamide | **Thiophenol:** 2-amino-4-chlorothiophenol | MS obsd. (ESI⁺) [(M+H)⁺]: 452.1. |
| | 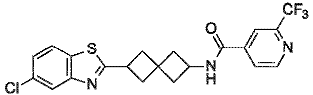 | **Acid:** 2-(trifluoromethyl)pyridine-4-carboxylic acid | ¹H NMR (400 MHz, CD₃OD) *δ* ppm: 8.85 (d, *J* = 5.2 Hz, 1H), 8.18 (s, 1H), 8.00 (dd, *J=* 5.2, 1.2 Hz, 1H), 7.88-7.93 (m, 2H), 7.39 (dd, *J =* 8.4, 2.0 Hz, 1H), 4.44 (t, *J* = 8.0 Hz, 1H), 3.83-3.96 (m, 1H), 2.67-2.78 (m, 2H), 2.42-2.60 (m, 4H), 2.28 (dd, *J =* 11.0, 8.8 Hz, 1H), 2.19 (dd, *J =* 11.6, 8.8 Hz, 1H). |
| 5 | *N*-[6-(5-chloro-1,3-benzothiazol-2-yl)spiro[3.3]heptan-2-yl]-2-cyano-pyridine-4-carboxamide | **Thiophenol:** 2-amino-4-chlorothiophenol | MS obsd. (ESI⁺) [(M+H)⁺]: 409.0. |
| | | | ¹H NMR (400 MHz, CD₃OD) δ ppm: 8.83 (dd, *J* = 5.2, 0.8 Hz, 1H), 8.21 (dd, *J* = 1.6, 0.8 Hz, 1H), 7.99 (dd, *J* = 5.2, 1.6 Hz, 1H), 7.89-7.93 (m, 2H), 7.39 (dd, *J =* 8.4, 2.0 Hz, 1H), 4.36-4.50 (m, 1H), 3.91 (p, *J* = 8.6 Hz, 1H), 2.66-2.78 (m, 2H), 2.49-2.53 (m, 4H), 2.27 (dd, J = 11.2, 8.8 Hz, 1H), 2.17 (dd, J = 11.2, 8.8 Hz, 1H). |
| | | **Acid:** 2-cyanopyridine-4-carboxylic acid | |
| | 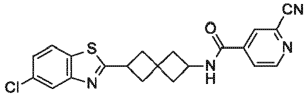 | | |
| 6 | *N*-[6-(5-chloro-1,3-benzothiazol-2-yl)spiro[3.3]heptan-2-yl]-2-methyl-pyridine-4-carboxamide | **Thiophenol:** 2-amino-4-chlorothiophenol | MS obsd. (ESI⁺) [(M+H)⁺]: 398.1. |
| | | | ¹H NMR (400 MHz, CD₃OD) δ ppm: 8.78 (d, *J* = 6.0 Hz, 1H), 8.06-8.31 (m, 2H), 7.87-7.91 (m, 2H), 7.37 (dd, *J* = 8.6, 2.0 Hz, 1H), 4.43-4.49 (m, 1H), 3.91 (dd, *J=* 17.0, 8.4 Hz, 1H), 2.82 (s, 3H), 2.71-2.75 (m, 2H), 2.42-2.66 (m, 4H), 2.17-2.42 (m, 2H). |
| | | **Acid:** 2-methylpyridine-4-carboxylic acid | |
| | 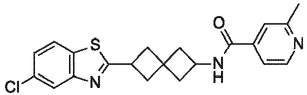 | | |
| 7 | N-[6-(5-chloro-1,3-benzothiazol-2-yl)spiro[3.3]heptan-2-yl]-2-(methylsulfonylmethyl)pyridin e-4-carboxamide | **Thiophenol:** 2-amino-4-chlorothiophenol | MS obsd. (ESI⁺) [(M+H)⁺]: 476.0. |
| | | **Acid: Int-2** | ¹H NMR (400 MHz, CD₃OD) δ ppm: 8.72 (d, *J* = 5.2 Hz, 1H), 7.88-7.92 (m, 3H), 7.75 (dd, *J*= 5.2, 1.6 Hz, 1H), 7.39 (dd, *J* = 8.6, 2.0 Hz, 1H), 4.65 (s, 2H), 4.37-4.50 (m, 1H), 3.91 (p, *J* = 8.6 Hz, 1H), 2.98-3.02 (m, 3H), 2.66-2.79 (m, 2H), 2.41-2.61 (m, 4H), 2.22-2.32 (m, 1H), 2.17 (dd, *J=* 11.2, 9.0 Hz, 1H). |
| | 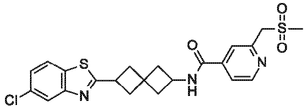 | | |
| 8 | *N*-[6-(5-chloro-1,3-benzothiazol-2-yl)spiro[3.3]heptan-2-yl]-2-(1-methyl-1-methylsulfonylethyl)pyridine-4-carboxamide | **Thiophenol:** 2-amino-4-chlorothiophenol | MS obsd. (ESI⁺) [(M+H)⁺]: 504.0. |
| | | | ¹H NMR (400 MHz, CD₃OD) δ ppm: 8.74 (d, *J* = 5.2 Hz, 1H), 8.04 (s, 1H), 7.90-7.94 (m, 2H), 7.72 (d, *J* = 5.2 Hz, 1H), 7.39 (d, *J* = 8.4 Hz, 1H), 4.38-4.51 (m, 1H), 3.86-3.98 (m, 1H), 2.84 (s, 3H), 2.67-2.79 (m, 2H), 2.42-2.62 (m, 4H), 2.25-2.32 (m, 1H), 2.15-2.23 (m, 1H), 1.91 (s, 6H). |
| | | **Acid: Int-3** | |
| | 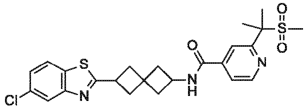 | | |
| 9 | *N*-[6-(5-chloro-1,3-benzothiazol-2-yl)spiro[3.3]heptan-2-yl]-2-ethylsulfonyl-pyridine-4-carboxamide | **Thiophenol:** 2-amino-4-chlorothiophenol | MS obsd. (ESI⁺) ([M+H]⁺): 476.0. |
| | | | ¹H NMR (400 MHz, DMSO-*d₆*) δ ppm: 9.22 (d, *J* = 7.6 Hz, 1H), 8.94 (d, *J* = 5.2 Hz, 1H), 8.43 (s, 1H), 8.11-8.08 (m, 2H), 8.02 (m, 1H), 7.46-7.44 (m, 1H), 4.41-4.31 (m, 1H), 3.96-3.88 (m, 1H), 3.48 (q, *J* = 7.6 Hz, 2H), 2.70-2.64 (m, 1H), 2.62-2.56 (m, 1H), 2.52-2.51 (m, 1H), 2.44-2.40 (m, 2H), 2.37-2.32 (m, 1H), 2.27-2.22 (m, 1H), 2.18-2.13 (m, 1H), 1.14 (t, *J* = 7.6 Hz, 3H). |
| | | **Acid: Int-4** | |
| | 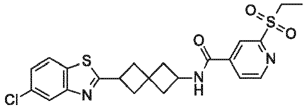 | | |
| 10 | *N*-[6-(5-chloro-1,3-benzothiazol-2-yl)spiro[3.3]heptan-2-yl]-2-ureido-pyridine-4-carboxamide | **Thiophenol:** 2-amino-4-chlorothiophenol | MS obsd. (ESI⁺) ([M+H]⁺): 442.0. |
| | | | ¹H NMR (400 MHz, DMSO-*d₆*) *δ* ppm: 9.33 (s, 1H), 8.80 (d, *J* = 7.2 Hz, 1H), 8.28 (d, *J* = 5.2 Hz, 1H), 8.08 (d, *J* = 8.8 Hz, 1H), 8.02 (d, *J* = 2.0 Hz, 1H), 7.79 (s, 1H), 7.45 (dd, *J* = 8.8, 2.0 Hz, 1H), *7.26* (*J* = 5.2 Hz, 1H), 6.94 (s, 2H), 4.36-4.26 (m, 1H), 3.95-3.86 (m, 1H), 2.66-2.62 (m, 1H), 2.58-2.52 (m, 1H), 2.46-2.38 (m, 3H), 2.34-2.28 (m, 1H), 2.23-2.18 (m, 1H), 2.13-2.08 (m, 1H). |
| | | **Acid:** 2-ureidopyridine-4-carboxylic acid | |
| | 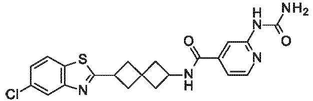 | | |
| 11 | *N4*-[6-(5-chloro-1,3-benzothiazol-2-yl)spiro[3.3]heptan-2-yl]pyridine-2,4-dicarboxamide | **Thiophenol:** 2-amino-4-chlorothiophenol | MS obsd. (ESI⁺) ([M+H]⁺): 427.0. |
| | | | ¹H NMR (400 MHz, DMSO-*d₆*) *δ* ppm: 9.12 (d, *J* = 7.2 Hz, 1H), 8.76 (d, *J* = 4.8Hz, 1H), 8.46 (s, 1H), 8.20 (s, 1H), 8.09 (d, *J* = 8.4 Hz, 1H), 8.02 (d, *J* = 2.0 Hz, 1H), 7.94 (dd, *J* = 4.8, 2.0 Hz, 1H), 7.75 (d, *J* = 2.0 Hz, 1H), 7.45 (dd, *J* = 8.4, 2.0 Hz, 1H), 4.41-4.31 (m, 1H), 3.96-3.87 (m, 1H), 2.69-2.63 (m, 1H), 2.60-2.54 (m, 1H), 2.46-2.39 (m, 3H), 2.36-2.30 (m, 1H), 2.27-2.23 (m, 1H), 2.18-2.13 (m, 1H). |
| | | **Acid:** 2-carbamoylpyridi ne-4-carboxylic acid | |
| | 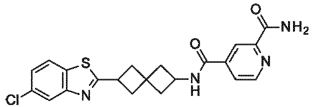 | | |
| 12 | *N4*-[6-([1,3]dioxolo[4,5-f][1,3]benzothiazol-6-yl)spiro[3.3]heptan-2-yl]pyridine-2,4-dicarboxamide | **Thiophenol:** 6-amino-1,3-benzodioxole-5-thiol | MS obsd. (ESI⁺) [(M+H)⁺]: 437.4. |
| | | | ¹H NMR (400 MHz, DMSO-*d₆*) δ ppm: 9.10 (d, *J* = 7.2 Hz, 1H), 8.76 (d, *J* = 5.2 Hz, 1H), 8.46 (s, 1H), 8.19 (s, 1H), 7.94 (dd, *J* = 1.6, 4.8 Hz, 1H), 7.74 (d, *J* = 1.2 Hz, 1H), 7.57 (s, 1H), 7.45 (s, 1H), 6.11 (s, 2H), 4.44-4.26 (m, 1H), 3.86-3.75 (m, 1H), 2.69-2.54 (m, 2H), 2.46-2.29 (m, 4H), 2.24 (dd, *J* = 9.2, 10.8 Hz, 1H), 2.15 (dd, *J=* 9.2, 10.8 Hz, 1H). |
| | | **Acid:** 2-carbamoylpyridi ne-4-carboxylic acid | |
| | 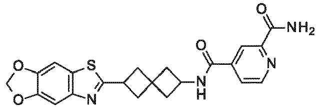 | | |
| 13 | *N*-[6-(5-chloro-1,3-benzothiazol-2-yl)spiro[3.3]heptan-2-yl]-5-cyclopropylsulfonyl-furan-2-carboxamide | **Thiophenol:** 2-amino-4-chlorothiophenol | MS obsd. (ESI⁺) [(M+H)⁺]: 477.1. |
| | | | ¹H NMR (400 MHz, CDCl₃) *δ* ppm: 8.00 (d, *J* = 2.0 Hz, 1H), 7.76 (d, *J* = 8.4 Hz, 1H), 7.35 (dd, *J* = 8.4, 2.0 Hz, 1H), 7.19 (d, *J=* 3.6 Hz, 1H), 7.14 (d, *J* = 3.6 Hz, 1H), 6.59 (d, *J* = 7.6 Hz, 1H), 4.48-5.53 (m, 1H), 3.88-3.92 (m, 1H), 2.72-2.76 (m, 2H), 2.51-2.67 (m, 5H), 2.07-2.21 (m, 2H), 1.40-1.46 (m, 2H), 1.14-1.18 (m, 2H). |
| | | **Acid: Int-5** | |
| | 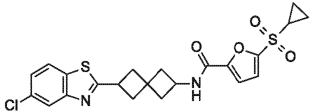 | | |
| 14 | *N*-[6-(5-chloro-1,3-benzothiazol-2-yl)spiro[3.3]heptan-2-yl]-5-(trifluoromethyl)furan-2-carboxamide | **Thiophenol:** 2-amino-4-chlorothiophenol | MS obsd. (ESI⁺) [(M+H)⁺]: 441.0. |
| | | | ¹H NMR (400 MHz, DMSO-*d₆*) δ ppm: 8.85 (d, *J* = 8.0 Hz, 1H), 8.09 (d, *J=* 8.0 Hz, 1H), 8.03 (d, *J=* 2.0 Hz, 1H), 7.42-7.49 (m, 1H), 7.34-7.39 (m, 1H), 7.25-7.29 (m, 1H), 4.26-4.36 (m, 1H), 3.87-3.95 (m, 1H), 2.55-2.69 (m, 2H), 2.12-2.48 (m, 6H). |
| | | **Acid:** 5-(trifluoromethyl) furan-2-carboxylic acid | |
| | 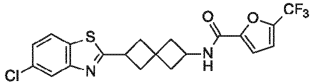 | | |
| 15 | *N*-[6-(6-chloro-1,3-benzothiazol-2-yl)spiro[3.3]heptan-2-yl]-5-(trifluoromethyl)furan-2-carboxamide | **Thiophenol:** 2-amino-5-chlorothiophenol | MS obsd. (ESI⁺) [(M+H)⁺]: 441.0. |
| | | | ¹H NMR (400 MHz, CDCl₃) *δ* ppm: 7.88 (d, *J* = 8.8 Hz, 1H), 7.82 (d, *J* = 2.0 Hz, 1H), 7.42 (dd, *J* = 8.8, 2.0 Hz, 1H), 7.12-7.16 (m, 1H), 6.84-6.92 (m, 1H), 6.49 (d, *J* = 7.6 Hz, 1H), 4.45-4.57 (m, 1H), 3.86 (p, *J* = 8.6 Hz, 1H), 2.67-2.78 (m, 2H), 2.54-2.58 (m, 4H), 2.12-2.16 (m, 2H). |
| | | **Acid:** 5-(trifluoromethyl) furan-2-carboxylic acid | |
| | 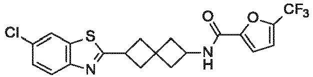 | | |
| 16 | *N*-[6-(5-chloro-1,3-benzothiazol-2-yl)spiro[3.3]heptan-2-yl]-5-(1-methylsulfonylethyl)furan-2-carboxamide | **Thiophenol:** 2-amino-4-chlorothiophenol | MS obsd. (ESI⁺) [(M+H)⁺]: 479.0. |
| | | | ¹H NMR (400 MHz, CD₃OD) δ ppm: 7.89-7.93 (m, 2H), 7.39 (dd, *J* = 8.4, 2.0 Hz, 1H), 7.12 (d, *J* = 3.6 Hz, 1H), 6.69 (d, *J* = 3.6 Hz, 1H), 4.64 (q, *J* = 7.2 Hz, 1H), 4.40-4.44 (m, 1H), 3.91 (p, *J* = 8.4 Hz, 1H), 2.91 (s, 3H), 2.65-2.78 (m, 2H), 2.39-2.58 (m, 4H), 2.24-2.31 (m, 1H), 2.16-2.20 (m, 1H), 1.77 (d, J = 7.2 Hz, 3H). |
| | | **Acid: Int-6** | |
| | 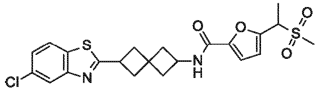 | | |
| 17 | *N*-[6-(5-chloro-1,3-benzothiazol-2-yl)spiro[3.3]heptan-2-yl]-5-(1-methyl-1-methylsulfonylethyl)furan-2-carboxamide | **Thiophenol:** 2-amino-4-chlorothiophenol | MS obsd. (ESI⁺) [(M+H)⁺]: 493.1. |
| | | | ¹H NMR (400 MHz, CD₃OD) δ ppm: 7.89-7.93 (m, 2H), 7.39 (dd, *J=* 8.4, 2.0 Hz, 1H), 7.13 (d, *J=* 3.6 Hz, 1H), 6.73 (d, *J* = 3.6 Hz, 1H), 4.35-4.45 (m, 1H), 3.85-3.95 (m, 1H), 2.83 (s, 3H), 2.64-2.76 (m, 2H), 2.39-2.58 (m, 4H), 2.25-2.33 (m, 1H), 2.15-2.23 (m, 1H), 1.81 (s, 6H). |
| | | **Acid: Int-7** | |
| | 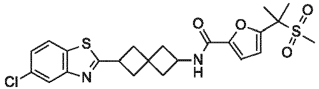 | | |
| 18 | *N*-[6-(5-chloro-1,3-benzothiazol-2-yl)spiro[3.3]heptan-2-yl]-5-(2,2,2-trifluoro-1-hydroxyethyl)furan-2-carboxamide | **Thiophenol:** 2-amino-4-chlorothiophenol | MS obsd. (ESI⁺) [(M+H)⁺]: 471.1. |
| | | | ¹H NMR (400 MHz, CD₃OD) δ ppm: 7.88-7.93 (m, 2H), 7.39 (d, *J* = 8.4 Hz, 1H), 7.10 (d, *J* = 3.2 Hz, 1H), 6.66 (d, *J* = 3.2 Hz, 1H), 5.18-5.22 (m, 1H), 4.33-4.47 (m, 1H), 3.81-3.99 (m, 1H), 2.63-2.80 (m, 2H), 2.37-2.62 (m, 4H), 2.20-2.24 (m, 2H). |
| | | **Acid: Int-8** | |
| | 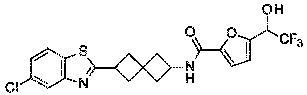 | | |
| 19 | 2-amino-*N*-[6-(5-chloro-1,3-benzothiazol-2-yl)spiro[3.3]heptan-2-yl]thiazole-4-carboxamide | **Thiophenol:** 2-amino-4-chlorothiophenol | MS obsd. (ESI⁺) ([M+H]⁺): 405.0. |
| | | | ¹H NMR (400 MHz, DMSO-*d₆*) δ ppm: 8.09 (d, *J* = 8.6 Hz, 1H), 8.02 (d, *J* = 2.1 Hz, 1H), 7.88 (br d, *J=* 8.2 Hz, 1H), 7.45 (dd, *J* = 8.6, 2.1 Hz, 1H), 7.18 (s, 1H), 7.12 (br s, 2H), 4.26 (sxt, *J* = 8.3 Hz, 1H), 3.89 (quin, *J* = 8.5 Hz, 1H), 2.58-2.69 (m, 1H), 2.32-2.55 (m, 4H), 2.16-2.30 (m, 2H), 2.03-2.14 (m, 1H). |
| | | **Acid:** 2-aminothiazole-4-carboxylic acid | |
| | 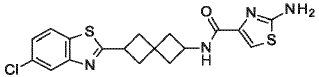 | | |
| 20 | 5-(azetidin-1-ylsulfonyl)-*N*-[6-(5-chloro-1,3-benzothiazol-2-yl)spiro[3.3]heptan-2-yl]furan-2-carboxamide | **Thiophenol:** 2-amino-4-chlorothiophenol | MS obsd. (ESI⁺) [(M+H)⁺]: 492.1. |
| | | | ¹H NMR (400 MHz, DMSO-*d₆*) δ ppm: 8.89 (d, *J* = 7.5 Hz, 1H), 8.09 (d, *J* = 8.6 Hz, 1H), 8.03 (d, *J* = 2.0 Hz, 1H), 7.34-7.47 (m, 3H), 4.34 (sxt, *J* = 8.1 Hz, 1H), 3.76-3.96 (m, 5H), 2.53-2.74 (m, 2H), 2.06-2.49 ppm (m, 8H). |
| | | **Acid: Int-9** | |
| | 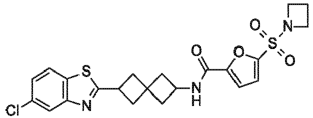 | | |
| 21 | *N*-[6-(5-chloro-1,3-benzothiazol-2-yl)spiro[3.3]heptan-2-yl]-5-(3,3-difluoroazetidin-1-yl)sulfonyl-furan-2-carboxamide | **Thiophenol:** 2-amino-4-chlorothiophenol | MS obsd. (ESI⁺) [(M+H)⁺]: 528.1. |
| | | | ¹H NMR (400 MHz, DMSO-*d₆*) δ ppm: 8.94 (d, *J* = 7.5 Hz, 1H), 8.09 (d, *J* = 8.6 Hz, 1H), 8.03 (d, *J=* 2.0 Hz, 1H), 7.54 (d, *J* = 3.8 Hz, 1H), 7.46 (dd, *J* = 8.6, 2.1 Hz, 1H), 7.35 (d, *J* = 3.7 Hz, 1H), 4.50 (t, *J* = 12.7 Hz, 4H), 4.33 (sxt, *J* = 8.1 Hz, 1H), 3.87-3.96 (m,1H), 2.54-2.74 (m, 2H), 2.39-2.48 (m, 3H), 2.14-2.35 (m, 3H). |
| | | **Acid: Int-10** | |
| | 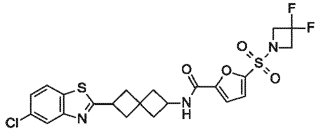 | | |
| 22 | *N*-[6-(5-chloro-1,3-benzothiazol-2-yl)spiro[3.3]heptan-2-yl]-5-(cyclobutylsulfamoyl)furan-2-carboxamide | **Thiophenol:** 2-amino-4-chlorothiophenol | MS obsd. (ESI⁺) [(M+H)⁺]: 506.1. |
| | | | ¹H NMR (400 MHz, CD₃OD) δ ppm: 7.90-7.94 (m, 2H), 7.41 (dd, *J* = 8.5, 2.0 Hz, 1H), 7.07-7.20 (m, 2H), 4.38-4.47 (m, 1H), 3.84-3.96 (m, 2H), 2.66-2.78 (m, 2H), 2.41-2.60 (m, 4H), 2.29 (dd, *J* = 11.0, 8.9 Hz, 1H), 2.11-2.23 (m, 3H), 1.83-1.96 (m, 2H), 1.56-1.71 (m, 2H). |
| | | **Acid: Int-11** | |
| | 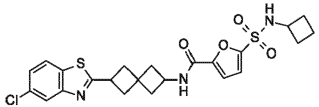 | | |
| 23 | *N*-[6-(5-chloro-1,3-benzothiazol-2-yl)spiro[3.3]heptan-2-yl]-5-[(3,3-difluorocyclobutyl)sulfamoyl]f uran-2-carboxamide | **Thiophenol:** 2-amino-4-chlorothiophenol | MS obsd. (ESI⁺) [(M+H)⁺]: 542.1. |
| | | | 1H NMR (400 MHz, CD₃OD) *δ* ppm: 7.91-7.95 (m, 2H), 7.42 (dd, *J=* 8.6, 2.1 Hz, 1H), 7.21 (d, *J =* 3.7 Hz, 1H), 7.15 (d, *J* = 3.7 Hz, 1H), 4.39-4.48 (m, 1H), 3.80-3.98 (m, 2H), 2.67-2.92 (m, 4H), 2.42-2.62 (m, 6H), 2.16-2.33 (m, 2H). |
| | | **Acid: Int-12** | |
| | 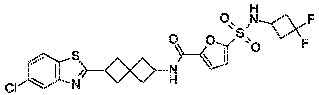 | | |
| 24 | *N*-[6-(5-chloro-1,3-benzothiazol-2-yl)spiro[3.3]heptan-2-yl]-5-[[3-(trifluoromethyl)cyclobutyl]sul famoyl]furan-2-carboxamide | **Thiophenol:** 2-amino-4-chlorothiophenol | MS obsd. (ESI⁺) [(M+H)⁺]: 574.1. |
| | | | ¹H NMR (400 MHz, CD₃OD) δ ppm: 7.92-7.96 (m, 2H), 7.42 (dd, *J* = 8.6, 2.1 Hz, 1H), 7.21 (d, *J* = 3.7 Hz, 1H), 7.12 (t, *J* = 3.9 Hz, 1H), 4.38-4.48 (m, 1H), 3.93 (quin, *J* = 8.5 Hz, 2H), 2.67-2.80 (m, 3H), 2.37-2.61 (m, 6H), 2.16-2.32 (m, 3H), 1.96-2.09 ppm (m, 1H). |
| | | **Acid: Int-13** | |
| | 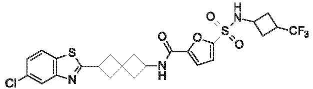 | | |
| 25 | *N*-[6-(5-chloro-1,3-benzothiazol-2-yl)spiro[3.3]heptan-2-yl]-1,1-dioxo-thietane-3-carboxamide | **Thiophenol:** 2-amino-4-chlorothiophenol | MS obsd. (ESI⁺) [(M+H)⁺]: 394.1. |
| | | | ¹H NMR (400 MHz, CD₃OD): *δ* ppm: 7.88-7.92 (m, 2H), 7.39 (dd, *J=* 8.4, 2.0 Hz, 1H), 4.29-4.37 (m, 2H), 4.16-4.27 (m, 3H), 3.83-3.93 (m, 1H), 3.21-3.25 (m, 1H), 2.60-2.72 (m, 2H), 2.43-2.55 (m, 3H), 2.35-2.42 (m, 1H), 2.05-2.12 (m, 1H), 1.98-2.02 (m, 1H). |
| | | Acid: 1,1-dioxothietane-3-carboxylic acid | |
| | 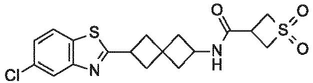 | | |
| 26 | *N*-[6-(5-chloro-1,3-benzothiazol-2-yl)spiro[3.3]heptan-2-yl]-1,1-dioxo-thiolane-3-carboxamide | **Thiophenol:** 2-amino-4-chlorothiophenol | MS obsd. (ESI⁺) [(M+H)⁺]: 425.1. |
| | | | ¹H NMR (400 MHz, DMSO-*d₆*) δ ppm: 8.35 (d, *J = 8.0* Hz, 1H), 8.09 (d, *J = 8.0* Hz, 1H), 8.02 (d, *J =* 2.0 Hz, 1H), 7.44-7.47 (m, 1H), 4.02-4.14 (m, 1H), 3.84-3.93 (m, 1H), 3.02-3.27 (m, 5H), 2.53-2.63 (m, 2H), 2.25-2.48 (m, 5H), 1.84-2.07 (m, 3H). |
| | | **Acid:** 1,1-dioxothiolane-3-carboxylic acid | |
| | 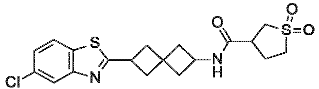 | | |
| 27 | *N*-[6-(6-chloro-1,3-benzothiazol-2-yl)spiro[3.3]heptan-2-yl]-1,1-dioxo-thiolane-3-carboxamide | **Thiophenol:** 2-amino-5-chlorothiophenol | MS obsd. (ESI⁺) [(M+H)⁺]: 425.1. |
| | | | ¹H NMR (400 MHz, CDCl₃) δ ppm: 7.87 (d, *J* = 8.8 Hz, 1H), 7.81 (d, *J* = 2.0 Hz, 1H), 7.42 (dd, *J=* 8.8, 2.0 Hz, 1H), 5.80 (s, 1H), 4.27-4.39 (m, 1H), 3.83 (p, *J =* 8.6 Hz, 1H), 3.16-3.37 (m, 3H), 2.96-3.00 (m, 2H), 2.65-2.69 (m, 2H), 2.33-2.59 (m, 6H), 1.88-2.04 (m, 2H). |
| | | **Acid:** 1,1-dioxothiolane-3-carboxylic acid | |
| | 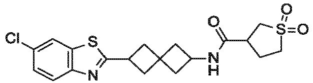 | | |
| 28 | *N*-[6-(5-chloro-1,3-benzothiazol-2-yl)spiro[3.3]heptan-2-yl]-2-(1,1-dioxothietan-3-yl)acetamide | **Thiophenol:** 2-amino-4-chlorothiophenol | MS obsd. (ESI⁺) [(M+H)⁺]: 425.1. |
| | | | ¹H NMR (400 MHz, CD₃OD) *δ* ppm: 7.87-7.95 (m, 2H), 7.39 (dd, *J* = 8.4, 2.0 Hz, 1H), 4.14-4.31 (m, 3H), 3.83-3.87 (m, 3H), 2.86-2.90 (m, 1H), 2.56-2.71 (m, 4H), 2.48-2.52 (m, 3H), 2.32-2.40 (m, 1H), 2.04-2.11 (m, 1H), 1.97 (dd, *J* = 11.4, 8.8 Hz, 1H). |
| | | **Acid:** 2-(1,1-dioxothietan-3-yl)acetic acid | |
| | 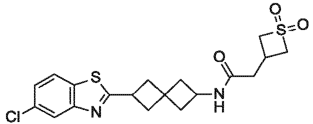 | | |
| 29 | *N*-[6-(5-chloro-1,3-benzothiazol-2-yl)spiro[3.3]heptan-2-yl]-2-(1,1-dioxothiolan-2-yl)acetamide | **Thiophenol:** 2-amino-4-chlorothiophenol | MS obsd. (ESI⁺) [(M+H)⁺]: 439.1. |
| | | | ¹H NMR (400 MHz, CD₃OD) *δ* ppm: 7.87-7.91 (m, 2H), 7.38 (dd, *J* = 8.6, 2.0 Hz, 1H), 4.20 (dt, *J* = 16.0, 8.0 Hz, 1H), 3.86 (dt, *J* = 17.0, 8.6 Hz, 1H), 3.41 (d, *J* = 7.6 Hz, 1H), 3.12-3.23 (m, 1H), 2.93 - 3.07 (m, 1H), 2.57-2.81 (m, 4H), 2.36-2.50 (m, 5H), 1.97-2.23 (m, 4H), 1.71-1.94 (m, 1H). |
| | | **Acid:** 2-(1,1-dioxothiolan-2-yl)acetic acid | |
| | 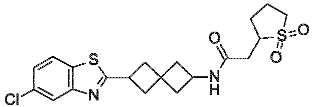 | | |
| 30 | *N*-[6-(5-chloro-1,3-benzothiazol-2-yl)spiro[3.3]heptan-2-yl]-2-(1,1-dioxothian-3-yl)acetamide | **Thiophenol:** 2-amino-4-chlorothiophenol | MS obsd. (ESI⁺) [(M+H)⁺]: 453.1. |
| | | **Acid:** 2-(1,1-dioxothian-3-yl)acetic acid | ¹H NMR (400 MHz, CD₃OD) *δ* ppm: 7.88-7.92 (m, 2H), 7.39 (dd, *J=* 8.6, 2.0 Hz, 1H), 4.15-4.24 (m, 1H), 3.82-3.93 (m, 1H), 2.99-3.03 (m, 2H), 2.83-2.93 (m, 1H), 2.60-2.72 (m, 2H), 2.42-2.55 (m, 4H), 2.33-2.41 (m, 1H), 1.93-2.23 (m, 6H), 1.83 (d, *J* = 13.4 Hz, 1H), 1.25-1.29 (m, 2H). |
| | 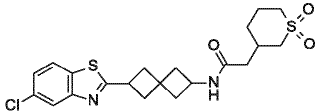 | | |
| 31 | *N*-[6-(5-chloro-1,3-benzothiazol-2-yl)spiro[3.3]heptan-2-yl]-1-ethyl-5-oxo-pyrrolidine-3-carboxamide | **Thiophenol:** 2-amino-4-chlorothiophenol | MS obsd. (ESI⁺) [(M+H)⁺]: 418.1. |
| | | | ¹H NMR (400 MHz, CD₃OD) δ ppm: 7.78-8.04 (m, 2H), 7.39 (dd, *J=* 8.4, 2.0 Hz, 1H), 4.13-4.27 (m, 1H), 3.82-3.96 (m, 1H), 3.61 (dd, *J* = 13.8, 4.8 Hz, 1H), 3.46-3.56 (m, 1H), 3.30-3.34 (m, 2H), 3.14 (dd, *J* = 15.0, 8.4 Hz, 1H), 2.28-2.73 (m, 8H), 2.04-2.13 (m, 1H), 1.94-2.03 (m, 1H), 1.12 (t, *J* = 7.6 Hz, 3H). |
| | | **Acid:** 1-ethyl-5-oxo-pyrrolidine-3-carboxylic acid | |
| | 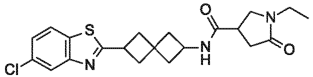 | | |
| 32 | *N*-[6-(5-chloro-1,3-benzothiazol-2-yl)spiro[3.3]heptan-2-yl]-1-methyl-2-oxo-piperidine-4-carboxamide | **Thiophenol:** 2-amino-4-chlorothiophenol | MS obsd. (ESI⁺) [(M+H)⁺]: 418.1. |
| | | | ¹H NMR (400 MHz, CD₃OD) δ ppm: 7.88-7.92 (m, 2H), 7.39 (dd, *J =* 8.6, 2.0 Hz, 1H), 4.13-4.24 (m, 1H), 3.82-3.93 (m, 1H), 3.34-3.40 (m, 2H), 2.92 (s, 3H), 2.58-2.74 (m, 3H), 2.31-2.56 (m, 6H), 2.05-2.13 (m, 1H), 1.83-2.02 (m, 3H). |
| | | **Acid:** 1-methyl-2-oxo-piperidine-4-carboxylic acid | |
| | 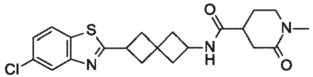 | | |
| 33 | *N*-[6-(5-chloro-1,3-benzothiazol-2-yl)spiro[3.3]heptan-2-yl]-2-(5-oxopyrrolidin-2-yl)acetamide | **Thiophenol:** 2-amino-4-chlorothiophenol | MS obsd. (ESI⁺) [(M+H)⁺]: 404.2. |
| | | | ¹H NMR (400 MHz, CD₃OD) *δ* ppm: 7.99-7.92 (m, 2H), 7.39 (dd, *J* = 8.4, Hz, 1H), 4.19-4.23 (m, 1H), 3.99-4.03 (m, 1H), 3.83-3.93 (m, 1H), 2.59-2.73 (m, 2H), 2.22-2.55 (m, 8H), 1.71-2.15 (m, 4H). |
| | 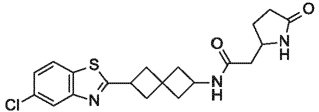 | **Acid:** 2-(5-oxopyrrolidin-2-yl)acetic acid | |
| 34 | *N*-[6-(5-chloro-1,3-benzothiazol-2-yl)spiro[3.3]heptan-2-yl]-1,1-dioxo-1,2-thiazolidine-3-carboxamide | **Thiophenol:** 2-amino-4-chlorothiophenol | MS obsd. (ESI⁺) [(M+H)⁺]: 426.1. |
| | | | ¹H NMR (400 MHz, CD₃OD) *δ* ppm: 7.83-7.97 (m, 2H), 7.39 (dd, *J* = 8.4, 2.0 Hz, 1H), 4.17-4.33 (m, 1H), 4.04 (t, *J* = 8.0 Hz, 1H), 3.88 (p, *J* = 8.5 Hz, 1H), 3.19-3.23 (m, 1H), 3.02-3.07 (m, 1H), 2.30-2.82 (m, 8H), 2.01-2.21 (m, 2H). |
| | | **Acid:** 1,1-dioxo-1,2-thiazolidine-3-carboxylic acid | |
| | 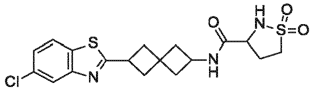 | | |
| 35 | *N*-[6-(5-chloro-1,3-benzothiazol-2-yl)spiro[3.3]heptan-2-yl]-2-(1,1-dioxothiazinan-2-yl)acetamide | **Thiophenol:** 2-amino-4-chlorothiophenol | MS obsd. (ESI⁺) [(M+H)⁺]: 454.1. |
| | | | ¹H NMR (400 MHz, CD₃OD) *δ* ppm: 7.86-7.97 (m, 2H), 7.39 (dd, *J* = 8.6, 1.2 Hz, 1H), 4.19-4.32 (m, 1H), 3.88 (p, *J* = 8.6 Hz, 1H), 3.74-3.78 (m, 2H), 3.39-3.47 (m, 2H), 3.09-3.20 (m, 2H), 2.58-2.73 (m, 2H), 2.52 (dt, *J* = 20.4, 7.6 Hz, 3H), 2.32-2.41 (m, 1H), 2.13-2.22 (m, 3H), 2.07 (dd, *J* = 11.4, 8.8 Hz, 1H), 1.69 (dt, *J* = 11.4, 6.0 Hz, 2H). |
| | | **Acid:** 2-(1,1-dioxothiazinan-2-yl)acetic acid | |
| | 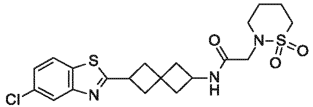 | | |
| 36 | *N*-[6-(5-chloro-1,3-benzothiazol-2-yl)spiro[3.3]heptan-2-yl]-2-methylsulfonyl-pyridine-4-carboxamide | **Thiophenol:** 2-amino-4-chlorothiophenol | MS obsd. (ESI⁺) [(M+H)⁺]: 462.0. |
| | | | ¹H NMR (400 MHz, DMSO-*d₆*) *δ* ppm: 9.24 (d, *J* = 7.2 Hz, 1H), 8.94 (d, J= 5.2 Hz, 1H), 8.44 (s, 1H), 8.07-8.13 (m, 2H), 8.03 (d, *J* = 2.0 Hz, 1H), 7.46 (dd, *J* = 8.6, 2.0 Hz, 1H), 4.30-4.44 (m, 1H), 3.88-3.97 (m, 1H), 3.33 (s, 3H), 2.51-2.73 (m, 3H), 2.41-2.48 (m, 2H), 2.32-2.40 (m, 1H), 2.12-2.29 (m, 2H). |
| | 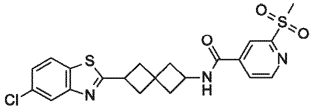 | **Acid:** 2-methylsulfonylpyridine-4-carboxylic acid | |
| 37 | *N*-[6-(6-chloro-1,3-benzothiazol-2-yl)spiro[3.3]heptan-2-yl]-2-cyclopropylsulfonyl-pyridine-4-carboxamide | **Thiophenol:** 2-amino-5-chlorothiophenol | MS obsd. (ESI⁺) [(M+H)⁺]: 488.0. |
| | | | ¹H NMR (400 MHz, CD₃OD) *δ* ppm: 8.89 (d, *J* = 4.8 Hz, 1H), 8.37 (s, 1H), 8.02 (dd, *J* = 5.2, 1.6 Hz, 1H), 7.99 (d, *J* = 2.0 Hz, 1H), 7.86 (d, *J* = 8.8 Hz, 1H), 7.48 (dd, *J =* 8.8, 2.0 Hz, 1H), 4.39-4.51 (m, 1H), 3.85-3.96 (m, 1H), 2.90-2.93 (m, 1H), 2.68-2.78 (m, 2H), 2.43-2.60 (m, 4H), 2.25-2.34 (m, 1H), 2.17-2.20 (m, 1H), 1.25-1.33 (m, 2H), 1.08-1.16 (m, 2H). |
| | | **Acid:** 2-cyclopropylsulfo nylpyridine-4-carboxylic acid | |
| | 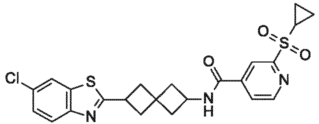 | | |

### Example 38

### 2-tert-butylsulfonyl-N-[6-(5-chloro-1,3-benzothiazol-2-yl)spiro[3.3]heptan-2-yl]pyridine-4-carboxamide

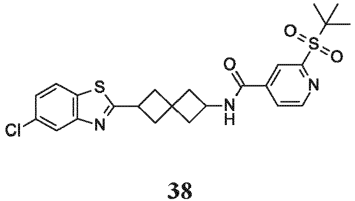

The title compound was prepared according to the following scheme:

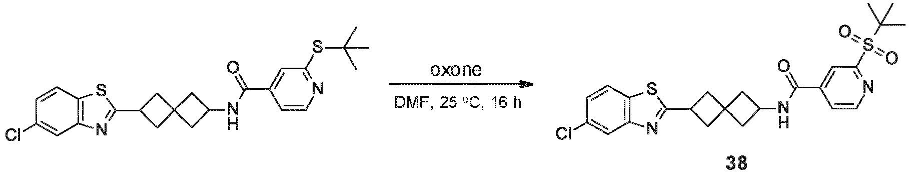

To a solution of 2-*tert*-butylsulfonylpyridine-4-carboxylic acid 2-tert-butylsulfanyl-*N*-[6-(5-chloro-1,3-benzothiazol-2-yl)spiro[3.3]heptan-2-yl]pyridine-4-carboxamide (100.0 mg, prepared in analogy to the procedure described for the preparation of **Example 1,** by using **Int-14** instead of 3-(trifluoromethyl)benzoic acid) in DMF (1 mL) and THF (3 mL) was added Oxone (467.76 mg). The resulting mixture was stirred at ambient temperature for 16 h and then filtered by Celite. The filtrate was concentrated under reduced pressure. The residue was purified by prep-HPLC (column: Gemini - C18, 250 × 30 mm, 5µm; mobile phase: ACN - H₂O (0.05% NH₃); gradient: 55 - 65% ACN, flow rate: 20 mL/min) to give 2-*tert*-butylsulfonyl-*N*-[6-(5-chloro-1,3-benzothiazol-2-yl)spiro[3.3]heptan-2-yl]pyridine-4-carboxamide **(Example 38,** 46 mg) as a white solid. MS obsd. (ESI⁺) [(M+H)⁺]: 504.1. ¹H NMR (400 MHz, CDCl₃) δ ppm: 8.90 (d, *J* = 4.8 Hz, 1H), 8.26 (s, 1H), 7.95 (dd, *J* = 4.8, 1.6 Hz, 2H), 7.75 (d, *J* = 8.4 Hz, 1H), 7.33 (dd, *J* = 8.4, 1.6 Hz, 1H), 6.50 (d, *J=* 7.6 Hz, 1H), 4.54 (d, *J* = 7.6 Hz, 1H), 3.78-3.96 (m, 1H), 2.66-2.84 (m, 2H), 2.49-2.65 (m, 4H), 2.04-2.26 (m, 2H), 1.45 (s, 9H).

### Example 39

### N-[6-(5-chloro-1,3-benzothiazol-2-yl)spiro[3.3]heptan-2-yl]-2-(cyclopropylmethylsulfonyl)pyridine-4-carboxamide

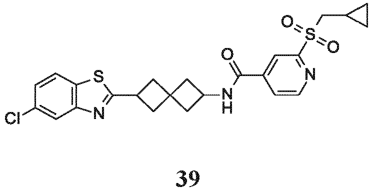

The title compound was prepared in analogy to the procedure described for the preparation of **Example 38,** by using methyl 2-((cyclopropylmethyl)thio)isonicotinate instead of methyl 2-tert-butylsulfanylpyridine-4-carboxylate. The product was purified by preparative HPLC to afford **Example 39** as a white solid. MS obsd. (ESI⁺) [(M+H)⁺]: 502.1. ¹H NMR (400 MHz, CD₃OD) δ ppm: 8.73 (d, *J* = 4.8 Hz, 1H), 8.31 (s, 1H), 7.88 (d, *J* = 4.8 Hz, 1H), 7.73-7.77 (m, 2H), 7.23 (dd, *J* = 8.4, 1.6 Hz, 1H), 4.23-4.34 (m, 1H), 3.70-3.80 (m, 1H), 3.22 (d, *J=* 7.2 Hz, 2H), 2.62-2.52 (m, 2H), 2.28-2.44 (m, 4H), 1.98-2.16 (m, 2H), 0.81-0.85 (m, 1H), 0.34 (q, *J* = 5.8 Hz, 2H), 0.01 (q, *J* = 5.2 Hz, 2H).

### Example 40

### N-[6-(5-chloro-1,3-benzothiazol-2-yl)spiro[3.3]heptan-2-yl]-2-(methylsulfonimidoyl)pyridine-4-carboxamide

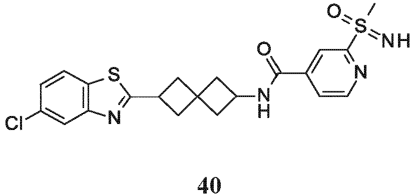

The title compound was prepared in analogy to the procedure described for the preparation of **Example 19,** by using 2-methylsulfanylpyridine-4-carboxylic acid instead of 2-*tert-*butylsulfanylpyridine-4-carboxylic acid. The product was purified by preparative HPLC to afford **Example 40** as a white solid. MS obsd. (ESI⁺) [(M+H)⁺]: 461.0. ¹H NMR (400 MHz, CDCl₃) *δ* ppm: 8.85 (br. s, 1H), 8.47 (br. s, 1H), 7.94-8.00 (m, 2H), 7.74 (d, *J* = 8.4 Hz, 1H), 7.33 (dd, *J=* 8.4, 2.0 Hz, 1H), 7.02 (br. s, 1H), 4.53 (d, *J* = 6.8 Hz, 1H), 3.85 (dd, *J* = 16.8, 8.4 Hz, 1H), 3.39 (s, 3H), 2.55-2.78 (m, 7H), 2.15-2.19 (m, 2H).

### Example 41

### N-6-(5-chloro-1,3-benzothiazol-2-yl)spiro[3.3]heptan-2-yl]-2-(cyclopropylsulfonimidoyl)pyridine-4-carboxamide

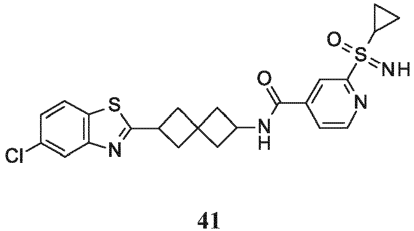

The title compound was prepared in analogy to the procedure described for the preparation of **Example 39,** by using 2-cyclopropylsulfanylpyridine-4-carboxylic acid instead of 2-*tert-*butylsulfanylpyridine-4-carboxylic acid. The product was purified by preparative HPLC to afford **Example 41** as a white solid. MS obsd. (ESI⁺) [(M+H)⁺]: 487.0. ¹H NMR (400 MHz, CDCl₃) *δ* ppm: 8.86 (d, *J* = 4.8 Hz, 1H), 8.29 (s, 1H), 7.89-7.99 (m, 2H), 7.75 (d, *J* = 8.4 Hz, 1H), 7.33 (dd, *J* = 8.4, 2.0 Hz, 1H), 6.65 (d, *J* = 7.2 Hz, 1H), 4.54 (d, *J* = 7.8 Hz, 1H), 3.81-3.92 (m, 1H), 2.83-2.95 (m, 1H), 2.67-2.82 (m, 2H), 2.51-2.64 (m, 3H), 2.12-2.16 (m, 3H), 1.36-1.47 (m, 1H), 0.96-1.29 (m, 3H).

### Example 42

### N-[6-(5-chloro-1,3-benzothiazol-2-yl)spiro[3.3]heptan-2-yl]-2-methoxy-pyridine-4-carboxamide

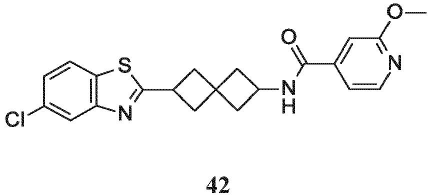

The title compound was prepared according to the following scheme:

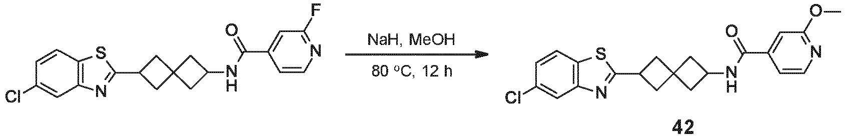

To a solution of *N*-[6-(5-chloro-1,3-benzothiazol-2-yl)spiro[3.3]heptan-2-yl]-2-fluoro-pyridine-4-carboxamide (100.0 mg, prepared in analogy to the procedure described for the preparation of **Example 1,** by using 2-fluoropyridine-4-carboxylic acid instead of 3-(trifluoromethyl)benzoic acid) in methanol (5 mL) was added sodium hydride (17.06 mg, 60% in oil) at 0°C. The reaction mixture was stirred for 12 h at 80 °C. After being cooled to room temperature, water (30 mL) was added and the mixture was extracted with ethyl acetate (3 × 20 mL). The combined organic layer was dried over Na₂SO₄ and concentrated under reduced pressure. The residue was purified by prep-HPLC (Chromatographic columns: Kromasil-C18 100 × 21.2 mm, 5µm; Mobile Phase: CAN-H₂O (0.1% FA); Gradient: 50%-60%) to afford *N*-[6-(5-chloro-1,3-benzothiazol-2-yl)spiro[3.3]heptan-2-yl]-2-methoxy-pyridine-4-carboxamide (14.6 mg) as a white solid. MS obsd. (ESI⁺) [(M+H)⁺]: 414.1. ¹H NMR (400 MHz, CDCl₃) *δ* ppm: 8.23 (d, *J* = 5.0 Hz, 1H), 7.95 (s, 1H), 7.74 (d, *J* = 8.4 Hz, 1H), 7.25-7.28 (m, 1H), 7.15 (d, *J* = 4.6 Hz, 1H), 7.03 (s, 1H), 6.45 (br. s, 1H), 4.50 (dd, *J* = 15.2, 7.6 Hz, 1H), 3.96 (s, 3H), 3.84 (p, *J* = 8.4 Hz, 1H), 2.70-2.74 (m, 2H), 2.49-2.57 (m, 4H), 2.01-2.14 (m, 2H).

The following **Example 43** to **Example 52** were prepared in analogy to the procedure described for the preparation of **Example 42,** replacing methanol with **"Alcohol"** or **"Amine"** as the reagents indicated in Table 2.

**Table 2: Compound synthesis and characterization**

| Examples | Compound Name and Structure | **"Alcohol"** or **"Amine"** | MS (ESI⁺) and ¹H NMR |
|---|---|---|---|
| 43 | *N*-[6-(5-chloro-1,3-benzothiazol-2-yl)spiro[3.3]heptan-2-yl]-2-isopropoxy-pyridine-4-carboxamide | **Alcohol:** 2-propanol | MS obsd. (ESI⁺) [(M+H)⁺]: 442.1. |
| | | | ¹H NMR (400 MHz, CD₃OD) *δ* ppm: 8.20 (d, *J* = 5.2 Hz, 1H), 7.90-7.94 (m, 2H), 7.40 (dd, *J* = 8.4, 1.8 Hz, 1H), 7.21 (d, *J* = 5.2 Hz, 1H), 7.05 (s, 1H), 5.35-5.22 (m, 1H), 4.40 (t, *J* = 8.0 Hz, 1H), 3.91 (t, *J* = 8.4 Hz, 1H), 2.78-2.64 (m, 2H), 2.59-2.40 (m, 4H), 2.30-2.12 (m, 2H), 1.33 (d, *J* = 6.4 Hz, 6H). |
| | 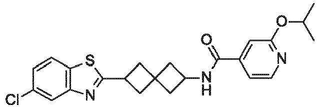 | | |
| 44 | *N*-[6-(5-chloro-1,3-benzothiazol-2-yl)spiro[3.3]heptan-2-yl]-2-(methylamino)pyridine-4-carboxamide | **Amine:** monomethylami ne | MS obsd. (ESI⁺) [(M+H)⁺]: 413.1. |
| | | | ¹H NMR (400 MHz, CD₃OD) δ ppm: 8.01 (d, *J* = 5.4 Hz, 1H), 7.88-7.93 (m, 2H), 7.40 (dd, *J* = 8.6, 2.0 Hz, 1H), 6.83-6.87 (m, 2H), 4.40 (t, *J* = 8.2 Hz, 1H), 3.86-3.96 (m, 1H), 2.90 (s, 3H), 2.66-2.76 (m, 2H), 2.53 (m, 4H), 2.11-2.27 (m, 2H). |
| | 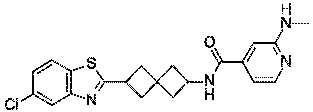 | | |
| 45 | *N*-[6-(5-chloro-1,3-benzothiazol-2-yl)spiro[3.3]heptan-2-yl]-2-(cyclopropylamino)pyridine-4-carboxamide | **Amine:** cyclopropylamine | MS obsd. (ESI⁺) [(M+H)⁺]: 439.1. |
| | | | ¹H NMR (400 MHz, CD₃OD) *δ* ppm: 8.03 (d, *J* = 5.2 Hz, 1H), 7.90-7.93 (m, 2H), 7.39 (dd, *J* = 8.6, 1.8 Hz, 1H), 7.04 (s, 1H), 6.90 (d, *J* = 5.2Hz, 1H), 4.39-4.42 (m, 1H), 3.87-3.93 (m, 1H), 2.69-2.73 (m, 2H), 2.50-2.54 (m, 4H), 2.27-2.15 (m, 2H), 1.26-1.28 (m, 1H), 0.78-0.82 (m, 2H), 0.55-0.50 (m, 2H). |
| | 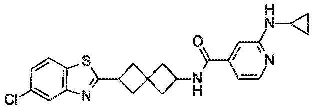 | | |
| 46 | *N*-[6-(5-chloro-1,3-benzothiazol-2-yl)spiro[3.3]heptan-2-yl]-2-(dimethylamino)pyridine-4-carboxamide | **Amine:** dimethylamine | MS obsd. (ESI⁺) [(M+H)⁺]: 427.1. |
| | | | ¹H NMR (400 MHz, CD₃OD) *δ* ppm: 8.12 (d, *J* = 5.2 Hz, 1H), 7.88-7.94 (m, 2H), 7.40 (dd, J = 8.4, 1.8 Hz, 1H), 6.99 (s, 1H), 6.88 (d, *J =* 5.2 Hz, 1H), 4.40-4.45 (m, 1H), 3.88-3.96 (m, 1H), 3.11 (s, 6H), 2.67-2.78 (m, 2H), 2.42-2.58 (m, 4H), 2.14-2.28 (m, 2H). |
| | 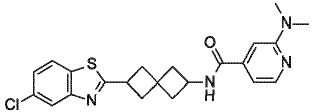 | | |
| 47 | *N*-[6-(5-chloro-1,3-benzothiazol-2-yl)spiro[3.3]heptan-2-yl]-2-[ethyl(methyl)amino]pyridine-4-carboxamide | **Amine:** N-ethylmethylamin e | MS obsd. (ESI⁺) [(M+H)⁺]: 441.1. |
| | | | ¹H NMR (400 MHz, CDCl₃) *δ* ppm: 8.20 (d, *J* = 5.2 Hz, 1H), 7.96 (s, 1H), 7.75 (d, *J* = 7.6 Hz, 1H), 7.33 (d, *J* = 7.6 Hz, 1H), 6.92 (s, 1H), 6.65 (d, *J* = 4.8 Hz, 1H), 6.28 (br. s, 1H), 4.50-5.53 (m, 1H), 3.81-3.91 (m, 1H), 3.63 (q, *J =* 7.0 Hz, 2H), 3.09 (s, 3H), 2.69-2.74 (m, 2H), 2.51-2.57 (m, 4H), 2.02-2.18 (m, 2H), 1.17 (t, *J* = 7.0 Hz, 3H). |
| | 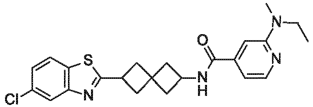 | | |
| 48 | *N*-[6-(5-chloro-1,3-benzothiazol-2-yl)spiro[3.3]heptan-2-yl]-2-(diethylamino)pyridine-4-carboxamide | **Amine:** diethylamine | MS obsd. (ESI⁺) [(M+H)⁺]: 455.1. |
| | 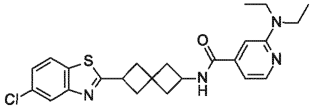 | | ¹H NMR (400 MHz, CD₃OD) *δ* ppm: 8.09 (d, *J =* 5.2 Hz, 1H), 7.90-7.94 (m, 2H), 7.40 (d, *J* = 8.6 Hz, 1H), 6.92 (s, 1H), 6.81 (d, *J =* 5.2 Hz, 1H), 4.40-4.45 (m, 1H), 3.86-3.97 (m, 1H), 3.56 (q, *J* = 6.8 Hz, 4H), 2.65-2.79 (m, 2H), 2.50 (m, 4H), 2.12-2.31 (m, 2H), 1.18 (t, *J =* 6.8 Hz, 6H). |
| 49 | *N*-[6-(5-chloro-1,3-benzothiazol-2-yl)spiro[3.3]heptan-2-yl]-2-[isopropyl(methyl)amino]pyrid ine-4-carboxamide | **Amine:** *N-*isopropylmethyl amine | MS obsd. (ESI⁺) [(M+H)⁺]: 455.1. |
| | | | ¹H NMR (400 MHz, CD₃OD) *δ* ppm: 8.11 (d, *J* = 5.2 Hz, 1H), 7.88-7.93 (m, 2H), 7.40 (dd, *J* = 8.6, 2.0 Hz, 1H), 6.97 (s, 1H), 6.85 (dd, *J =* 5.2*,* 1.2 Hz, 1H), 4.79 (dt, *J* = 13.2, 6.7 Hz, 1H), 4.41 (p, *J* = 8.2 Hz, 1H), 3.91 (p, *J* = 8.6 Hz, 1H), 2.89 (s, 3H), 2.64-2.78 (m, 2H), 2.41-2.60 (m, 4H), 2.12-2.31 (m, 2H), 1.20 (d, *J* = 6.6 Hz, 6H). |
| | 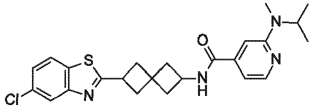 | | |
| 50 | *N*-[6-(5-chloro-1,3-benzothiazol-2-yl)spiro[3.3]heptan-2-yl]-2-pyrrolidin-1-yl-pyridine-4-carboxamide | **Amine:** pyrrolidine | MS obsd. (ESI⁺) [(M+H)⁺]: 453.1. |
| | | | ¹H NMR (400 MHz, CDCl₃) *δ* ppm: 8.19 (d, *J =* 5.2 Hz, 1H), 7.95 (s, 1H), 7.74 (d, *J =* 8.6 Hz, 1H), 7.32 (d, *J* = 8.6 Hz, 1H), 6.75 (s, 1H), 6.65 (d, *J* = 5.2 Hz, 1H), 6.29 (d, *J =* 6.2 Hz, 1H), 4.51 (dd, *J =* 15.8, 8.0 Hz, 1H), 3.82-3.90 (m, 1H), 3.45-3.52 (m, 4H), 2.67-2.78 (m, 2H), 2.52-2.57 (m, 4H), 2.10-2.15 (m, 2H), 2.02-2.06 (m, 2H), 1.99-2.02 (m, 2H). |
| | 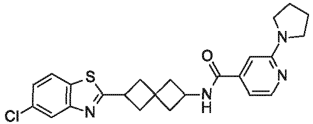 | | |
| 51 | *N*-[6-(5-chloro-1,3-benzothiazol-2-yl)spiro[3.3]heptan-2-yl]-2-(1-piperidyl)pyridine-4-carboxamide | **Amine:** piperidine | MS obsd. (ESI⁺) [(M+H)⁺]: 467.1. |
| | | | ¹H NMR (400 MHz, CD₃OD) *δ* ppm: 8.13 (d, *J =* 5.2 Hz, 1H), 7.87-7.96 (m, 2H), 7.40 (dd, *J =* 8.6, 2.0 Hz, 1H), 7.12 (s, 1H), 6.90 (d, *J =* 5.2 Hz, 1H), 4.41 (t, *J* = 8.2 Hz, 1H), 3.84-3.96 (m, 1H), 3.55-3.60 (m, 4H), 2.64-2.77 (m, 2H), 2.42-2.60 (m, 4H), 2.12-2.31 (m, 2H), 1.59-1.71 (m, 6H). |
| | 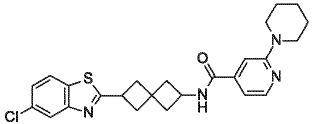 | | |
| 52 | *N*-[6-(5-chloro-1,3-benzothiazol-2-yl)spiro[3.3]heptan-2-yl]-2-morpholino-pyridine-4-carboxamide | **Amine:** morpholine | MS obsd. (ESI⁺) [(M+H)⁺]: 469.1. |
| | | | ¹H NMR (400 MHz, CD₃OD) *δ* ppm: 8.20 (d, *J* = 5.2 Hz, 1H), 7.89-7.93 (m, 2H), 7.40 (dd, *J* = 8.6, 2.0 Hz, 1H), 7.13 (s, 1H), 7.01 (d, *J =* 5.2 Hz, 1H), 4.41 (t, *J* = 8.2 Hz, 1H), 3.85-3.94 (m, 1H), 3.76-3.83 (m, 4H), 3.50-3.56 (m, 4H), 2.66-2.77 (m, 2H), 2.42-2.60 (m, 4H), 2.12-2.30 (m, 2H). |
| | 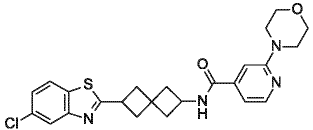 | | |

### Example 53-a and Example 53-b

The two enantiomers **(Example 53-a** and **Example 53-b)** were obtained through SFC chiral separation of *N4*-[6-(5-chloro-1,3-benzothiazol-2-yl)spiro[3 .3]heptan-2-yl]pyridine-2,4-dicarboxamide **(Example 11). Example 53-a** was eluted out before **Example 53-b.** SFC conditions: Instrument: Thar 350 preparative SFC (SFC-10); Column: ChiralPak AD, 300×50mm I.D., 10µm; Mobile phase: A for CO₂ and B for Methanol; Gradient: B 40%; Flow rate: 200 mL/min; Back pressure: 100 bar; Column temperature: 38 °C; Wavelength: 254nm.

### (Sₐ)-N4-[6-(5-chloro-1,3-benzothiazol-2-yl)spiro[3.3]heptan-2-yl]pyridine-2,4-dicarboxamide

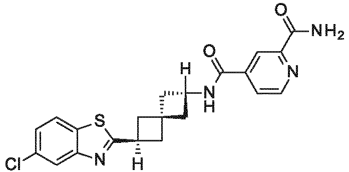

### (Rₐ)-N4-[6-(5-chloro-1,3-benzothiazol-2-yl)spiro[3.3]heptan-2-yl]pyridine-2,4-dicarboxamide

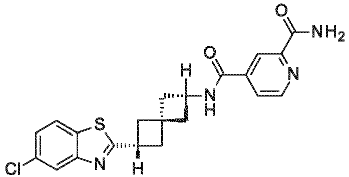

**Example 53-a:** white solid. MS obsd. (ESI⁺) [(M+H)⁺]: 427.0. ¹H NMR (400 MHz, DMSO-*d₆*) *δ* ppm: 9.12 (d, *J =* 7.2 Hz, 1H), 8.77 (d, *J* = 5.2 Hz, 1H), 8.46 (d, *J =* 0.8 Hz, 1H), 8.20 (d, *J =* 2.0 Hz, 1H), 8.10 (d, *J* = 8.6 Hz, 1H), 8.03 (d, *J* = 2.0 Hz, 1H), 7.94 (dd, *J* = 5.2, 2.0 Hz, 1H), 7.76 (d, *J* = 2.0 Hz, 1H), 7.46 (dd, *J* = 8.6, 2.0 Hz, 1H), 4.37 (d, *J =* 7.8 Hz, 1H), 3.92 (t, *J* = 8.4 Hz, 1H), 2.65-2.69 (m, 3H), 2.10-2.47 (m, 5H).

**Example 53-b**: white solid. MS obsd. (ESI⁺) [(M+H)⁺]: 427.0. ¹H NMR (400 MHz, DMSO-*d₆*): *δ* ppm: 9.13 (d, *J* = 7.2 Hz, 1H), 8.77 (d, *J =* 5.2 Hz, 1H), 8.47 (d, *J =* 0.8 Hz, 1H), 8.21 (d, *J =* 1.6 Hz, 1H), 8.05-8.09 (m, 2H), 7.95 (dd, *J* = 5.2, 1.6 Hz, 1H), 7.77 (d, *J* = 2.0 Hz, 1H), 7.46 (dd, *J* = 8.6, 2.0 Hz, 1H), 4.38 (dd, *J* = 15.8, 8.0 Hz, 1H), 3.91 (dd, *J =* 17.0, 8.6 Hz, 1H), 2.52-2.72 (m, 3H), 2.11-2.49 (m, 5H).

### Example 54-a and Example 54-b

The two enantiomers **(Example 54-a** and **Example 54-b)** were obtained through SFC chiral separation of *N*-[6-(5-chloro-1,3-benzothiazol-2-yl)spiro[3.3]heptan-2-yl]-2-cyclopropylsulfonyl-pyridine-4-carboxamide **(Example 36). Example 54-a** was eluted out before **Example 54-b.** SFC conditions: Instrument: MG II preparative SFC (SFC-1); Column: ChiralPak AD, 250×30mm I.D., 5µm; Mobile phase: A for CO₂ and B for Ethanol; Gradient: B 45%; Flow rate: 50 mL/min; Back pressure: 100 bar; Column temperature: 38 °C; Wavelength: 254nm.

### (Sₐ)-N-[6-(5-chloro-1,3-benzothiazol-2-yl)spiro[3.3]heptan-2-yl]-2-methylsulfonyl-pyridine-4-carboxamide

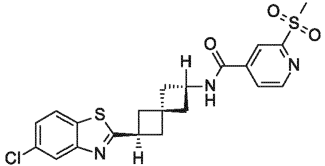

### (Rₐ)-N-[6-(5-chloro-1,3-benzothiazol-2-yl)spiro[3.3]heptan-2-yl]-2-methylsulfonyl-pyridine-4-carboxamide

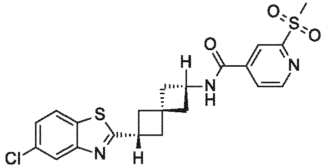

**Example 54-a:** white solid. MS obsd. (ESI⁺) [(M+H)⁺]: 462.0. ¹H NMR (400 MHz, CDCl₃) *δ* ppm: 8.86 (d, *J =* 4.8 Hz, 1H), 8.33 (s, 1H), 8.13 (s, 1H), 8.02 (d, *J* = 4.8 Hz, 1H), 7.79 (d, *J* = 8.6 Hz, 1H), 7.43 (d, *J* = 8.6 Hz, 1H), 6.74 (s, 1H), 4.53-4.57 (m, 1H), 3.97-4.06 (m, 1H), 3.27 (s, 3H), 2.59-2.82 (m, 6H), 2.17-2.20 (m, 2H).

**Example 54-b:** white solid. MS obsd. (ESI⁺) [(M+H)⁺]: 462.1. ¹H NMR (400 MHz, CDCl₃) *δ* ppm: 8.86 (d, *J* = 4.8 Hz, 1H), 8.30 (s, 1H), 7.97-8.05 (m, 2H), 7.77 (d, *J* = 8.6 Hz, 1H), 7.37 (dd, *J* = 8.6, 2.0 Hz, 1H), 6.62 (d, *J =* 7.2 Hz, 1H), 4.54 (d, *J* = 7.8 Hz, 1H), 3.86-4.02 (m, 1H), 2.75 (dd, *J* = 11.6, 7.6 Hz, 2H), 3.27 (s, 3H), 2.49-2.64 (m, 4H), 2.06-2.26 (m, 2H).

### Example 55, Example 55-a and Example 55-b

**Example 55, *N*-[6-(5-chloro-1,3-benzothiazol-2-yl)spiro[3.3]heptan-2-yl]-2-cyclopropylsulfonyl-pyridine-4-carboxamide,** was prepared in analogy to the procedure described for the preparation of **Example 1,** by using 2-cyclopropylsulfonylpyridine-4-carboxylic acid instead of 3-(trifluoromethyl)benzoic acid.

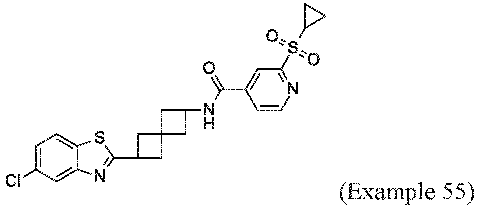

The two enantiomers **(Example 55-a** and **Example 55-b)** were obtained through SFC chiral separation of Example 55. **Example 55-a** was eluted out before **Example 55-b.** SFC conditions: Instrument: MG II preparative SFC (SFC-11); Column: ChiralPak AD, 250×30mm I.D., 10µm; Mobile phase: A for CO₂ and B for Methanol; Gradient: B 50%; Flow rate: 70 mL/min; Back pressure: 100 bar; Column temperature: 38 °C; Wavelength: 254nm

### (Sₐ)-N-[6-(5-chloro-1,3-benzothiazol-2-yl)spiro[3.3]heptan-2-yl]-2-cyclopropylsulfonyl-pyridine-4-carboxamide

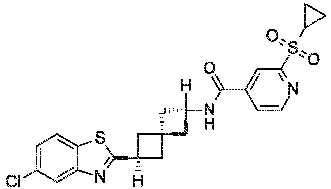

### (Rₐ)-N-[6-(5-chloro-1,3-benzothiazol-2-yl)spiro[3.3]heptan-2-yl]-2-cyclopropylsulfonyl-pyridine-4-carboxamide

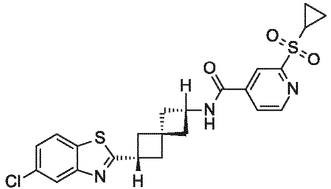

**Example 55-a:** white solid. MS obsd. (ESI⁺) [(M+H)⁺]: 488.0. ¹HNMR (400 MHz, CDCl₃) *δ* ppm: 8.88 (d, *J* = 4.8 Hz, 1H), 8.22 (s, 1H), 7.97-8.01 (m, 2H), 7.76 (d, *J* = 8.4 Hz, 1H), 7.36 (dd, *J =* 8.4, 2.0 Hz, 1H), 6.62 (s, 1H), 4.52-4.56 (m, 1H), 3.90-3.94 (m, 1H), 2.56-2.88 (m, 6H), 2.05-2.28 (m, 3H), 1.38-1.42 (m, 2H), 1.10-1.14 (m, 2H).

**Example 55-b:** white solid. MS obsd. (ESI⁺) [(M+H)⁺]:488.1. ¹H NMR (400 MHz, CDCl₃) *δ* ppm: 8.87 (s, 1H), 7.64-8.36 (m, 4H), 7.35 (s, 1H), 6.69 (br. s, 1H), 4.56-4.59 (m, 1H), 3.91-3.94 (m, 1H), 2.57-2.65 (m, 9H), 0.96-1.49 (m, 4H).

### Example 56, Example 56-a and Example 56-b

### N6-[6-(5-chloro-1,3-benzothiazol-2-yl)spiro[3.3]heptan-2-yl]pyrimidine-4,6-dicarboxamide

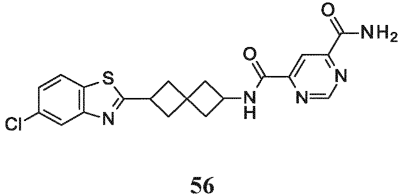

The title compound was prepared according to the following scheme:

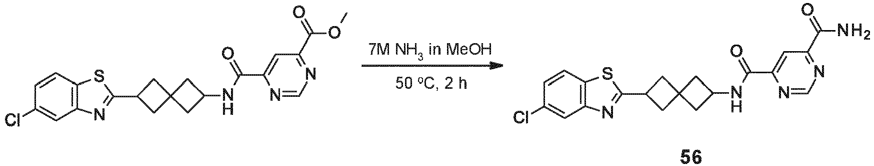

A solution of methyl 6-[[6-(5-chloro-1,3-benzothiazol-2-yl)spiro[3.3]heptan-2-yl]carbamoyl]pyrimidine-4-carboxylate (850.0 mg, prepared in analogy to the procedure described for the preparation of **Example 1,** by using 6-methoxycarbonylpyrimidine-4-carboxylic acid instead of 3-(trifluoromethyl)benzoic acid) in the solution of ammonia in methanol **(Example 56,** 10.0 mL, 7M) was stirred at 50 °C for 2 h. The resulted mixture was concentrated under reduced pressure.

The two enantiomers **(Example 56-a** and **Example 56-b)** were obtained through SFC chiral separation of **Example 56. Example 56-a** was eluted out before **Example 56-b.** SFC conditions: Instrument: MG II preparative SFC (SFC-11); Column: ChiralPak AD, 250×30mm I.D., 5µm; Mobile phase: A for CO₂ and B for Ethanol, Gradient: B 50%; Flow rate: 50 ml/min; Wave length: 254 nm; Temperature: 38 °C.

### (Sₐ)-N6-[6-(5-chloro-1,3-benzothiazol-2-yl)spiro[3.3]heptan-2-yl]pyrimidine-4,6-dicarboxamide

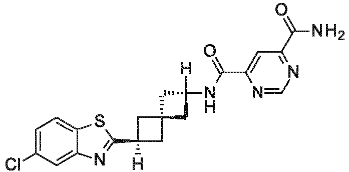

### (Rₐ)-N6-[6-(5-chloro-1,3-benzothiazol-2-yl)spiro[3.3]heptan-2-yl]pyrimidine-4,6-dicarboxamide

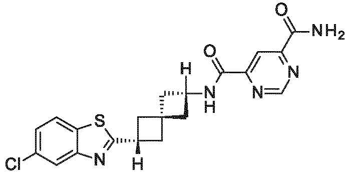

**Example 56-a:** light yellow solid. MS obsd. (ESI⁺) [(M+H)⁺]: 428.0. ¹H NMR (400 MHz, DMSO-*d₆*) *δ* ppm: 9.42 (d, *J* = 0.8 Hz, 1H), 9.29 (d, *J* = 8.0 Hz, 1H), 8.42 (s, 2H), 7.98-8.17 (m, 3H), 7.46 (dd, *J* = 8.6, 2.0 Hz, 1H), 4.33-4.49 (m, 1H), 3.91 (p, *J* = 8.6 Hz, 1H), 2.62-2.72 (m, 1H), 2.53-2.59 (m, 1H), 2.33-2.49 (m, 4H), 2.19-2.33 (m, 2H).

**Example 56-b:** white solid. MS obsd. (ESI⁺) [(M+H)⁺]: 428.1. ¹H NMR (400 MHz, DMSO-*d₆*) *δ* ppm: 9.42 (s, 1H), 9.30 (d, *J* = 8.0 Hz, 1H), 8.43 (s, 2H), 7.97-8.16 (m, 3H), 7.46 (dd, *J* = 8.6, 2.0 Hz, 1H), 4.33-4.48 (m, 1H), 3.85-3.98 (m, 1H), 2.62-2.75 (m, 1H), 2.53 - 2.60 (m, 1H), 2.37-2.43 (m, 4H), 2.14-2.33 (m, 2H)..

### BIOLOGICAL EXAMPLES

### Example 57

### PHH Natural Infection Assay

Detailed procedures regarding primary human hepatocyte (PHH) HBV natural infection assay are described as below. One tube of frozen PHH (10 million cells) is thawed in 37 °C water bath and then transferred to 20 mL of PHH thawing medium (Sigma, InVitroGRO HT Medium, Cat. S03319) with gently mixing. The cells were then centrifuged at 80 g/min for 5 min, the supernatant was discarded and the tube was refilled with 25 mL of PHH plating medium (Sigma , InVitroGRO CP Medium, Cat. S03317). The tube was shaken very gently to re-suspend all cells, and then 50 µl of cells were transferred to each well 384-well collagen I coated plate with appropriate liquid handling equipment, e.g. Integra VIAFLO384 or Agilent Bravo. The cells were then cultured for 24 hours in a cell incubator. For HBV infection, after PHH attachment on the culture plate, the plating medium was removed and replenished with PHH culture medium containing HBV virus. The PHH culture medium was prepared with Dulbecco's Modified Eagle Medium (DMEM)/F12 (1: 1 in volume ratio) containing 10% fetal bovine serum (Gibco, Cat.10099141), 5 ng/mL human epidermal growth factor (Gibco, Cat.PHG0311L), 20 ng/mL dexamethasone (Sigma, Cat.D4902-100mg), 250 ng/mL human recombinant insulin (Gibco, Cat.41400045) and 100 U/mL penicillin. HBV virus at 200 genome equivalent (GE) per cell with 4% PEG8000 (Sigma, Cat.P1458) containing culture medium were added to the PHH culture medium for infection. The cells were then cultured for 24 hours in cell incubator. Then the cell culture supernatant was removed. The HBV-infected PHH were cultured with sandwich culture method with PHH culture medium containing 1% DMSO and 0.25 mg/mL matrix gel for 72 hours. The supernatant was then refreshed with PHH culture medium containing different concentrations of testing compounds for two times with 72-hour interval. At the end of treatment, the supernatant was collected for viral markers measurements, including HBsAg, HBeAg, HBV DNA and cytotoxicity. HBsAg and HBeAg were detected using alphalisa method using their specific antibodies. For HBV DNA detection, HBV DNA Quantitative Fluorescence Diagnostic Kit (Sansure Biotech Inc.) was used following the manufacture's protocol. Cytotoxicity was determined using Cell Counting Kit-8 (CCK8, Dojindo Molecular Technologies, Inc.).

The compounds of the present invention were tested for their capacity to inhibit HBsAg and HBeAg as described herein. The Examples were tested in the above assay and found to have IC₅₀ below 10 µM. Results of PHH assay are given in Table 1.

**Table 1: Activity data of compounds of this invention**

| Example No. | HBsAg IC₅₀ (µM) | HBeAg IC₅₀ (µM) | CC₅₀ (µM) |
|---|---|---|---|
| Example 1 | 6.55 | 4.53 | >10.00 |
| Example 2 | 3.71 | 3.33 | >10.00 |
| Example 3 | 6.37 | 4.39 | >10.00 |
| Example 4 | 5.05 | 4.34 | >10.00 |
| Example 5 | 3.47 | 2.92 | >10.00 |
| Example 6 | 3.63 | 3.30 | >10.00 |
| Example 7 | 3.33 | 3.09 | >10.00 |
| Example 8 | 1.89 | 1.23 | 35.90 |
| Example 9 | 1.35 | 1.21 | 13.40 |
| Example 10 | 7.70 | 8.43 | >50.00 |
| Example 11 | 4.06 | 6.81 | >50.00 |
| Example 12 | 6.58 | 6.53 | 23.30 |
| Example 13 | 0.48 | 0.36 | 94.90 |
| Example 14 | 2.48 | 2.52 | >100.00 |
| Example 15 | 2.96 | 2.07 | >100.00 |
| Example 16 | 2.69 | 1.92 | 23.87 |
| Example 17 | 2.11 | 1.60 | 35.70 |
| Example 18 | 5.52 | 4.59 | >10.00 |
| Example 19 | 7.55 | 6.83 | 31.50 |
| Example 20 | 4.88 | 4.06 | >10.00 |
| Example 21 | 1.49 | 1.14 | >10.00 |
| Example 22 | 1.39 | 1.00 | 21.90 |
| Example 23 | 1.25 | 0.85 | 14.30 |
| Example 24 | 1.40 | 1.04 | 19.70 |
| Example 25 | 2.59 | 2.07 | >10.00 |
| Example 26 | 1.99 | 1.18 | >100.00 |
| Example 27 | 1.15 | 0.56 | >100.00 |
| Example 28 | 3.28 | 1.66 | >10.00 |
| Example 29 | 4.74 | 3.59 | >10.00 |
| Example 30 | 3.47 | 3.47 | >10.00 |
| Example 31 | 9.58 | 8.29 | >10.00 |
| Example 32 | 6.11 | 4.13 | >10.00 |
| Example 33 | 4.08 | 4.12 | >10.00 |
| Example 34 | 7.11 | 5.67 | >10.00 |
| Example 35 | 3.18 | 2.98 | >10.00 |
| Example 36 | 1.08 | 0.86 | >50.00 |
| Example 37 | 0.23 | 0.20 | 10.10 |
| Example 38 | 1.95 | 1.49 | 30.80 |
| Example 39 | 0.79 | 0.34 | >10.00 |
| Example 40 | 2.53 | 2.41 | >10.00 |
| Example 41 | 0.90 | 0.67 | >10.00 |
| Example 42 | 3.12 | 2.67 | >10.00 |
| Example 43 | 3.23 | 2.86 | >10.00 |
| Example 44 | 3.24 | 3.18 | >10.00 |
| Example 45 | 6.08 | 5.18 | >10.00 |
| Example 46 | 4.20 | 3.35 | >10.00 |
| Example 47 | 5.85 | 4.80 | >10.00 |
| Example 48 | 5.92 | 4.96 | >10.00 |
| Example 49 | 3.14 | 2.38 | >10.00 |
| Example 50 | 9.95 | 9.63 | >10.00 |
| Example 51 | 5.51 | 4.35 | >10.00 |
| Example 52 | 2.59 | 2.79 | >10.00 |
| Example 53-a | 0.29 | 0.24 | 36.80 |
| Example 53-b | 5.96 | 5.25 | >10.00 |
| Example 54-a | 2.46 | 1.79 | >10.00 |
| Example 54-b | 0.28 | 0.23 | 15.30 |
| Example 55-a | 1.56 | 1.09 | 22.50 |
| Example 55-b | 0.54 | 0.40 | 11.60 |
| Example 56-a | 3.29 | 2.47 | >10.00 |
| Example 56-b | 3.30 | 2.27 | >10.00 |

## Claims

1. A compound of the formula (I), 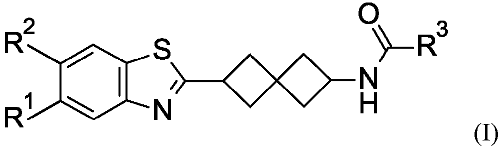 wherein
R¹ is H or halogen;
R² is H or halogen;
or R¹ and R² together with the phenyl they are attached to form a 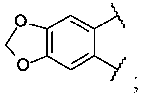
R³ is (1,1-dioxothian-3-yl)C₁₋₆alkyl;
(1,1-dioxothiazinan-2-yl)C₁₋₆alkyl;
(1,1-dioxothietan-3-yl)C₁₋₆alkyl;
(1,1-dioxothiolan-2-yl)C₁₋₆alkyl;
(5-oxopyrrolidin-2-yl)C₁₋₆alkyl;
1,1-dioxo-1,2-thiazolidinyl;
1,1-dioxo-thietanyl;
1,1-dioxo-thiolanyl;
aminobenzothiazolyl;
C₁₋₆alkyl-2-oxo-piperidinyl;
C₁₋₆alkyl-5-oxo-pyrrolidinyl;
furanyl which is substituted by ((haloC₁₋₆alkyl)C₃₋₇cycloalkyl)sulfamoyl, (haloazetidinyl)sulfonyl, (haloC₃₋₇cycloalkyl)sulfamoyl, azetidin-1-ylsulfonyl, C₁₋₆alkylsulfonylC₁₋₆alkyl, C₃₋₇cycloalkylsulfamoyl, C₃₋₇cycloalkylsulfonyl, haloC₁₋₆alkyl or hydroxyhaloC₁₋₆alkyl;
phenyl which is substituted by (C₁₋₆alkyl)₂phosphoryl, haloC₁₋₆alkyl or sulfamoyl;
pyridinyl which is substituted by (C₁₋₆alkyl)₂amino, C₁₋₆alkoxy, C₁₋₆alkyl, C₁₋₆alkylamino, C₁₋₆alkylsulfonimidoyl, C₁₋₆alkylsulfonyl, C₁₋₆alkylsulfonylC₁₋₆alkyl, C₃₋₇cycloalkylamino, C₃₋₇cycloalkylC₁₋₆alkylsulfonyl, C₃₋₇cycloalkylsulfonimidoyl, C₃₋₇cycloalkylsulfonyl, carbamoyl, cyano, haloC₁₋₆alkyl, morpholinyl, piperidinyl, pyrrolidinyl or ureido; or
pyrimidinyl which is substituted by carbamoyl;
with the proviso that R¹ and R² are not H or halogen simultaneously;
or a pharmaceutically acceptable salt thereof.

2. A compound according to claim 1, wherein
R¹ is H or chloro;
R² is H or chloro;
or R¹ and R² together with the phenyl they are attached to form a 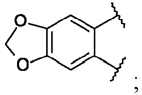
R³ is (1,1-dioxothian-3-yl)methyl;
(1,1-dioxothiazinan-2-yl)methyl;
(1,1-dioxothietan-3-yl)methyl;
(1,1-dioxothiolan-2-yl)methyl;
(5-oxopyrrolidin-2-yl)methyl;
1,1-dioxo-1,2-thiazolidinyl;
1,1-dioxo-thietanyl;
1,1-dioxo-thiolanyl;
aminobenzothiazolyl;
1-ethyl-5-oxo-pyrrolidinyl;
1-methyl-2-oxo-piperidinyl;
((3-(trifluoromethyl)cyclobutyl)sulfamoyl)furanyl;
(1-methyl-1-methylsulfonyl-ethyl)furanyl;
(1-methylsulfonylethyl)furanyl;
(2,2,2-trifluoro-1-hydroxy-ethyl)furanyl;
(3,3-difluoroazetidin-1-yl)sulfonylfuranyl;
(azetidin-1-ylsulfonyl)furanyl;
(cyclobutylsulfamoyl)furanyl;
(trifluoromethyl)furanyl;
[(3,3-difluorocyclobutyl)sulfamoyl]furanyl;
cyclopropylsulfonylfuranyl;
dimethylphosphorylphenyl;
sulfamoylphenyl;
trifluoromethylphenyl;
(1-methyl-1-methylsulfonyl-ethyl)pyridinyl;
(1-piperidinyl)pyridinyl;
(cyclopropylamino)pyridinyl;
(cyclopropylmethylsulfonyl)pyridinyl;
(cyclopropylsulfonimidoyl)pyridinyl;
(diethylamino)pyridinyl;
(dimethylamino)pyridinyl;
(methylamino)pyridinyl;
(methylsulfonimidoyl)pyridinyl;
(methylsulfonylmethyl)pyridinyl;
[ethyl(methyl)amino]pyridinyl;
[isopropyl(methyl)amino]pyridinyl;
carbamoylpyridinyl;
cyanopyridinyl;
cyclopropylsulfonylpyridinyl;
ethylsulfonylpyridinyl;
isopropoxypyridinyl;
mehtylpyridinyl;
methoxypyridinyl;
methylsulfonylpyridinyl;
morpholinylpyridinyl;
pyrrolidin-1-ylpyridinyl;
tert-butylsulfonylpyridinyl;
trifluoromethylpyridinyl;
ureidopyridinyl; or
carbamoylpyrimidinyl;
with the proviso that R¹ and R² are not H or halogen simultaneously;
or a pharmaceutically acceptable salt thereof.

3. A compound according to claim 1, or a pharmaceutically acceptable salt thereof, wherein R¹ is halogen.

4. A compound according to any one of claims 1-3, or a pharmaceutically acceptable salt thereof, wherein R¹ is chloro.

5. A compound according to any one of claims 1-4, or a pharmaceutically acceptable salt thereof, wherein R² is H.

6. A compound according to any one of claims 1, 3-5, or a pharmaceutically acceptable salt thereof, wherein
R³ is furanyl which is substituted by C₃₋₇cycloalkylsulfonyl; or
pyridinyl which is substituted by C₁₋₆alkylsulfonyl, C₃₋₇cycloalkylC₁₋₆alkylsulfonyl, C₃₋₇cycloalkylsulfonimidoyl, C₃₋₇cycloalkylsulfonyl or carbamoyl.

7. A compound according to any one of claims 1-6, or a pharmaceutically acceptable salt thereof, wherein R³ is cyclopropylsulfonylfuranyl, cyclopropylsulfonylpyridinyl, (cyclopropylmethylsulfonyl)pyridinyl, (cyclopropylsulfonimidoyl)pyridinyl, carbamoylpyridinyl or methylsulfonylpyridinyl.

8. A compound according to any one of claims 1, 3, 5, and 6, wherein
R¹ is halogen;
R² is H;
R³ is furanyl which is substituted by C₃₋₇cycloalkylsulfonyl; or
pyridinyl which is substituted by C₁₋₆alkylsulfonyl, C₃₋₇cycloalkylC₁₋₆alkylsulfonyl, C₃₋₇cycloalkylsulfonimidoyl, C₃₋₇cycloalkylsulfonyl or carbamoyl;
or a pharmaceutically acceptable salt thereof.

9. A compound according to any one of claims 1-8, wherein
R¹ is chloro;
R² is H;
R³ is cyclopropylsulfonylfuranyl, cyclopropylsulfonylpyridinyl, (cyclopropylmethylsulfonyl)pyridinyl, (cyclopropylsulfonimidoyl)pyridinyl, carbamoylpyridinyl or methylsulfonylpyridinyl;
or a pharmaceutically acceptable salt thereof.

10. A compound according to any of claims 1-9, selected from:
*N*-[6-(5-chloro-1,3-benzothiazol-2-yl)spiro[3.3]heptan-2-yl]-3-(trifluoromethyl)benzamide;
*N*-[6-(5-chloro-1,3-benzothiazol-2-yl)spiro[3.3]heptan-2-yl]-3-sulfamoyl-benzamide;
*N*-[6-(5-chloro-1,3-benzothiazol-2-yl)spiro[3.3]heptan-2-yl]-3-dimethylphosphoryl-benzamide;
*N*-[6-(5-chloro-1,3-benzothiazol-2-yl)spiro[3.3]heptan-2-yl]-2-(trifluoromethyl)pyridine-4-carboxamide;
*N*-[6-(5-chloro-1,3-benzothiazol-2-yl)spiro[3.3]heptan-2-yl]-2-cyano-pyridine-4-carboxamide;
*N*-[6-(5-chloro-1,3-benzothiazol-2-yl)spiro[3.3]heptan-2-yl]-2-methyl-pyridine-4-carboxamide;
N-[6-(5-chloro-1,3-benzothiazol-2-yl)spiro[3.3]heptan-2-yl]-2-(methylsulfonylmethyl)pyridine-4-carboxamide;
*N*-[6-(5-chloro-1,3-benzothiazol-2-yl)spiro[3.3]heptan-2-yl]-2-(1-methyl-1-methylsulfonylethyl)pyridine-4-carboxamide;
*N*-[6-(5-chloro-1,3-benzothiazol-2-yl)spiro[3.3]heptan-2-yl]-2-ethylsulfonyl-pyridine-4-carboxamide;
*N*-[6-(5-chloro-1,3-benzothiazol-2-yl)spiro[3.3]heptan-2-yl]-2-ureido-pyridine-4-carboxamide;
*N4*-[6-(5-chloro-1,3-benzothiazol-2-yl)spiro[3.3]heptan-2-yl]pyridine-2,4-dicarboxamide;
*N4*-[6-([1,3]dioxolo[4,5-f][1,3]benzothiazol-6-yl)spiro[3.3]heptan-2-yl]pyridine-2,4-dicarboxamide;
*N*-[6-(5-chloro-1,3-benzothiazol-2-yl)spiro[3.3]heptan-2-yl]-5-cyclopropylsulfonyl-furan-2-carboxamide;
*N*-[6-(5-chloro-1,3-benzothiazol-2-yl)spiro[3.3]heptan-2-yl]-5-(trifluoromethyl)furan-2-carboxamide;
*N*-[6-(6-chloro-1,3-benzothiazol-2-yl)spiro[3.3]heptan-2-yl]-5-(trifluoromethyl)furan-2-carboxamide;
*N*-[6-(5-chloro-1,3-benzothiazol-2-yl)spiro[3.3]heptan-2-yl]-5-(1-methylsulfonylethyl)furan-2-carboxamide;
*N*-[6-(5-chloro-1,3-benzothiazol-2-yl)spiro[3.3]heptan-2-yl]-5-(1-methyl-1-methylsulfonylethyl)furan-2-carboxamide;
*N*-[6-(5-chloro-1,3-benzothiazol-2-yl)spiro[3.3]heptan-2-yl]-5-(2,2,2-trifluoro-1-hydroxyethyl)furan-2-carboxamide;
5-(azetidin-1-ylsulfonyl)-*N*-[6-(5-chloro-1,3-benzothiazol-2-yl)spiro[3.3]heptan-2-yl]furan-2-carboxamide;
*N*-[6-(5-chloro-1,3-benzothiazol-2-yl)spiro[3.3]heptan-2-yl]-5-(3,3-difluoroazetidin-1-yl)sulfonyl-furan-2-carboxamide;
*N*-[6-(5-chloro-1,3-benzothiazol-2-yl)spiro[3.3]heptan-2-yl]-5-(cyclobutylsulfamoyl)furan-2-carboxamide;
*N*-[6-(5-chloro-1,3-benzothiazol-2-yl)spiro[3.3]heptan-2-yl]-5-[(3,3-difluorocyclobutyl)sulfamoyl]furan-2-carboxamide;
*N*-[6-(5-chloro-1,3-benzothiazol-2-yl)spiro[3.3]heptan-2-yl]-5-[[3-(trifluoromethyl)cyclobutyl]sulfamoyl]furan-2-carboxamide;
*N*-[6-(5-chloro-1,3-benzothiazol-2-yl)spiro[3.3]heptan-2-yl]-1,1-dioxo-thietane-3-carboxamide;
*N*-[6-(5-chloro-1,3-benzothiazol-2-yl)spiro[3.3]heptan-2-yl]-1,1-dioxo-thiolane-3-carboxamide;
*N*-[6-(6-chloro-1,3-benzothiazol-2-yl)spiro[3.3]heptan-2-yl]-1,1-dioxo-thiolane-3-carboxamide;
*N*-[6-(5-chloro-1,3-benzothiazol-2-yl)spiro[3.3]heptan-2-yl]-2-(1,1-dioxothietan-3-yl)acetamide;
*N*-[6-(5-chloro-1,3-benzothiazol-2-yl)spiro[3.3]heptan-2-yl]-2-(1,1-dioxothiolan-2-yl)acetamide;
*N*-[6-(5-chloro-1,3-benzothiazol-2-yl)spiro[3.3]heptan-2-yl]-2-(1,1-dioxothian-3-yl)acetamide;
*N*-[6-(5-chloro-1,3-benzothiazol-2-yl)spiro[3.3]heptan-2-yl]-1-ethyl-5-oxo-pyrrolidine-3-carboxamide;
*N*-[6-(5-chloro-1,3-benzothiazol-2-yl)spiro[3.3]heptan-2-yl]-1-methyl-2-oxo-piperidine-4-carboxamide;
*N*-[6-(5-chloro-1,3-benzothiazol-2-yl)spiro[3.3]heptan-2-yl]-2-(5-oxopyrrolidin-2-yl)acetamide;
*N*-[6-(5-chloro-1,3-benzothiazol-2-yl)spiro[3.3]heptan-2-yl]-2-(1,1-dioxothiazinan-2-yl)acetamide;
*N*-[6-(5-chloro-1,3-benzothiazol-2-yl)spiro[3.3]heptan-2-yl]-2-methylsulfonyl-pyridine-4-carboxamide;
*N*-[6-(6-chloro-1,3-benzothiazol-2-yl)spiro[3.3]heptan-2-yl]-2-cyclopropylsulfonyl-pyridine-4-carboxamide;
2-*tert*-butylsulfonyl-*N*-[6-(5-chloro-1,3-benzothiazol-2-yl)spiro[3.3]heptan-2-yl]pyridine-4-carboxamide;
*N*-[6-(5-chloro-1,3-benzothiazol-2-yl)spiro[3.3]heptan-2-yl]-2-(cyclopropylmethylsulfonyl)pyridine-4-carboxamide;
*N*-[6-(5-chloro-1,3-benzothiazol-2-yl)spiro[3.3]heptan-2-yl]-2-(methylsulfonimidoyl)pyridine-4-carboxamide;
*N*-[6-(5-chloro-1,3-benzothiazol-2-yl)spiro[3.3]heptan-2-yl]-2-(cyclopropylsulfonimidoyl)pyridine-4-carboxamide;
*N*-[6-(5-chloro-1,3-benzothiazol-2-yl)spiro[3.3]heptan-2-yl]-2-methoxy-pyridine-4-carboxamide;
*N*-[6-(5-chloro-1,3-benzothiazol-2-yl)spiro[3.3]heptan-2-yl]-2-isopropoxy-pyridine-4-carboxamide;
*N*-[6-(5-chloro-1,3-benzothiazol-2-yl)spiro[3.3]heptan-2-yl]-2-(methylamino)pyridine-4-carboxamide;
*N*-[6-(5-chloro-1,3-benzothiazol-2-yl)spiro[3.3]heptan-2-yl]-2-(cyclopropylamino)pyridine-4-carboxamid;
*N*-[6-(5-chloro-1,3-benzothiazol-2-yl)spiro[3.3]heptan-2-yl]-2-(dimethylamino)pyridine-4-carboxamide;
*N*-[6-(5-chloro-1,3-benzothiazol-2-yl)spiro[3.3]heptan-2-yl]-2-[ethyl(methyl)amino]pyridine-4-carboxamide;
*N*-[6-(5-chloro-1,3-benzothiazol-2-yl)spiro[3.3]heptan-2-yl]-2-(diethylamino)pyridine-4-carboxamide;
*N*-[6-(5-chloro-1,3-benzothiazol-2-yl)spiro[3.3]heptan-2-yl]-2-[isopropyl(methyl)amino]pyridine-4-carboxamide;
*N*-[6-(5-chloro-1,3-benzothiazol-2-yl)spiro[3.3]heptan-2-yl]-2-pyrrolidin-1-yl-pyridine-4-carboxamide;
*N*-[6-(5-chloro-1,3-benzothiazol-2-yl)spiro[3.3]heptan-2-yl]-2-(1-piperidyl)pyridine-4-carboxamide;
*N*-[6-(5-chloro-1,3-benzothiazol-2-yl)spiro[3.3]heptan-2-yl]-2-morpholino-pyridine-4-carboxamide;
(*S*ₐ)-*N4*-[6-(5-chloro-1,3-benzothiazol-2-yl)spiro[3.3]heptan-2-yl]pyridine-2,4-dicarboxamide;
(*Rₐ*)-*N4*-[6-(5-chloro-1,3-benzothiazol-2-yl)spiro[3.3]heptan-2-yl]pyridine-2,4-dicarboxamide;
(*S*ₐ)-*N*-[6-(5-chloro-1,3-benzothiazol-2-yl)spiro[3.3]heptan-2-yl]-2-methylsulfonyl-pyridine-4-carboxamide;
(*Rₐ*)-*N*-[6-(5-chloro-1,3-benzothiazol-2-yl)spiro[3.3]heptan-2-yl]-2-methylsulfonyl-pyridine-4-carboxamide;
*N*-[6-(5-chloro-1,3-benzothiazol-2-yl)spiro[3.3]heptan-2-yl]-2-cyclopropylsulfonyl-pyridine-4-carboxamide;
(*S*ₐ)-*N*-[6-(5-chloro-1,3-benzothiazol-2-yl)spiro[3.3]heptan-2-yl]-2-cyclopropylsulfonyl-pyridine-4-carboxamide;
(*Rₐ*)-*N*-[6-(5-chloro-1,3-benzothiazol-2-yl)spiro[3.3]heptan-2-yl]-2-cyclopropylsulfonyl-pyridine-4-carboxamide;
*N6*-[6-(5-chloro-1,3-benzothiazol-2-yl)spiro[3.3]heptan-2-yl]pyrimidine-4,6-dicarboxamide;
(*S*ₐ)-*N6*-[6-(5-chloro-1,3-benzothiazol-2-yl)spiro[3.3]heptan-2-yl]pyrimidine-4,6-dicarboxamide; and
(*Rₐ*)-*N6*-[6-(5-chloro-1,3-benzothiazol-2-yl)spiro[3.3]heptan-2-yl]pyrimidine-4,6-dicarboxamide; or a pharmaceutically acceptable salt thereof.

11. A compound selected from:
2-amino-*N*-[6-(5-chloro-1,3-benzothiazol-2-yl)spiro[3.3]heptan-2-yl]thiazole-4-carboxamide; *N*-[6-(5-chloro-1,3-benzothiazol-2-yl)spiro[3.3]heptan-2-yl]-1,1-dioxo-1,2-thiazolidine-3-carboxamide.

12. A process for the preparation of a compound according to claim 1 comprising any one or more of the following steps,
(a) Reaction of a compound of formula (IV), 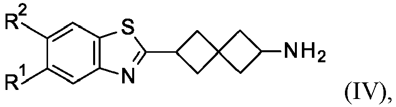 with a compound of formula (V), 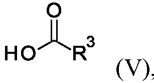 in the presence of a coupling reagent, wherein the coupling reagent is O-(7-aza-1H-benzotriazole-1-yl)-N,N,N',N'-tetramethyluronium hexafluorophosphate (HATU) or 2,4,6-tripropyl-1,3,5,2,4,6-trioxatriphosphinane 2,4,6-trioxide (T₃P);
wherein R³ is defined as any one of claims 1-9.
(b) Reaction of a compound of formula (I-2), 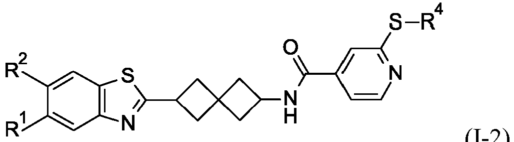 with an oxidant, wherein the oxidant is Oxone, or PhI(OAc)₂ and (NH₄)₂CO₃; wherein R⁴ is C₁₋₆alkyl, C₃₋₇cycloalkylC₁₋₆alkyl, or C₃₋₇cycloalkyl;
(c) Reaction of a compound of formula (I-4), 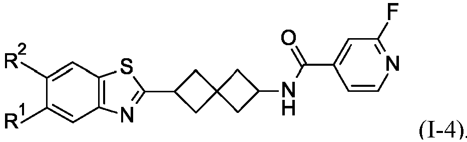 with an alcohol or amine (compound HR⁵) in the presence of a base, such as NaH or Cs₂CO₃; wherein R⁵ is C₁₋₆alkoxy, C₁₋₆alkylamino, (C₁₋₆alkyl)₂amino, C₃₋₇cycloalkylamino, morpholinyl, piperidinyl or pyrrolidinyl;
(d) Amination of a compound of formula (I-6), 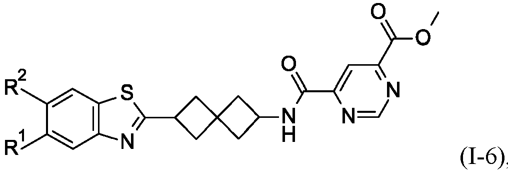 in a solution of ammonia in methanol.

13. A compound according to any one of claims 1 to 11, or a pharmaceutically acceptable salt thereof, for use as therapeutically active substance.

14. A pharmaceutical composition comprising a compound according to any one of claims 1 to 11, or a pharmaceutically acceptable salt thereof, and a pharmaceutically acceptable excipient.

15. A compound according to any one of claims 1 to 11, or a pharmaceutically acceptable salt thereof, for use in the treatment or prophylaxis of HBV infection.

## Patentansprüche

1. Verbindung der Formel (I), 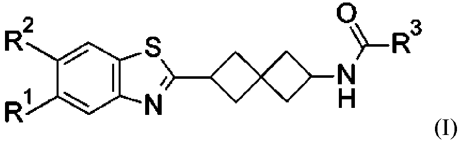 wobei
R¹ H oder Halogen ist;
R² H oder Halogen ist;
oder R¹ und R² gemeinsam mit dem Phenyl, mit dem sie verknüpft sind, ein 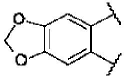 bilden;
R³ Folgendes ist: (1,1-Dioxothian-3-yl)C₁₋₆-alkyl;
(1,1-Dioxothiazinan-2-yl)C₁₋₆-alkyl;
(1,1-Dioxothietan-3-yl)C₁₋₆-alkyl;
(1,1-Dioxothian-2-yl)C₁₋₆-alkyl;
(5-Oxopyrrolidin-2-yl)C₁₋₆-alkyl;
1,1-Dioxo-1,2-thiazolidinyl;
1,1-Dioxothietanyl;
1,1-Dioxothiolanyl;
Aminobenzothiazolyl;
C₁₋₆-Alkyl-2-oxopiperidinyl;
C₁₋₆-Alkyl-5-oxopyrrolidinyl;
Furanyl, das mit ((Halogen-C₁₋₆-alkyl)-C₃₋₇-cycloalkyl)sulfamoyl, (Halogenazetidinyl)sulfonyl, (Halogen-C₃₋₇-cycloalkyl)sulfamoyl, Azetidin-1-ylsulfonyl, C₁₋₆-Alkylsulfonyl-C₁₋₆-alkyl, C₃₋₇-Cycloalkylsulfamoyl, C₃₋₇-Cycloalkylsulfonyl, Halogen-C₁₋₆-alkyl oder Hydroxyhalogen-C₁₋₆-alkyl substituiert ist;
Phenyl, das mit (C₁₋₆-Alkyl)₂-phosphoryl, Halogen-C₁₋₆-alkyl oder Sulfamoyl substituiert ist;
Pyridinyl, das mit (C₁₋₆-Alkyl)₂-amino, C₁₋₆-Alkoxy, C₁₋₆-Alkyl, C₁₋₆-Alkylamino, C₁₋₆-Alkylsulfonimidoyl, C₁₋₆-Alkylsulfonyl, C₁₋₆-Alkylsulfonyl- C₁₋₆-alkyl, C₃₋₇-Cycloalkylamino, C₃₋₇-Cycloalkyl-C₁₋₆-alkylsulfonyl, C₃₋₇-Cycloalkylsulfonimidoyl, C₃₋₇-Cycloalkylsulfonyl, Carbamoyl, Cyano, Halogen-C₁₋₆-alkyl, Morpholinyl, Piperidinyl, Pyrrolidinyl oder Ureido substituiert ist; oder
Pyrimidinyl, das mit Carbamoyl substituiert ist;
mit der Maßgabe, dass R¹ und R² nicht gleichzeitig H oder Halogen sind;
oder ein pharmazeutisch verträgliches Salz davon.

2. Verbindung nach Anspruch 1, wobei
R¹ H oder Chlor ist;
R² H oder Chlor ist;
oder R¹ und R² gemeinsam mit dem Phenyl, mit dem sie verknüpft sind, ein 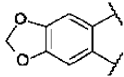 bilden;
R³ Folgendes ist: (1,1-Dioxothian-3-yl)methyl;
(1,1-Dioxothiazinan-2-yl)methyl;
(1,1-Dioxothietan-3-yl)methyl;
(1,1-Dioxothiolan-2-yl)methyl;
(5-Oxopyrrolidin-2-yl)methyl;
1,1-Dioxo-1,2-thiazolidinyl;
1,1-Dioxothietanyl;
1,1-Dioxothiolanyl;
Aminobenzothiazolyl;
1-Ethyl-5-oxopyrrolidinyl;
1-Methyl-2-oxopiperidinyl;
((3-(Trifluormethyl)cyclobutyl)sulfamoyl)furanyl;
(1-Methyl-1-methylsulfonylethyl)furanyl;
(1-Methylsulfonylethyl)furanyl;
(2,2,2-Trifluor-1-hydroxyethyl)furanyl;
(3,3-Difluorazetidin-1-yl)sulfonylfuranyl;
(Azetidin-1-ylsulfonyl)furanyl;
(Cyclobutylsulfamoyl)furanyl;
(Trifluormethyl)furanyl;
[(3,3-Difluorcyclobutyl)sulfamoyl]furanyl;
Cyclopropylsulfonylfuranyl;
Dimethylphosphorylphenyl;
Sulfamoylphenyl;
Trifluormethylphenyl;
(1-Methyl-1-methylsulfonylethyl)pyridinyl;
(1-Piperidinyl)pyridinyl;
(Cyclopropylamino)pyridinyl;
(Cyclopropylmethylsulfonyl)pyridinyl;
(Cyclopropylsulfonimidoyl)pyridinyl;
(Diethylamino)pyridinyl;
(Dimethylamino)pyridinyl;
(Methylamino)pyridinyl;
(Methylsulfonimidoyl)pyridinyl;
(Methylsulfonylmethyl)pyridinyl;
[Ethyl(methyl)amino]pyridinyl;
[Isopropyl(methyl)amino]pyridinyl;
Carbamoylpyridinyl;
Cyanopyridinyl;
Cyclopropylsulfonylpyridinyl;
Ethylsulfonylpyridinyl;
Isopropoxypyridinyl;
Methylpyridinyl;
Methoxypyridinyl;
Methylsulfonylpyridinyl;
Morpholinylpyridinyl;
Pyrrolidin-1-ylpyridinyl;
tert.-Butylsulfonylpyridinyl;
Trifluormethylpyridinyl;
Ureidopyridinyl; oder
Carbamoylpyrimidinyl;
mit der Maßgabe, dass R¹ und R² nicht gleichzeitig H oder Halogen sind;
oder ein pharmazeutisch verträgliches Salz davon.

3. Verbindung nach Anspruch 1 oder ein pharmazeutisch verträgliches Salz davon, wobei R¹ Halogen ist.

4. Verbindung nach einem der Ansprüche 1-3 oder ein pharmazeutisch verträgliches Salz davon, wobei R¹ Chlor ist.

5. Verbindung nach einem der Ansprüche 1-4 oder ein pharmazeutisch verträgliches Salz davon, wobei R² H ist.

6. Verbindung nach einem der Ansprüche 1, 3-5 oder ein pharmazeutisch verträgliches Salz davon, wobei
R³ Furanyl, das mit C₃₋₇-Cycloalkylsulfonyl substituiert ist; oder
Pyridinyl, das mit C₁₋₆-Alkylsulfonyl, C₃₋₇-Cycloalkyl-C₁₋₆-alkylsulfonyl, C₃₋₇-Cycloalkylsulfonimidoyl, C₃₋₇-Cycloalkylsulfonyl oder Carbamoyl substituiert ist, ist.

7. Verbindung nach einem der Ansprüche 1-6 oder ein pharmazeutisch verträgliches Salz davon, wobei R³ Cyclopropylsulfonylfuranyl, Cyclopropylsulfonylpyridinyl, (Cyclopropyl-methylsulfonyl)pyridinyl, (Cyclopropylsulfonimidoyl)pyridinyl, Carbamoylpyridinyl oder Methylsulfonylpyridinyl ist.

8. Verbindung nach einem der Ansprüche 1, 3, 5 und 6, wobei
R¹ Halogen ist;
R² H ist;
R³ Furanyl, das mit C₃₋₇-Cycloalkylsulfonyl substituiert ist; oder
Pyridinyl, das mit C₁₋₆-Alkylsulfonyl, C₃₋₇-Cycloalkyl-C₁₋₆-alkylsulfonyl, C₃₋₇-Cyclo-alkylsulfonimidoyl, C₃₋₇-Cycloalkylsulfonyl oder Carbamoyl substituiert ist, ist;
oder ein pharmazeutisch verträgliches Salz davon.

9. Verbindung nach einem der Ansprüche 1-8, wobei
R¹ Chlor ist;
R² H ist;
R³ Cyclopropylsulfonylfuranyl, Cyclopropylsulfonylpyridinyl, (Cyclopropylmethylsulfonyl)-pyridinyl, (Cyclopropylsulfonimidoyl)pyridinyl, Carbamoylpyridinyl oder Methylsulfonylpyridinyl ist;
oder ein pharmazeutisch verträgliches Salz davon.

10. Verbindung nach einem der Ansprüche 1-9, ausgewählt aus:
*N*-[6-(5-Chlor-1,3-benzothiazol-2-yl)spiro[3.3]heptan-2-yl]-3-(trifluormethyl)benzamid;
*N*-[6-(5-Chlor-1,3-benzothiazol-2-yl)spiro[3.3]heptan-2-yl]-3-sulfamoylbenzamid;
*N*-[6-(5-Chlor-1,3-benzothiazol-2-yl)spiro[3.3]heptan-2-yl]-3-dimethylphosphorylbenzamid;
*N*-[6-(5-Chlor-1,3-benzothiazol-2-yl)spiro[3.3]heptan-2-yl]-2-(trifluormethyl)pyridin-4-carboxamid;
*N*-[6-(5-Chlor-1,3-benzothiazol-2-yl)spiro[3.3]heptan-2-yl]-2-cyanopyridin-4-carboxamid;
*N*-[6-(5-Chlor-1,3-benzothiazol-2-yl)spiro[3.3]heptan-2-yl]-2-methylpyridin-4-carboxamid;
*N*-[6-(5-Chlor-1,3-benzothiazol-2-yl)spiro[3.3]heptan-2-yl]-2-(methylsulfonylmethyl)pyridin-4-carboxamid;
*N*-[6-(5-Chlor-1,3-benzothiazol-2-yl)spiro[3.3]heptan-2-yl]-2-(1-methyl-1-methylsulfonylethyl)pyridin-4-carboxamid;
*N*-[6-(5-Chlor-1,3-benzothiazol-2-yl)spiro[3.3]heptan-2-yl]-2-ethylsulfonylpyridin-4-carboxamid;
*N*-[6-(5-Chlor-1,3-benzothiazol-2-yl)spiro[3.3]heptan-2-y1]-2-ureidopyridin-4-carboxamid;
*N4*-[6-(5-Chlor-1,3-benzothiazol-2-yl)spiro[3.3]heptan-2-yl]pyridin-2,4-dicarboxamid;
*N4*-[6-([1,3]Dioxolo[4,5-f][1,3]benzothiazol-6-yl)spiro[3.3]heptan-2-yl]pyridin-2,4-dicarboxamid;
*N*-[6-(5-Chlor-1,3-benzothiazol-2-yl)spiro[3.3]heptan-2-yl]-5-cyclopropylsulfonylfuran-2-carboxamid;
*N*-[6-(5-Chlor-1,3-benzothiazol-2-yl)spiro[3.3]heptan-2-yl]-5-(trifluormethyl)furan-2-carboxamid;
*N*-[6-(6-Chlor-1,3-benzothiazol-2-yl)spiro[3.3]heptan-2-yl]-5-(trifluormethyl)furan-2-carboxamid;
*N*-[6-(5-Chlor-1,3-benzothiazol-2-yl)spiro[3.3]heptan-2-yl]-5-(1-methylsulfonylethyl)furan-2-carboxamid;
*N*-[6-(5-Chlor-1,3-benzothiazol-2-yl)spiro[3.3]heptan-2-yl]-5-(1-methyl-1-methylsulfonylethyl)furan-2-carboxamid;
*N*-[6-(5-Chlor-1,3-benzothiazol-2-yl)spiro[3.3]heptan-2-yl]-5-(2,2,2-trifluor-1-hydroxyethyl)furan-2-carboxamid;
5-(Azetidin-1-ylsulfonyl)-*N*-[6-(5-chlor-1,3-benzothiazol-2-yl)spiro[3.3]heptan-2-yl]furan-2-carboxamid;
*N*-[6-(5-Chlor-1,3-benzothiazol-2-yl)spiro[3.3]heptan-2-yl]-5-(3,3-difluorazetidin-1-yl)sulfonylfuran-2-carboxamid;
*N*-[6-(5-Chlor-1,3-benzothiazol-2-yl)spiro[3.3]heptan-2-yl]-5-(cyclobutylsulfamoyl)furan-2-carboxamid;
*N*-[6-(5-Chlor-1,3-benzothiazol-2-yl)spiro[3.3]heptan-2-yl]-5-[(3,3-difluorcyclobutyl)sulfamoyl]furan-2-carboxamid;
*N*-[6-(5-Chlor-1,3-benzothiazol-2-yl)spiro[3.3]heptan-2-yl]-5-[[3-(trifluormethyl)cyclobutyl]sulfamoyl]furan-2-carboxamid;
*N*-[6-(5-Chlor-1,3-benzothiazol-2-yl)spiro[3.3]heptan-2-yl]-1,1-dioxothietan-3-carboxamid;
*N*-[6-(5-Chlor-1,3-benzothiazol-2-yl)spiro[3.3]heptan-2-yl]-1,1-dioxothiolan-3-carboxamid;
*N*-[6-(6-Chlor-1,3-benzothiazol-2-yl)spiro[3.3]heptan-2-yl]-1,1-dioxothiolan-3-carboxamid;
*N*-[6-(5-Chlor-1,3-benzothiazol-2-yl)spiro[3.3]heptan-2-y1]-2-(1,1-dioxothietan-3-yl)acetamid;
*N*-[6-(5-Chlor-1,3-benzothiazol-2-yl)spiro[3.3]heptan-2-yl]-2-(1,1-dioxothiolan-2-yl)acetamid;
*N*-[6-(5-Chlor-1,3-benzothiazol-2-yl)spiro[3.3]heptan-2-yl]-2-(1,1-dioxothian-3-yl)acetamid;
*N*-[6-(5-Chlor-1,3-benzothiazol-2-yl)spiro[3.3]heptan-2-yl]-1-ethyl-5-oxopyrrolidin-3-carboxamid;
*N*-[6-(5-Chlor-1,3-benzothiazol-2-yl)spiro[3.3]heptan-2-yl]-1-methyl-2-oxopiperidin-4-carboxamid;
*N*-[6-(5-Chlor-1,3-benzothiazol-2-yl)spiro[3.3]heptan-2-yl]-2-(5-oxopyrrolidin-2-yl)acetamid;
*N*-[6-(5-Chlor-1,3-benzothiazol-2-yl)spiro[3.3]heptan-2-yl]-2-(1,1-dioxothiazinan-2-yl)acetamid;
*N*-[6-(5-Chlor-1,3-benzothiazol-2-yl)spiro[3.3]heptan-2-y1]-2-methylsulfonylpyridin-4-carboxamid;
*N*-[6-(6-Chlor-1,3-benzothiazol-2-yl)spiro[3.3]heptan-2-yl]-2-cyclopropylsulfonylpyridin-4-carboxamid;
2-*tert*.-Butylsulfonyl-*N*-[6-(5-chlor-1,3-benzothiazol-2-yl)spiro[3.3]heptan-2-yl]pyridin-4-carboxamid;
*N*-[6-(5-Chlor-1,3-benzothiazol-2-yl)spiro[3.3]heptan-2-yl]-2-(cyclopropylmethylsulfonyl)pyridin-4-carboxamid;
*N*-[6-(5-Chlor-1,3-benzothiazol-2-yl)spiro[3.3]heptan-2-yl]-2-(methylsulfonimidoyl)pyridin-4-carboxamid;
*N*-[6-(5-Chlor-1,3-benzothiazol-2-yl)spiro[3.3]heptan-2-yl]-2-(cyclopropylsulfonimidoyl)pyridin-4-carboxamid;
*N*-[6-(5-Chlor-1,3-benzothiazol-2-yl)spiro[3.3]heptan-2-yl]-2-methoxypyridin-4-carboxamid;
*N*-[6-(5 -Chlor-1,3 -benzothiazol-2-yl)spiro[3.3]heptan-2-yl]-2-isopropoxypyridin-4-carboxamid;
*N*-[6-(5-Chlor-1,3-benzothiazol-2-yl)spiro[3.3]heptan-2-yl]-2-(methylamino)pyridin-4-carboxamid;
*N*-[6-(5-Chlor-1,3-benzothiazol-2-yl)spiro[3.3]heptan-2-y1]-2-(cyclopropylamino)pyridin-4-carboxamid;
*N*-[6-(5-Chlor-1,3-benzothiazol-2-yl)spiro[3.3]heptan-2-y1]-2-(dimethylamino)pyridin-4-carboxamid;
*N*-[6-(5-Chlor-1,3-benzothiazol-2-yl)spiro[3.3]heptan-2-yl]-2-[ethyl(methyl)amino]pyridin-4-carboxamid;
*N*-[6-(5-Chlor-1,3-benzothiazol-2-yl)spiro[3.3]heptan-2-yl]-2-(diethylamino)pyridin-4-carboxamid;
*N*-[6-(5-Chlor-1,3-benzothiazol-2-yl)spiro[3.3]heptan-2-yl]-2-[isopropyl(methyl)amino]pyridin-4-carboxamid;
*N*-[6-(5-Chlor-1,3-benzothiazol-2-yl)spiro[3.3]heptan-2-yl]-2-pyrrolidin-1-ylpyridin-4-carboxamid;
*N*-[6-(5-Chlor-1,3-benzothiazol-2-yl)spiro[3.3]heptan-2-yl]-2-(1-piperidyl)pyridin-4-carboxamid;
*N*-[6-(5-Chlor-1,3-benzothiazol-2-yl)spiro[3.3 ]heptan-2-y1]-2-morpholinopyridin-4-carboxamid;
*(Sₐ)*-*N4*-[6-(5-Chlor-1,3-benzothiazol-2-yl)spiro[3.3]heptan-2-yl]pyridin-2,4-dicarboxamid;
*(Rₐ)-N4*-[6-(5-Chlor-1,3-benzothiazol-2-yl)spiro[3.3]heptan-2-yl]pyridin-2,4-dicarboxamid;
*(Sₐ)-N*-[6-(5-Chlor-1,3-benzothiazol-2-yl)spiro[3.3]heptan-2-yl]-2-methylsulfonylpyridin-4-carboxamid;
*(Rₐ)-N*-[6-(5-Chlor-1,3-benzothiazol-2-yl)spiro[3.3]heptan-2-yl]-2-methylsulfonylpyridin-4-carboxamid;
*N*-[6-(5-Chlor-1,3-benzothiazol-2-yl)spiro[3.3]heptan-2-yl]-2-cyclopropylsulfonylpyridin-4-carboxamid;
*(Sₐ)-N*-[6-(5-Chlor-1,3-benzothiazol-2-yl)spiro[3.3]heptan-2-yl]-2-cyclopropylsulfonylpyridin-4-carboxamid;
*(Rₐ)-N*-[6-(5-Chlor-1,3-benzothiazol-2-yl)spiro[3.3]heptan-2-yl]-2-cyclopropylsulfonylpyridin-4-carboxamid;
*N6*-[6-(5-Chlor-1,3-benzothiazol-2-yl)spiro[3.3]heptan-2-yl]pyrimidin-4,6-dicarboxamid;
*(Sₐ)-N6*-[6-(5-Chlor-1,3-benzothiazol-2-yl)spiro[3.3]heptan-2-yl]pyrimidin-4,6-dicarboxamid; und
*(Rₐ)-N6*-[6-(5-Chlor-1,3-benzothiazol-2-yl)spiro[3.3]heptan-2-yl]pyrimidin-4,6-dicarboxamid; oder ein pharmazeutisch verträgliches Salz davon.

11. Verbindung, ausgewählt aus:
2-Amino-*N*-[6-(5-chlor-1,3-benzothiazol-2-yl)spiro[3.3]heptan-2-yl]thiazol-4-carboxamid;
*N*-[6-(5-Chlor-1,3-benzothiazol-2-yl)spiro[3.3]heptan-2-yl]-1,1-dioxo-1,2-thiazolidin-3-carboxamid.

12. Verfahren zur Herstellung einer Verbindung nach Anspruch 1, umfassend einen oder mehrere der folgenden Schritte,
(a) Reagieren einer Verbindung der Formel (IV), 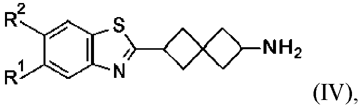 mit einer Verbindung der Formel (V), 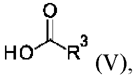 in Gegenwart eines Kupplungsreagenzes, wobei das Kuppplungsreagenz O-(7-Aza-1H-benzotriazol-1-yl)-N,N,N',N'-tetramethyluroniumhexafluorphosphat (HATU) oder 2,4,6-Tripropyl-1,3,5,2,4,6-trioxatriphosphinan-2,4,6-trioxid (T₃P) ist;
wobei R³ wie in einem der Ansprüche 1-9 definiert ist,
(b) Reagieren einer Verbindung der Formel (I-2), 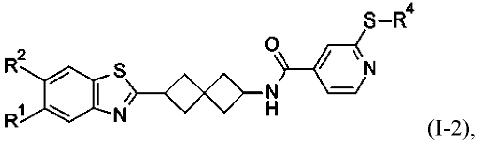 mit einem Oxidationsmittel, wobei das Oxidationsmittel Oxone oder PhI(OAc)₂ und (NH₄)₂CO₃ ist; wobei R⁴ C₁₋₆-Alkyl, C₃₋₇-Cycloalkyl-C₁₋₆-alkyl oder C₃₋₇-Cycloalkyl ist;
(c) Reagieren einer Verbindung der Formel (1-4), 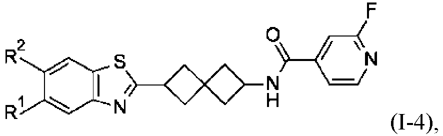 mit einem Alkohol oder Amin (Verbindung HR⁵) in Gegenwart einer Base, wie NaH oder CS₂CO₃; wobei R⁵ C₁₋₆-Alkoxy, C₁₋₆-Alkylamino, (C₁₋₆-Alkyl)₂amino, C₃₋₇-Cycloalkylamino, Morpholinyl, Piperidinyl oder Pyrrolidinyl ist;
(d) Aminieren einer Verbindung der Formel (1-6), 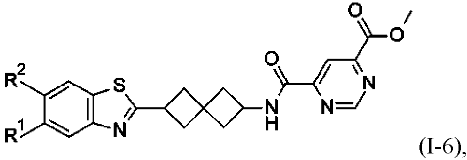 in einer Lösung von Ammoniak in Methanol.

13. Verbindung nach einem der Ansprüche 1 bis 11 oder ein pharmazeutisch verträgliches Salz davon zur Verwendung als therapeutisch wirksame Substanz.

14. Pharmazeutische Zusammensetzung, umfassend eine Verbindung nach einem der Ansprüche 1 bis 11 oder ein pharmazeutisch verträgliches Salz davon und einen pharmazeutisch verträglichen Exzipienten.

15. Verbindung nach einem der Ansprüche 1 bis 11 oder ein pharmazeutisch verträgliches Salz davon zur Verwendung bei der Behandlung oder Prophylaxe von HBV-Infektionen.

## Revendications

1. Composé de formule (I), 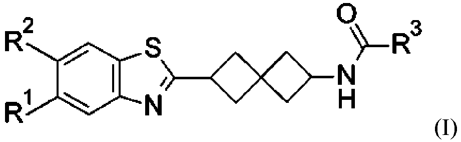 dans lequel
R¹ représente H ou un halogène ;
R² représente H ou un halogène ;
ou R¹ et R² conjointement avec le phényle auquel ils sont liés forment un 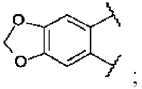
R³ représente un (1,1-dioxothian-3-yl)alkyle en C₁₋₆ ;
un (1,1-dioxothiazinan-2-yl)alkyle en C₁₋₆ ;
un (1,1-dioxothiétan-3-yl)alkyle en C₁₋₆ ;
un (1,1-dioxothiolan-2-yl)alkyle en C₁₋₆ ;
un (5-oxopyrrolidin-2-yl)alkyle en C₁₋₆ ;
un 1,1-dioxo-1,2-thiazolidinyle ;
un 1,1-dioxo-thiétanyle ;
un 1,1-dioxo-thiolanyle ;
un aminobenzothiazolyle ;
un alkyle en C₁₋₆-2-oxo-pipéridinyle ;
un alkyle en C₁₋₆-5-oxo-pyrrolidinyle ;
un furanyle qui est substitué par un ((halogénoalkyle en C₁₋₆)cycloalkyle en C₃₋₇)sulfamoyle, un (halogénoazétidinyl)sulfonyle, un (halogénocycloalkyle en C₃₋₇)sulfamoyle, un azétidin-1-ylsulfonyle, un alkyle en C₁₋₆-sulfonylalkyle en C₁₋₆, un cycloalkyle en C₃₋₇-sulfamoyle, un cycloalkyle en C₃₋₇-sulfonyle, un halogénoalkyle en C₁₋₆ ou un hydroxyhalogénoalkyle en C₁₋₆ ;
un phényle qui est substitué par un (alkyle en C₁₋₆)₂phosphoryle, un halogénoalkyle en C₁₋₆ ou un sulfamoyle ;
un pyridinyle qui est substitué par un (alkyle en C₁₋₆)₂amino, un alcoxy en C₁₋₆, un alkyle en C₁₋₆, un alkyle en C₁₋₆-amino, un alkyle en C₁₋₆-sulfonimidoyle, un alkyle en C₁₋₆-sulfonyle, un alkyle en C₁₋₆-sulfonylalkyle en C₁₋₆, un cycloalkyle en C₃₋₇-amino, un cycloalkyle en C₃₋₇-alkyle en C₁₋₆-sulfonyle, un cycloalkyle en C₃₋₇-sulfonimidoyle, un cycloalkyle en C₃₋₇-sulfonyle, un carbamoyle, un cyano, un halogénoalkyle en C₁₋₆, un morpholinyle, un pipéridinyle, un pyrrolidinyle ou un uréido ; ou
un pyrimidinyle qui est substitué par un carbamoyle ;
à condition que R¹ et R² ne représentent pas H ou un halogène simultanément ;
ou un sel pharmaceutiquement acceptable de celui-ci.

2. Composé selon la revendication 1, dans lequel
R¹ représente H ou un chlore ;
R² représente H ou un chlore ;
ou R¹ et R² conjointement avec le phényle auquel ils sont liés forment un 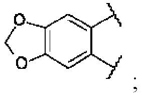
R³ représente un (1,1-dioxothian-3-yl)méthyle ;
un (1,1-dioxothiazinan-2-yl)méthyle ;
un (1,1-dioxothiétan-3-yl)méthyle ;
un (1,1-dioxothiolan-2-yl)méthyle ;
un (5-oxopyrrolidin-2-yl)méthyle ;
un 1,1-dioxo-1,2-thiazolidinyle ;
un 1,1-dioxo-thiétanyle ;
un 1,1-dioxo-thiolanyle ;
un aminobenzothiazolyle ;
un 1-éthyl-5-oxo-pyrrolidinyle ;
un 1-méthyl-2-oxo-pipéridinyle ;
un ((3-(trifluorométhyl)cyclobutyl)sulfamoyl)furanyle ;
un (1-méthyl-1-méthylsulfonyl-éthyl)furanyle ;
un (1-méthylsulfonyléthyl)furanyle ;
un (2,2,2-trifluoro-1-hydroxy-éthyl)furanyle ;
un (3,3-difluoroazétidin-1-yl)sulfonylfuranyle ;
un (azétidin-1-ylsulfonyl)furanyle ;
un (cyclobutylsulfamoyl)furanyle ;
un (trifluorométhyl)furanyle ;
un [(3,3-difluorocyclobutyl)sulfamoyl]furanyle ;
un cyclopropylsulfonylfuranyle ;
un diméthylphosphorylphényle ;
un sulfamoylphényle ;
un trifluorométhylphényle ;
un (1-méthyl-1-méthylsulfonyl-éthyl)pyridinyle ;
un (1-pipéridinyl)pyridinyle ;
un (cyclopropylamino)pyridinyle ;
un (cyclopropylméthylsulfonyl)pyridinyle ;
un (cyclopropylsulfonimidoyl)pyridinyle ;
un (diéthylamino)pyridinyle ;
un (diméthylamino)pyridinyle ;
un (méthylamino)pyridinyle ;
un (méthylsulfonimidoyl)pyridinyle ;
un (méthylsulfonylméthyl)pyridinyle ;
un [éthyl(méthyl)amino]pyridinyle ;
un [isopropyl(méthyl)amino]pyridinyle ;
un carbamoylpyridinyle ;
un cyanopyridinyle ;
un cyclopropylsulfonylpyridinyle ;
un éthylsulfonylpyridinyle ;
un isopropoxypyridinyle ;
un méthylpyridinyle ;
un méthoxypyridinyle ;
un méthylsulfonylpyridinyle ;
un morpholinylpyridinyle ;
un pyrrolidin-1-ylpyridinyle ;
un tert-butylsulfonylpyridinyle ;
un trifluorométhylpyridinyle ;
un uréidopyridinyle ; ou
un carbamoylpyrimidinyle ;
à condition que R¹ et R² ne représentent pas H ou un halogène simultanément ;
ou un sel pharmaceutiquement acceptable de celui-ci.

3. Composé selon la revendication 1, ou un sel pharmaceutiquement acceptable de celui-ci, dans lequel R¹ représente un halogène.

4. Composé selon l'une quelconque des revendications 1 à 3, ou un sel pharmaceutiquement acceptable de celui-ci, dans lequel R¹ représente un chlore.

5. Composé selon l'une quelconque des revendications 1 à 4, ou sel pharmaceutiquement acceptable de celui-ci, dans lequel R² représente H.

6. Composé selon l'une quelconque des revendications 1 et 3 à 5, ou un sel pharmaceutiquement acceptable de celui-ci, dans lequel
R³ représente un furanyle qui est substitué par un cycloalkyle en C₃₋₇-sulfonyle ; ou
un pyridinyle qui est substitué par un alkyle en C₁₋₆-sulfonyle, un cycloalkyle en C₃₋₇-alkyle en C₁₋₆-sulfonyle, un cycloalkyle en C₃₋₇-sulfonimidoyle, un cycloalkyle en C₃₋₇-sulfonyle ou un carbamoyle.

7. Composé selon l'une quelconque des revendications 1 à 6, ou un sel pharmaceutiquement acceptable de celui-ci, dans lequel R³ représente un cyclopropylsulfonylfuranyle, un cyclopropylsulfonylpyridinyle, un (cyclopropylméthylsulfonyl)pyridinyle, un (cyclopropylsulfonimidoyl)pyridinyle, un carbamoylpyridinyle ou un méthylsulfonylpyridinyle.

8. Composé selon l'une quelconque des revendications 1, 3, 5 et 6, dans lequel
R¹ représente un halogène ;
R² représente H ;
R³ représente un furanyle qui est substitué par un cycloalkyle en C₃₋₇-sulfonyle ; ou
un pyridinyle qui est substitué par un alkyle en C₁₋₆-sulfonyle, un cycloalkyle en C₃₋₇-alkyle en C₁₋₆-sulfonyle, un cycloalkyle en C₃₋₇-sulfonimidoyle, un cycloalkyle en C₃₋₇-sulfonyle ou un carbamoyle ;
ou un sel pharmaceutiquement acceptable de celui-ci.

9. Composé selon l'une quelconque des revendications 1 à 8, dans lequel
R¹ représente un chlore ;
R² représente H ;
R³ représente un cyclopropylsulfonylfuranyle, un cyclopropylsulfonylpyridinyle, un (cyclopropylméthylsulfonyl)pyridinyle, un (cyclopropylsulfonimidoyl)pyridinyle, un carbamoylpyridinyle ou un méthylsulfonylpyridinyle ;
ou un sel pharmaceutiquement acceptable de celui-ci.

10. Composé selon l'une quelconque des revendications 1 à 9, choisi parmi :
le *N*-[6-(5-chloro-1,3-benzothiazol-2-yl)spiro[3.3]heptan-2-yl]-3-(trifluorométhyl)benzamide ;
le *N*-[6-(5-chloro-1,3-benzothiazol-2-yl)spiro[3.3]heptan-2-yl]-3-sulfamoyl-benzamide ;
le *N*-[6-(5-chloro-1,3-benzothiazol-2-yl)spiro[3.3]heptan-2-yl]-3-diméthylphosphoryl-benzamide ;
le *N*-[6-(5-chloro-1,3-benzothiazol-2-yl)spiro[3.3]heptan-2-yl]-2-(trifluorométhyl)pyridine-4-carboxamide ;
le *N*-[6-(5-chloro-1,3-benzothiazol-2-yl)spiro[3.3]heptan-2-yl]-2-cyano-pyridine-4-carboxamide ;
le *N*-[6-(5-chloro-1,3-benzothiazol-2-yl)spiro[3.3]heptan-2-yl]-2-méthyl-pyridine-4-carboxamide ;
le *N*-[6-(5-chloro-1,3-benzothiazol-2-yl)spiro[3.3]heptan-2-yl]-2-(méthylsulfonylméthyl)pyridine-4-carboxamide ;
le *N*-[6-(5-chloro-1,3-benzothiazol-2-yl)spiro[3.3]heptan-2-yl]-2-(1-méthyl-1-méthylsulfonyl-éthyl)pyridine-4-carboxamide ;
le *N*-[6-(5-chloro-1,3-benzothiazol-2-yl)spiro[3.3]heptan-2-yl]-2-éthylsulfonyl-pyridine-4-carboxamide ;
le *N*-[6-(5-chloro-1,3-benzothiazol-2-yl)spiro[3.3]heptan-2-yl]-2-uréido-pyridine-4-carboxamide ;
le *N4*-[6-(5-chloro-1,3-benzothiazol-2-yl)spiro[3.3]heptan-2-yl]pyridine-2,4-dicarboxamide ;
le *N4*-[6-([1,3]dioxolo[4,5-f][1,3]benzothiazol-6-yl)spiro[3.3]heptan-2-yl]pyridine-2,4-dicarboxamide ;
le *N*-[6-(5-chloro-1,3-benzothiazol-2-yl)spiro[3.3]heptan-2-yl]-5-cyclopropylsulfonyl-furan-2-carboxamide ;
le *N*-[6-(5-chloro-1,3-benzothiazol-2-yl)spiro[3.3]heptan-2-yl]-5-(trifluorométhyl)furan-2-carboxamide ;
le *N*-[6-(6-chloro-1,3-benzothiazol-2-yl)spiro[3.3]heptan-2-yl]-5-(trifluorométhyl)furan-2-carboxamide ;
le *N*-[6-(5-chloro-1,3-benzothiazol-2-yl)spiro[3.3]heptan-2-yl]-5-(1-méthylsulfonyléthyl)furan-2-carboxamide ;
le *N*-[6-(5-chloro-1,3-benzothiazol-2-yl)spiro[3.3]heptan-2-yl]-5-(1-méthyl-1-méthylsulfonyl-éthyl)furan-2-carboxamide ;
le *N*-[6-(5-chloro-1,3-benzothiazol-2-yl)spiro[3.3]heptan-2-yl]-5-(2,2,2-trifluoro-1-hydroxy-éthyl)furan-2-carboxamide ;
le 5-(azétidin-1-ylsulfonyl)-*N*-[6-(5-chloro-1,3-benzothiazol-2-yl)spiro[3.3]heptan-2-yl]furan-2-carboxamide ;
le *N*-[6-(5-chloro-1,3-benzothiazol-2-yl)spiro[3.3]heptan-2-yl]-5-(3,3-difluoroazétidin-1-yl)sulfonyl-furan-2-carboxamide ;
le *N*-[6-(5-chloro-1,3-benzothiazol-2-yl)spiro[3.3]heptan-2-yl]-5-(cyclobutylsulfamoyl)furan-2-carboxamide ;
le *N*-[6-(5-chloro-1,3-benzothiazol-2-yl)spiro[3.3]heptan-2-yl]-5-[(3,3-difluorocyclobutyl)sulfamoyl]furan-2-carboxamide ;
le *N*-[6-(5-chloro-1,3-benzothiazol-2-yl)spiro[3.3]heptan-2-yl]-5-[[3-(trifluorométhyl)cyclobutyl]sulfamoyl]furan-2-carboxamide ;
le *N*-[6-(5-chloro-1,3-benzothiazol-2-yl)spiro[3.3]heptan-2-yl]-1,1-dioxo-thiétane-3-carboxamide ;
le *N*-[6-(5-chloro-1,3-benzothiazol-2-yl)spiro[3.3]heptan-2-yl]-1,1-dioxo-thiolane-3-carboxamide ;
le *N*-[6-(6-chloro-1,3-benzothiazol-2-yl)spiro[3.3]heptan-2-yl]-1,1-dioxo-thiolane-3-carboxamide ;
le *N*-[6-(5-chloro-1,3-benzothiazol-2-yl)spiro[3.3]heptan-2-yl]-2-(1,1-dioxothiétan-3-yl)acétamide ;
le *N*-[6-(5-chloro-1,3-benzothiazol-2-yl)spiro[3.3]heptan-2-yl]-2-(1,1-dioxothiolan-2-yl)acétamide ;
le *N*-[6-(5-chloro-1,3-benzothiazol-2-yl)spiro[3.3]heptan-2-yl]-2-(1,1-dioxothian-3-yl)acétamide ;
le *N*-[6-(5-chloro-1,3-benzothiazol-2-yl)spiro[3.3]heptan-2-yl]-1-éthyl-5-oxo-pyrrolidine-3-carboxamide ;
le *N*-[6-(5-chloro-1,3-benzothiazol-2-yl)spiro[3.3]heptan-2-yl]-1-méthyl-2-oxo-piperidine-4-carboxamide ;
le *N*-[6-(5-chloro-1,3-benzothiazol-2-yl)spiro[3.3]heptan-2-yl]-2-(5-oxopyrrolidin-2-yl)acétamide ;
le *N*-[6-(5-chloro-1,3-benzothiazol-2-yl)spiro[3.3]heptan-2-yl]-2-(1,1-dioxothiazinan-2-yl)acétamide ;
le *N*-[6-(5-chloro-1,3-benzothiazol-2-yl)spiro[3.3]heptan-2-yl]-2-méthylsulfonyl-pyridine-4-carboxamide ;
le *N*-[6-(6-chloro-1,3-benzothiazol-2-yl)spiro[3.3]heptan-2-yl]-2-cyclopropylsulfonyl-pyridine-4-carboxamide ;
le 2-*tert*-butylsulfonyl-N-[6-(5-chloro-1,3-benzothiazol-2-yl)spiro[3.3]heptan-2-yl]pyridine-4-carboxamide ;
le *N*-[6-(5-chloro-1,3-benzothiazol-2-yl)spiro[3.3]heptan-2-yl]-2-(cyclopropylméthylsulfonyl)pyridine-4-carboxamide ;
le *N*-[6-(5-chloro-1,3-benzothiazol-2-yl)spiro[3.3]heptan-2-yl]-2-(méthylsulfonimidoyl)pyridine-4-carboxamide ;
le *N*-[6-(5-chloro-1,3-benzothiazol-2-yl)spiro[3.3]heptan-2-yl]-2-(cyclopropylsulfonimidoyl)pyridine-4-carboxamide ;
le *N*-[6-(5-chloro-1,3-benzothiazol-2-yl)spiro[3.3]heptan-2-yl]-2-méthoxy-pyridine-4-carboxamide ;
le *N*-[6-(5-chloro-1,3-benzothiazol-2-yl)spiro[3.3]heptan-2-yl]-2-isopropoxy-pyridine-4-carboxamide ;
le *N*-[6-(5-chloro-1,3-benzothiazol-2-yl)spiro[3.3]heptan-2-yl]-2-(méthylamino)pyridine-4-carboxamide ;
le *N*-[6-(5-chloro-1,3-benzothiazol-2-yl)spiro[3.3]heptan-2-yl]-2-(cyclopropylamino)pyridine-4-carboxamide ;
le *N*-[6-(5-chloro-1,3-benzothiazol-2-yl)spiro[3.3]heptan-2-yl]-2-(diméthylamino)pyridine-4-carboxamide ;
le *N*-[6-(5-chloro-1,3-benzothiazol-2-yl)spiro[3.3]heptan-2-yl]-2-[éthyl(méthyl)amino]pyridine-4-carboxamide ;
le *N*-[6-(5-chloro-1,3-benzothiazol-2-yl)spiro[3.3]heptan-2-yl]-2-(diéthylamino)pyridine-4-carboxamide ;
le *N*-[6-(5-chloro-1,3-benzothiazol-2-yl)spiro[3.3]heptan-2-yl]-2-[isopropyl(méthyl)amino]pyridine-4-carboxamide ;
le *N*-[6-(5-chloro-1,3-benzothiazol-2-yl)spiro[3.3]heptan-2-yl]-2-pyrrolidin-1-yl-pyridine-4-carboxamide ;
le *N*-[6-(5-chloro-1,3-benzothiazol-2-yl)spiro[3.3]heptan-2-yl]-2-(1-piperidyl)pyridine-4-carboxamide ;
le *N*-[6-(5-chloro-1,3-benzothiazol-2-yl)spiro[3.3]heptan-2-yl]-2-morpholino-pyridine-4-carboxamide ;
le (*S*ₐ)-*N4*-[6-(5-chloro-1,3-benzothiazol-2-yl)spiro[3.3]heptan-2-yl]pyridine-2,4-dicarboxamide ;
le (*R*ₐ)-*N4*-[6-(5-chloro-1,3-benzothiazol-2-yl)spiro[3.3]heptan-2-yl]pyridine-2,4-dicarboxamide ;
le (*S*ₐ)-*N*-[6-(5-chloro-1,3-benzothiazol-2-yl)spiro[3.3]heptan-2-yl]-2-méthylsulfonyl-pyridine-4-carboxamide ;
le (*R*ₐ)-*N*-[6-(5-chloro-1,3-benzothiazol-2-yl)spiro[3.3]heptan-2-yll-2-méthylsulfonyl-pyridine-4-carboxamide ;
le *N*-[6-(5-chloro-1,3-benzothiazol-2-yl)spiro[3.3]heptan-2-yl]-2-cyclopropylsulfonyl-pyridine-4-carboxamide ;
le (*S*ₐ)-*N*-[6-(5-chloro-1,3-benzothiazol-2-yl)spiro[3.3]heptan-2-yl]-2-cyclopropylsulfonyl-pyridine-4-carboxamide ;
le (*R*ₐ)-*N*-[6-(5-chloro-1,3-benzothiazol-2-yl)spiro[3.3]heptan-2-yl]-2-cyclopropylsulfonyl-pyridine-4-carboxamide ;
le *N6*-[6-(5-chloro-1,3-benzothiazol-2-yl)spiro[3.3]heptan-2-yl]pyrimidine-4,6-dicarboxamide ;
le (*S*ₐ)-*N6*-[6-(5-chloro-1,3-benzothiazol-2-yl)spiro[3.3]heptan-2-yl]pyrimidine-4,6-dicarboxamide ; et
le (*R*ₐ)-*N6*-[6-(5-chloro-1,3-benzothiazol-2-yl)spiro[3.3]heptan-2-yl]pyrimidine-4,6-dicarboxamide ;
ou un sel pharmaceutiquement acceptable de ceux-ci.

11. Composé choisi parmi :
le 2-amino-*N*-[6-(5-chloro-1,3-benzothiazol-2-yl)spiro[3.3]heptan-2-yl]thiazole-4-carboxamide ;
le *N*-[6-(5-chloro-1,3-benzothiazol-2-yl)spiro[3.3]heptan-2-yl]-1,1-dioxo-1,2-thiazolidine-3-carboxamide.

12. Procédé de préparation d'un composé selon la revendication 1 comprenant une quelconque ou plusieurs des étapes suivantes,
(a) réaction d'un composé de formule (IV), 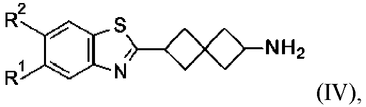 avec un composé de formule (V), 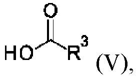 en présence d'un réactif de couplage, dans lequel le réactif de couplage est l'hexafluorophosphate de O-(7-aza-1H-benzotriazol-1-yl)-N,N,N',N'-tétraméthyluronium (HATU) ou le 2,4,6-trioxyde de 2,4,6-tripropyl-1,3,5,2,4,6-trioxatriphosphinane (T₃P) ;
dans lequel R³ est défini comme dans l'une quelconque des revendications 1 à 9,
(b) réaction d'un composé de formule (I-2), 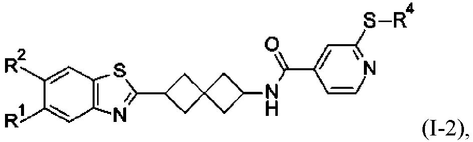 avec un oxydant, dans lequel l'oxydant est l'oxone ou PhI(OAc)₂ et (NH₄)₂CO₃ ; dans lequel R⁴ représente un alkyle en C₁₋₆, un cycloalkyle en C₃₋₇-alkyle en C₁₋₆ ou un cycloalkyle en C₃₋₇ ;
(c) réaction d'un composé de formule (I-4), 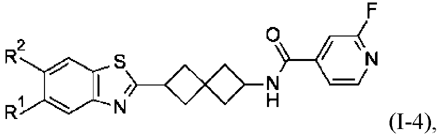 avec un alcool ou une amine (composé HR⁵) en présence d'une base, telle que NaH ou CS₂CO₃ ; dans lequel R⁵ représente un alcoxy en C₁₋₆, un alkyle en C₁₋₆-amino, un (alkyle en C₁₋₆)₂amino, un cycloalkyle en C₃₋₇-amino, un morpholinyle, un pipéridinyle ou un pyrrolidinyle ;
(d) amination d'un composé de formule (I-6), 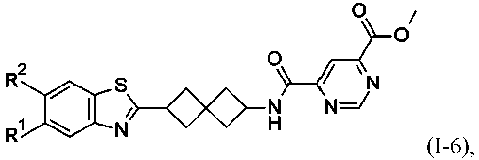 dans une solution d'ammoniac dans du méthanol.

13. Composé selon l'une quelconque des revendications 1 à 11, ou un sel pharmaceutiquement acceptable de celui-ci, pour une utilisation comme substance thérapeutiquement active.

14. Composition pharmaceutique comprenant un composé selon l'une quelconque des revendications 1 à 11, ou un sel pharmaceutiquement acceptable de celui-ci, et un excipient pharmaceutiquement acceptable.

15. Composé selon l'une quelconque des revendications 1 à 11, ou un sel pharmaceutiquement acceptable de celui-ci, pour une utilisation dans le traitement ou la prophylaxie d'une infection par le VHB.
